(19) Europäisches Patentamt
European Patent Office
Office européen des brevets

(11) EP 4 549 431 A1

(12) EUROPEAN PATENT APPLICATION
published in accordance with Art. 153(4) EPC

(43) Date of publication:
07.05.2025 Bulletin 2025/19

(21) Application number: 23830537.9

(22) Date of filing: 30.06.2023

(51) International Patent Classification (IPC):
C07D 237/20 (2006.01) C07D 403/04 (2006.01)
A61K 31/501 (2006.01) A61P 35/00 (2006.01)

(52) Cooperative Patent Classification (CPC):
A61K 31/501; A61P 35/00; C07D 237/20;
C07D 403/04

(86) International application number:
PCT/CN2023/105427

(87) International publication number:
WO 2024/002375 (04.01.2024 Gazette 2024/01)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 30.06.2022 CN 202210765063

(71) Applicant: Gan & Lee Pharmaceuticals Co., Ltd.
Beijing 101109 (CN)

(72) Inventors:
- XU, Fuming
  Beijing 101109 (CN)
- CHEN, Wenmin
  Beijing 101109 (CN)
- YUAN, Hang
  Beijing 101109 (CN)
- GAO, Zhigang
  Beijing 101109 (CN)
- LI, Xiaoguang
  Beijing 101109 (CN)
- WANG, Kanghua
  Beijing 101109 (CN)

(74) Representative: Kraus & Lederer PartGmbB
Thomas-Wimmer-Ring 15
80539 München (DE)

Remarks:
The complete document including Reference Table(s) and the Sequence Listing(s) can be downloaded from the EPO website

# (54) COMPOUND FUNCTIONING AS SMARCA2/4 INHIBITOR AND USE THEREOF

(57) Disclosed are a compound or a pharmaceutically acceptable salt thereof which function as a SMARCA2/4 inhibitor, said compound having the structural formula shown in formula I. Further disclosed are a pharmaceutical composition containing said compound, the use of said compound, and the use of said pharmaceutical composition. The compound provided by the present invention has improved inhibitory activity on SMARCA2/4, and may act as a SMARCA2/4 dual inhibitor.

EP 4 549 431 A1

## Description

## FIELD OF THE INVENTION

The present invention relates to the field of medicine and in particular relates to a compound used as a SMARCA2/4 inhibitor and a pharmaceutically acceptable salt thereof, which can be used for the treatment of disorders resulting from SMARCA2/4 mutations or deletions.

## BACKGROUND

SMARCA2/4 are important subunits of the SWI/SNF chromatin remodeling complex, also known as the BAF complex. Chromatin is the carrier of eukaryotic genetic information, and comprises DNA and nucleosomes, usually present a highly dense state. When a gene at a specific site needs to be transcribed, chromatin changes from a dense to a lax state, and this process requires the involvement of chromatin remodelling complexes. Chromatin remodelling complexes use the energy of ATP hydrolysis to relocate, assemble, migrate and reorganise nucleosomes, altering the chromatin structure, wherein the 'accessibility' of local DNA to transcription factors and cellular proteins. When the chromatin structure tends to be looser under the action of chromatin remodelling factors, the accessibility of RNA polymerase II and transcription factors to chromatin DNA is increased, then initiating the transcription of genes; on the contrary, when the chromatin structure tends to be denser, the accessibility of RNA polymerase II and transcription factors, etc. to chromatin DNA is weakened, and the transcription of the relevant genes is inhibited. As an important element of epigenetic regulation , chromatin remodelling plays an important role in eukaryotic DNA replication, transcription, recombination and DNA repair processes, which in turn control cell proliferation, division and maturation. SWI/SNF can alter the de-assembly of chromatin and the substitution of nucleosome stabilizing proteins, thereby regulating gene expression. SMARCA4 (encoding BRG1 protein) and SMARCA2 (encoding BRM protein) are two mutually exclusive catalytic subunits that comprise the SWI/SNF complex, both share 75% homology at the protein level, and have ATPase activity that provides the energy for chromatin remodelling. The ATP-dependent chromatin remodelling activity of SMARCA2 or SMARCA4 is necessary, mainly involved in double-strand breaks and nucleotide excision repair, and plays an important role in double-strand damage repair. Thus, SMARCA mutations can lead to uncorrectable or inappropriate repair of DNA damage and drivers of tumourigenesis and progression. Although SMARCA2 and SMARCA4 are both ATP hydrolases, there are differences in their functions, and the mutation spectra in malignant tumours are markedly different, with rare co-mutations. SMARCA2 deletions are relatively rare, occurring in rhabdomyosarcomas, oesophageal cancers, bladder cancers, and gastric cancers; whereas SMARCA4 is one of the genes frequently mutated in malignant tumours, and is frequently mutated in clear-cell carcinomas of ovary, renal cancers, haematological tumours and medulloblastoma, but usually not accompanied by SMARCA2 mutations. SMARCA2 is upregulated in most SMARCA4-deficient tumours to complement the loss of function of SMARCA4. Mutations in SMARCA4 make cell proliferation more dependent on the replaceable ATPase SMARCA2, therefore making these tumour cells susceptible to SMARCA2 deficiency. Selective inhibition of SMARCA2 activity has been shown to be an effective treatment for the growth of SMARCA4 mutant cancer cells, a relationship known as synthetic lethality. In vitro and mouse xenograft studies, knockdown of SMARCA2 protein expression using RNAi inhibits the growth of SMARCA4 mutant lung cancer tumours.

SMARCA proteins are multi-domain proteins, in addition to the conserved ATPase catalytic domain, containing a bromodomain that helps the complex bind to chromatin-specific sites by recognizing residues acetylated in the histone "tail". Most of the SMARCA2 inhibitors currently in development target the bromodomain, with a few studies targeting the core ATPase catalytic domain. Although it was found that the synthetic lethal effect of SMARCA2 with SMARCA4 can be achieved by RNAi knockdown of SMARCA2 or by inhibiting ATPase activity, however, small molecule inhibitors targeting the bromodomain are not effective in inhibiting tumor cell proliferation. Therefore, the SMARCA2 target comprises a bromodomain that is easy to target but does not play a major function, and an ATPase domain that plays a major function but is more difficult to target, which is a structural feature that makes it difficult to develop inhibitors targeted SMARCA2.

SMARCA2/4 and PB1, as SWI/SNF chromatin remodeling complexes, together constitute the VIII bromodomain. PFI-3(1) developed by Pfizer and GSK binds to the active pocket of the VIII bromodomain by substituting four conserved water molecules. However, the compound did not show better selectivity for SMARCA2/4/PB1. Moreover, it did not show significant inhibitory activity in 148 cancer cells. Most importantly, the poor chemical stability of the compound with aryl ketone linked to tertiary amines limited its intracellular exposure time (Current Opinion in Chemical Biology, 2016, 33, 58-66; 10.1021/acs.jmedchem; 660001210.1021/acs.jmedchem.0c01242). Luo's group found novel SMARCA2 inhibitors through high-throughput screening, which showed good selectivity against SMARCA2, but their pKD values still need to be improved. (Acta Pharmacol. Sin. 2018, 39, 1544-1552.).

## SUMMARY OF THE INVENTION

In view of the defects of the above-mentioned prior art inhibitor compounds for SMARCA2/4, which have poor stability and activity, the present invention provides a novel compound used as an inhibitor of SMARCA2/4. It has been verified that, compared to existing compounds, the novel inhibitor compounds provided by the present invention exhibit higher inhibitory activity.

One aspect of the invention provides a compound having a structure shown by formula $I_A$ or a pharmaceutically acceptable salt thereof,

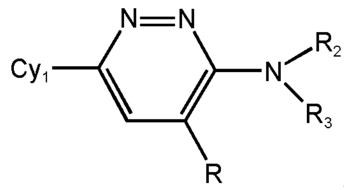

;

$I_A$

wherein,

$Cy_1$ is saturated or unsaturated cycloalkyl, saturated or unsaturated heteroalkyl containing one or more heteroatoms selected from N, O, and S, aryl, or heteroaryl containing one or more heteroatoms selected from N, O, and S, which is unsubstituted or substituted by one or more substituents independently selected from halo, cyano, haloalkyl, haloalkoxy, alkyl, hydroxy, amino, and alkyl amino; preferably, $Cy_1$ is saturated or unsaturated 3- to 10-membered cycloalkyl, saturated or unsaturated heteroalkyl containing 1 to 3 heteroatoms independently selected from N, O, and S, 6-to 10-membered aryl, or 5- to 10-membered heteroaryl containing 1 to 3 heteroatoms independently selected from N, O, and S, which is unsubstituted or substituted by one or more substituents independently selected from halo, cyano, $C_1$-$C_6$ haloalkyl, $C_1$-$C_6$ haloalkoxy, $C_1$-$C_6$ alkyl, hydroxy, amino, and $C_1$-$C_6$ alkylamino;

R is

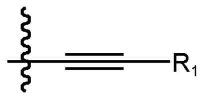

or

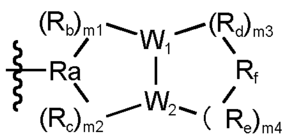

, and the wavy line represents the point of attachment;

$R_a$ is $CR_{aa}$ or N;

$R_f$ is selected from $C(R_{aa})_2$, CO, O, S, and $NR_{aa}$;

$W_1$ and $W_2$ are each independently selected from $CR_{aa}$ and N;

$R_d$, $R_c$, $R_d$, and $R_e$ are each independently, at each occurrence, selected from $C(R_{aa})_2$, CO, O, S, and $NR_{aa}$;

$R_{aa}$ is each independently, at each occurrence, selected from H, deuterium, halo, alkyl, deuterated alkyl, heteroalkyl, alkenyl, alkynyl, alkoxy, hydroxy, cycloalkyl, heterocyclyl, aryl, heteroaryl, - $N(R_g)_2$, cyano, $-C(O)-N(R_g)_2$, $-S(O)-N(R_g)_2$, $-S(O)_2-N(R_g)_2$, $-O-R_g$, $-S-R_g$, $-O-C(O)-R_g$, $-C(O)-R_g$, - $C(O)-OR_g$, $-S(O)-R_g$, $-S(O)_2-R_g$, $-N(R_g)-C(O)-R_g$, $-N(R_g)-S(O)-R_g$, $-N(R_g)-C(O)-N(R_g)_2$, and $-N(R_g)-S(O)_2-R_g$, and nitro, wherein optionally, said alkyl, heteroalkyl, alkenyl, alkynyl, alkoxy, cycloalkyl, heterocyclyl, aryl, and heteroaryl are each independently substituted with one or more substituents selected from halo, oxo (=O), alkyl, alkenyl, alkynyl, alkoxy, haloalkyl, haloalkoxy, hydroxyalkyl, cycloalkyl, heterocyclyl, aryl, heteroaryl, $-N(R_g)_2$, cyano, $-C(O)-N(R_g)_2$, $-S(O)-N(R_g)_2$, $-S(O)_2-N(R_g)_2$, $-O-R_g$, $-S-R_g$, $-O-C(O)-R_g$, $-C(O)-R_g$, $-C(O)-OR_g$, $-S(O)-R_g$, $-S(O)_2-R_g$, $-N(R_g)-C(O)-R_g$, $-N(R_g)-S(O)-R_g$, $-N(R_g)-C(O)-N(R_g)_2$, $-N(R_g)-S(O)_2-R_g$, and nitro; preferably, $R_{aa}$ is independently, at each occurrence, selected from H, deuterium, halo, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxy, $C_1$-$C_6$ hydroxyalkyl, $-C(O)-C_1$-$C_6$ alkyl, nitro, cyano, $C_3$-$C_{10}$ cycloalkyl, $C_3$-$C_{10}$ hetero-

cyclyl, $C_6$-$C_{10}$ aryl, $C_5$-$C_{10}$ heteroaryl, -NHR$_g$, benzyloxycarbonyl (Cbz), carboxyl (-COOH), -N($R_g$)$_2$, -NH(CO)$R_g$, acetyloxy (AcO), and -N($R_g$)$_2$, wherein optionally, said $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_{10}$ cycloalkyl, $C_3$-$C_{10}$ heterocyclyl, $C_6$-$C_{10}$ aryl, and $C_5$-$C_{10}$ heteroaryl are each independently substituted with one or more substituents selected from halo, $C_1$-$C_6$ alkyl, $C_1$-$C_6$ heteroalkyl, $C_1$-$C_6$ alkoxy, $C_1$-$C_6$ haloalkyl, hydroxy, $C_1$-$C_6$ hydroxyalkyl, cyano, nitro, $C_3$-$C_{10}$ cycloalkyl, $C_3$-$C_{10}$ heterocyclyl, $C_6$-$C_{10}$ aryl, $C_5$-$C_{10}$ heteroaryl, carboxyl (-COOH), -NH(CO)$R_g$, acetyloxy (AcO), and -N($R_g$)$_2$; preferably, $R_{aa}$ is independently, at each occurrence, selected from H, deuterium, halo, hydroxy, $C_1$-$C_6$ alkyl, $C_1$-$C_6$ hydroxyalkyl, $C_1$-$C_6$ alkoxy, $C_1$-$C_6$ haloalkyl, $C_1$-$C_6$ haloalkoxy, cyano, nitro, benzyloxycarbonyl (Cbz), carboxyl (-COOH), NH(CO)$R_g$, acetyloxy (AcO), and -N($R_g$)$_2$, wherein optionally, said $C_1$-$C_6$ alkyl and $C_1$-$C_6$ alkoxy are substituted with one or more substituents independently selected from halo, $C_1$-$C_6$ alkyl, $C_1$-$C_6$ heteroalkyl, $C_1$-$C_6$ alkoxy, $C_1$-$C_6$ haloalkyl, hydroxy, $C_1$-$C_6$ hydroxyalkyl, cyano, nitro, $C_3$-$C_8$ cycloalkyl, $C_3$-$C_{10}$ heterocyclyl, $C_6$-$C_{10}$ aryl, $C_5$-$C_{10}$ heteroaryl, carboxyl (-COOH), -NH(CO)$R_g$, acetyloxy (AcO), and -N($R_g$)$_2$.

m1, m2, m3, and m4 are each independently, at each occurrence, selected from 0, 1, 2, 3, 4, and 5;

$R_1$ is selected from H, deuterium, halo, alkyl, deuterated alkyl, heteroalkyl, alkenyl, alkynyl, alkoxy, hydroxy, cycloalkyl, heterocyclyl, aryl, heteroaryl, -N($R_g$)$_2$, cyano, -C(O)-N($R_g$)$_2$, -S(O)-N($R_g$)$_2$, -S(O)$_2$-N($R_g$)$_2$, -O-$R_g$, -S-$R_g$, -O-C(O)-$R_g$, -C(O)-$R_g$, -C(O)-O$R_g$, -S(O)-$R_g$, -S(O)$_2$-$R_g$, - N($R_g$)-C(O)-$R_g$, -N($R_g$)-S(O)-$R_g$, -N($R_g$)-C(O)-N($R_g$)$_2$, -N($R_g$)-S(O)$_2$-$R_g$, and nitro, wherein optionally, said alkyl, heteroalkyl, alkenyl, alkynyl, alkoxy, cycloalkyl, heterocyclyl, aryl, and heteroaryl are each independently substituted with one or more substituents independently selected from halo, oxo (=O), alkyl, alkenyl, alkynyl, alkoxy, haloalkyl, haloalkoxy, hydroxyalkyl, cycloalkyl, heterocycloalkyl, aryl, heteroaryl, -N($R_g$)$_2$, cyano, -C(O)-N($R_g$)$_2$, -S(O)-N($R_g$)$_2$, -S(O)$_2$-N($R_g$)$_2$, -O$R_g$, -S-$R_g$, -O-C(O)-$R_g$, -C(O)-$R_g$, -C(O)-O$R_g$, -S(O)-$R_g$, -S(O)$_2$-$R_g$, -N($R_g$)-C(O)-$R_g$, -N($R_g$)-S(O)-$R_g$, -N($R_g$)-C(O)-N($R_g$)$_2$, -N($R_g$)-S(O)$_2$-$R_g$, and nitro; preferably, $R_1$ is selected from H, deuterium, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, 3- to 15-membered cycloalkyl, 3- to 15-membered heterocycloalkyl, 6- to 14-membered aryl, and 5-to 14-membered heteroaryl, wherein said 3- to 15-membered cycloalkyl and 3- to 15-membered heterocycloalkyl are each independently monocyclic, fused-cyclic, bridged-cyclic, or spiro-cyclic group, said 6- to 14-membered aryl and 5- to 14-membered heteroaryl are independently monocyclic or fused-cyclic group, wherein optionally, said $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, 3- to 15-membered cycloalkyl, 3- to 15-membered heterocycloalkyl, 6- to 14-membered aryl, and 5- to 14-membered heteroaryl are substituted with one, two, or more substituents independently selected from halo, oxo (=O), $C_1$-$C_6$ alkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, $C_1$-$C_6$ hydroxyalkyl, 3- to 15-membered cycloalkyl, 3- to 15-membered heterocycloalkyl, 6- to 14-membered aryl, 5- to 14-membered heteroaryl, -N($R_g$)$_2$, cyano, -C(O)-N($R_g$)$_2$, -S(O)-N($R_g$)$_2$, -S(O)$_2$-N($R_g$)$_2$, -O-$R_g$, -S-$R_g$, -O-C(O)-$R_g$, -C(O)-$R_g$, -C(O)-O$R_g$, -S(O)-$R_g$, -S(O)$_2$-$R_g$, -N($R_g$)-C(O)-$R_g$, -N($R_g$)-S(O)-$R_g$, -N($R_g$)-C(O)-N($R_g$)$_2$, -N($R_g$)-S(O)$_2$-$R_g$, and nitro.

$R_g$ is each independently, at each occurrence, selected from H, deuterium, halo, alkyl, deuterated alkyl, heteroalkyl, alkenyl, alkynyl, alkoxy, haloalkyl, haloalkoxy, hydroxy, hydroxyalkyl, -C(O)-alkyl, cycloalkyl, heterocyclyl, aryl, heteroaryl, benzyloxycarbonyl (Cbz), wherein optionally, said alkyl, heteroalkyl, alkenyl, alkynyl, alkoxy, cycloalkyl, heterocyclyl, aryl, and heteroaryl are each independently substituted with one or more substituents selected from halo, alkyl, heteroalkyl, alkoxy, haloalkyl, hydroxy, hydroxyalkyl, cyano, amino, nitro, cycloalkyl, heterocyclyl, aryl, heteroaryl, carboxyl (-COOH), alkylamino, -NH(CO)H, -NH(CO)-alkyl, and acetyloxy (AcO); preferably, $R_g$ is each independently, at each occurrence, selected from H, deuterium, $C_1$-$C_6$ alkyl, $C_1$-$C_6$ heteroalkyl, $C_1$-$C_6$ alkoxy, $C_2$-$C_6$ alkenyl, $C_2$-$C_6$ alkynyl, $C_1$-$C_6$ haloalkyl, $C_1$-$C_6$ haloalkoxy, $C_1$-$C_6$ hydroxyalkyl, -C(O)-$C_1$-$C_6$ alkyl, $C_6$-$C_{10}$ aryl, $C_5$-$C_{10}$ heteroaryl, $C_3$-$C_{15}$ cycloalkyl, and $C_3$-$C_{15}$ heterocycloalkyl, wherein optionally, said $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_{15}$ cycloalkyl, $C_3$-$C_{15}$ heterocycloalkyl, $C_6$-$C_{10}$ aryl, and $C_5$-$C_{10}$ heteroaryl are each independently substituted with one or more substituents selected from halo, $C_1$-$C_6$ alkyl, $C_1$-$C_6$ heteroalkyl, $C_1$-$C_6$ alkoxy, $C_1$-$C_6$ haloalkyl, hydroxy, $C_1$-$C_6$ hydroxyalkyl, cyano, amino, nitro, $C_3$-$C_{15}$ cycloalkyl, $C_3$-$C_{15}$ heterocycloalkyl, $C_6$-$C_{10}$ aryl, $C_5$-$C_{10}$ heteroaryl, carboxyl (-COOH), $C_1$-$C_6$ alkylamino, - NH(CO)H, -NH(CO)-$C_1$-$C_6$ alkyl, and acetyloxy (AcO); and

$R_2$ and $R_3$ are each independently selected from H, alkyl, deuterium, F, Cl, Br, I, hydroxy, haloalkyl, hydroxyalkyl, alkoxy, and -C(=O)-alkyl; preferably, $R_2$ and $R_3$ are each independently selected from H, $C_1$-$C_6$ alkyl, deuterium, $C_1$-$C_6$ haloalkyl, $C_1$-$C_6$ hydroxyalkyl, $C_1$-$C_6$ alkoxy, and - C(=O)-$C_1$-$C_6$ alkyl.

In one of the embodiments, $Cy_1$ is aryl or heteroaryl, said aryl and heteroaryl are substituted with hydroxy, amino, or alkylamino, and optionally each independently being substituted with one or more substituents selected from halo, cyano, $C_1$-$C_6$ haloalkyl, $C_1$-$C_6$ haloalkoxy, and $C_1$-$C_6$ alkyl; preferably, $Cy_1$ is 6- to 10-membered aryl or 5- to 10-membered heteroaryl containing 1-3 heteroatoms each independently selected from N, O, and S, said 6- to 10-membered aryl and 5- to 10-membered heteroaryl are substituted with hydroxy, amino, or alkylamino, and optionally each independently being substituted with one or more substituents selected from halo, cyano, $C_1$-$C_6$ haloalkyl, $C_1$-$C_6$ haloalkoxy, and $C_1$-$C_6$ alkyl; preferably, $Cy_1$ is phenyl or 5- to 6-membered heteroaryl containing 1-3 heteroatoms each independently selected from N,

O, and S, said phenyl and 5- to 6-membered heteroaryl are substituted with hydroxy, amino, or alkylamino, and optionally each independently being substituted with one or more substituents selected from halo, cyano, $C_1$-$C_6$ haloalkyl, $C_1$-$C_6$ haloalkoxy, and $C_1$-$C_6$ alkyl; preferably, $Cy_1$ is phenyl or 5- to 6-membered heteroaryl containing 1-3 heteroatoms each independently selected from N, O, and S, said phenyl and 5- to 6-membered heteroaryl are substituted with hydroxy, amino, or alkylamino, and optionally each independently being substituted with one or more substituents selected from F, Cl, Br, cyano, trifluoromethyl, trifluoromethoxy, $C_1$-$C_3$ alkyl, and $C_1$-$C_3$ alkoxy; preferably, $Cy_1$ is phenyl or 5- to 6-membered heteroaryl containing 1-3 heteroatoms each independently selected from N, O, and S, said phenyl and 5- to 6-membered heteroaryl are substituted with at least one hydroxy, amino, or alkyl amino, preferably with one hydroxy, amino, or alkylamino; and/or

$R_1$ is selected from H, deuterium, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, 3- to 15-membered cycloalkyl, 3- to 15-membered heterocycloalkyl, 6- to 14-membered aryl, and 5- to 14-membered heteroaryl, wherein said 3- to 15-membered cycloalkyl and 3- to 15-membered heterocycloalkyl are each independently monocyclic, fused-cyclic, bridged-cyclic, or spiro-cyclic group, said 6- to 14-membered aryl and 5- to 14-membered heteroaryl are each independently monocyclic or fused-cyclic group, and said 3- to 15-membered heterocycloalkyl and 5- to 14-membered heteroaryl are each independently containing 1, 2, 3, 4, or 5 heteroatoms independently selected from N, O, and S, optionally, said $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, 3- to 15-membered cycloalkyl, 3- to 15-membered heterocycloalkyl, 6- to 14-membered aryl, and 5- to 14-membered heteroaryl are each independently substituted with one, two, or more substituents independently selected from F, Cl, Br, I, oxo (=O), hydroxy, $C_1$-$C_6$ alkyl, $C_1$-$C_6$ hydroxyalkyl, $C_1$-$C_6$ alkoxy, $C_1$-$C_6$ haloalkyl, $C_1$-$C_6$ haloalkoxy, amino, $C_1$-$C_6$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_6$ alkyl, 5- to 8-membered heterocycloalkyl containing 1, 2, or 3 heteroatoms independently selected from N, O, and S, and 9-fluorenylmethoxycarbonyl (Fmoc); and/or

$R_{aa}$ is each independently, at each occurrence, selected from H, deuterium, halo, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_6$ haloalkoxy, cyano, nitro, benzyloxycarbonyl (Cbz), carboxyl (-COOH), NH(CO)$R_g$, acetyloxy (AcO), and -N$(R_g)_2$, wherein optionally, said $C_1$-$C_3$ alkyl and $C_1$-$C_3$ alkoxy are each independently substituted with one or more substituents selected from halo, $C_1$-$C_3$ alkyl, $C_1$-$C_3$ heteroalkyl, $C_1$-$C_3$ alkoxy, $C_1$-$C_3$ haloalkyl, hydroxy, $C_1$-$C_3$ hydroxyalkyl, cyano, nitro, $C_3$-$C_6$ cycloalkyl, $C_3$-$C_6$ heterocycloalkyl, phenyl, $C_5$-$C_6$ heteroaryl, carboxyl (-COOH), -N(CO)$R_g$, acetyloxy (AcO), and -N$(R_g)_2$; and/or

$R_g$ is each independently, at each occurrence, selected from H, deuterium, $C_1$-$C_3$ alkyl, $C_1$-$C_3$ heteroalkyl, $C_1$-$C_3$ alkoxy, $C_2$-$C_3$ alkenyl, $C_2$-$C_3$ alkynyl, $C_1$-$C_3$ haloalkyl, $C_1$-$C_3$ haloalkoxy, $C_1$-$C_3$ hydroxyalkyl, -C(O)-$C_1$-$C_3$ alkyl, phenyl, $C_5$-$C_6$ heteroaryl, $C_3$-$C_6$ cycloalkyl, and $C_3$-$C_6$ heterocycloalkyl, wherein optionally, said $C_1$-$C_3$ alkyl, $C_1$-$C_3$ heteroalkyl, $C_2$-$C_3$ alkenyl, $C_2$-$C_3$ alkynyl, $C_1$-$C_3$ alkoxy, $C_3$-$C_6$ cycloalkyl, $C_3$-$C_6$ heterocyclyl, aryl, and $C_5$-$C_{10}$ heteroaryl are each independently substituted with one or more substituents selected from halo, $C_1$-$C_3$ alkyl, $C_1$-$C_3$ heteroalkyl, $C_1$-$C_3$ alkoxy, $C_1$-$C_3$ haloalkyl, hydroxy, $C_1$-$C_3$ hydroxyalkyl, cyano, amino, nitro, $C_3$-$C_6$ cycloalkyl, $C_3$-$C_6$ heterocyclokyl, $C_6$-$C_{10}$ aryl, $C_5$-$C_{10}$ heteroaryl, carboxyl (-COOH), $C_1$-$C_3$ alkyl amino, -NH(CO)H, -NH(CO)-$C_1$-$C_3$ alkyl, and acetyloxy (AcO); and/or

$R_2$ and $R_3$ are each independently selected from H, $C_1$-$C_3$ alkyl, deuterium, $C_1$-$C_3$ haloalkyl, $C_1$-$C_3$ hydroxyalkyl, $C_1$-$C_3$ alkoxy, and -C(=O)-$C_1$-$C_3$ alkyl.

In one of the embodiments,, said compound has a structure shown in formula I,

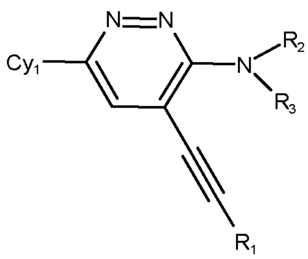

;

I

wherein, $Cy_1$ is aryl or heteroaryl, said aryl and heteroaryl are substituted with at least one hydroxy, amino, or alkylamino, and optionally being substituted with one or more substituents independently selected from halo, cyano, haloalkyl, haloalkoxy, alkyl, and alkoxy; preferably, $Cy_1$ is 6- to 10-membered aryl or 5- to 10-membered heteroaryl containing 1-3 heteroatoms independently selected from N, O, and S, said 6- to 10-membered aryl or 5- to 10-membered heteroaryl are substituted with at least one hydroxy, amino, or alkylamino, and optionally being substituted with one or more substituents independently selected from halo, cyano, $C_1$-$C_6$ haloalkyl, $C_1$-$C_6$ haloalkoxy, $C_1$-$C_6$ alkyl, and $C_1$-$C_6$ alkoxy.

$R_1$ is selected from H, $C_1$-$C_6$ alkyl, $C_2$-$C_6$ alkenyl, $C_2$-$C_6$ alkynyl, $C_1$-$C_6$ haloalkyl, $C_1$-$C_6$ haloalkoxy, and $C_1$-$C_6$ hydroxyalkyl; or $R_1$ is unsubstituted or substituted 3- to 15-membered cycloalkyl, or $R_1$ is 3- to 15-membered heterocycloalkyl containing 1-3 heteroatoms independently selected from N, O, and S, said 3- to 15-membered cycloalkyl and 3- to 15-membered heterocycloalkyl are each independently monocyclic, fused-cyclic, bridged-cyclic, or spiro-cyclic group; or $R_1$ is unsubstituted or substituted phenyl or an unsubstituted or substituted 8- to 10-membered aryl, said 8- to 10-membered aryl is a monocyclic or fused-cyclic group; or $R_1$ is unsubstituted or substituted 5- to 6-membered heteroaryl containing 1-3 heteroatoms independently selected from N, O, and S; or $R_1$ is unsubstituted or substituted 8- to 10-membered heteroaryl containing 1-3 heteroatoms independently selected from N, O, and S, said 8- to 10-membered heteroaryl is a monocyclic or fused-cyclic group; wherein said substituted 3- to 15-membered cycloalkyl, 3- to 15-membered heterocycloalkyl, phenyl, 8- to 10-membered aryl, 5- to 6-membered heteroaryl, and 8- to 10-membered heteroaryl are each independently substituted with one, two, or more substituents independently selected from halo, oxo (=O), $C_1$-$C_6$ alkyl, $C_2$-$C_6$ alkenyl, $C_2$-$C_6$ alkynyl, $C_1$-$C_6$ haloalkyl, $C_1$-$C_6$ haloalkoxy, $C_1$-$C_6$ hydroxyalkyl, 3- to 15-membered cycloalkyl, 3-to 15-membered heterocycloalkyl, $-N(R_g)_2$, cyano, $-C(O)-N(R_g)_2$, $-S(O)-N(R_g)_2$, $-S(O)_2-N(R_g)_2$, $-OR_g$, $-S-R_g$, $-O-C(O)-R_g$, $-C(O)-R_g$, $-C(O)-OR_g$, $-S(O)-R_g$, $-S(O)_2-R_g$, $-N(R_g)-C(O)-R_g$, $-N(R_g)-S(O)-R_g$, $-N(R_g)-C(O)-N(R_g)_2$, $-N(R_g)-S(O)_2-R_g$, and nitro; preferably, said substituted 3- to 15-membered cycloalkyl, 3- to 15-membered heterocycloalkyl, phenyl, 8- to 10-membered aryl, 5- to 6-membered heteroaryl, and 8- to 10-membered heteroaryl are each independently substituted with one, two, or more substituents independently selected from halo, oxo (=O), hydroxy, $C_1$-$C_6$ alkyl, $C_1$-$C_6$ hydroxyalkyl, $C_1$-$C_6$ alkoxy, $C_1$-$C_6$ haloalkyl, $C_1$-$C_6$ haloalkoxy, amino, $C_1$-$C_6$ alkyl amino, cyano, nitro, benzyloxycarbonyl (Cbz), carboxyl (-COOH), -NH(CO)-$C_1$-$C_6$ alkyl, acetyloxy (AcO), benzyl (Bn), tert-butoxycarbonyl (Boc), -(CO)H, 5- to 8-membered heterocycloalkyl containing one nitrogen atom, and 9-fluorenylmethoxycarbonyl (Fmoc).

$R_g$ is each independently, at each occurrence, selected from H, $C_1$-$C_6$ alkyl, $C_2$-$C_6$ alkenyl, $C_2$-$C_6$ alkynyl, $C_1$-$C_6$ haloalkyl, $C_1$-$C_6$ haloalkoxy, $C_1$-$C_6$ hydroxyalkyl, 3- to 15-membered cycloalkyl, and 3- to 15-membered heterocycloalkyl; preferably, $R_g$ is each independently, at each occurrence, selected from H, $C_1$-$C_3$ alkyl, $C_2$-$C_3$ alkenyl, $C_2$-$C_3$ alkynyl, $C_1$-$C_3$ haloalkyl, $C_1$-$C_3$ haloalkoxy, $C_1$-$C_3$ hydroxyalkyl, $C_3$-$C_8$ cycloalkyl, and $C_3$-$C_8$ heterocycloalkyl; and

$R_2$ and $R_3$ are each independently selected from H, alkyl, and -C(=O)-alkyl.

In one of the embodiments, wherein, $Cy_1$ is phenyl or 5- to 6-membered heteroaryl, said phenyl and 5- to 6-membered heteroaryl are substituted with at least one hydroxy, amino, or alkyl amino, and optionally being substituted with one or more substituents independently selected from halo, cyano, haloalkyl, haloalkoxy, alkyl, and alkoxy; preferably, $Cy_1$ is phenyl or 5- to 6-membered heteroaryl, said phenyl or heteroaryl are substituted with at least one hydroxy, and optionally being substituted with one or more substituents independently selected from halo, cyano, trifluoromethyl, trifluoromethoxy, $C_1$-$C_6$ alkyl, and $C_1$-$C_6$ alkoxy, preferably optionally being substituted with one or more substituents independently selected from F, Cl, Br, cyano, trifluoromethyl, trifluoromethoxy, $C_1$-$C_3$ alkyl, and $C_1$-$C_3$ alkoxy; preferably, $Cy_1$ is phenyl, and said phenyl is substituted with one hydroxy; and/or

$R_1$ is selected from H, $C_1$-$C_4$ alkyl, $C_2$-$C_4$ alkenyl, $C_2$-$C_4$ alkynyl, $C_1$-$C_4$ haloalkyl, $C_1$-$C_4$ haloalkoxy, and $C_1$-$C_4$ hydroxyalkyl; or $R_1$ is unsubstituted or substituted 3- to 15-membered cycloalkyl, or $R_1$ is 3- to 15-membered heterocycloalkyl containing 1-2 heteroatoms independently selected from N, O, and S, said 3- to 15-membered cycloalkyl and 3- to 15-membered heterocycloalkyl are each independently monocyclic, fused-cyclic or spiro-cyclic group; or $R_1$ isunsubstituted or substituted phenyl or an unsubstituted or substituted 10-membered aryl, said 10-membered aryl is a fused-cyclic group; or $R_1$ is substituted or unsubstituted 5- to 6-membered heteroaryl containing 1-2 heteroatoms independently selected from N, O, and S, or $R_1$ is substituted or unsubstituted 8- to 10-membered heteroaryl containing 1-3 heteroatoms independently selected from N, O, and S, said 8- to 10-membered heteroaryl is a fused-cyclic group; wherein, said substituted 3- to 15-membered cycloalkyl, 3- to 15-membered heterocycloalkyl, phenyl, 5- to 6-membered heteroaryl, 10-membered aryl, and 8- to 10-membered heteroaryl are each independently substituted with one, two, or more substituents independently selected from halo, oxo (=O), carboxyl, benzyloxycarbonyl (Cbz), acetyloxy (AcO), benzyl (Bn), tert-butoxycarbonyl (Boc), 5- to 8-membered heterocycloalkyl containing one nitrogen atom, 9-fluorenylmethoxycarbonyl (Fmoc), hydroxy, $C_1$-$C_6$ alkyl, $C_1$-$C_6$ hydroxyalkyl, $C_1$-$C_6$ alkoxy, $C_1$-$C_6$ haloalkyl, $C_1$-$C_6$ haloalkoxy, amino, $C_1$-$C_6$ alkyl amino, cyano, -(CO)H, -NH(CO)-$C_1$-$C_6$ alkyl, and nitro; preferably, said substituted 3-to 15-membered cycloalkyl, 3- to 15-membered heterocycloalkyl, phenyl, 5- to 6-membered heteroaryl, 10-membered aryl, and 8- to 10-membered heteroaryl are each independently substituted with one, two, or more substituents independently selected from Cl, Br, F, I, oxo, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_6$ alkyl, 5- to 6-membered heterocycloalkyl containing one nitrogen atom, and 9-fluorenylmethoxycarbonyl (Fmoc); preferably, said substituted 3- to 15-membered cycloalkyl, 3- to 15-membered heterocycloalkyl, phenyl, 5- to 6-membered heteroaryl, 10-membered aryl, and 8- to 10-membered heteroaryl are each independently substituted with one, two, or more substituents independently selected from Cl, Br, F, I, oxo, hydroxy, methyl, hydroxymethyl, methoxy, amino, dimethylamino, cyano, nitro, carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc); and/or

$R_2$ and $R_3$ are each independently H or $C_1$-$C_6$ alkyl; preferably, $R_2$ and $R_3$ are each independently H or $C_1$-$C_3$ alkyl; preferably, $R_2$ and $R_3$ are each independently H.

In one of the embodiments, said compound has a structure shown in formula II,

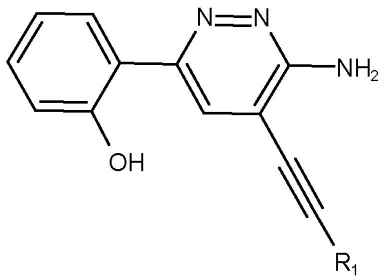

;

II

wherein,

$R_1$ is as defined in any one of claims 1, 2, 3, or 4;

preferably, $R_1$ is unsubstituted or substituted 4-, 5-, 6-, 7-, 8-, 9-, 10-, or 11-membered cycloalkyl, or $R_1$ is 4-, 5-, 6-, 7-, 8-, 9-, 10-, or 11-membered heterocycloalkyl containing 1-2 heteroatoms independently selected from N, O, and S, said cycloalkyl and heterocycloalkyl are each independently monocyclic, fused-cyclic, bridged-cyclic, or spiro-cyclic group; or $R_1$ is unsubstituted or substituted phenyl, or substituted or unsubstituted 10-membered aryl, said 10-membered aryl is a fused-cyclic group; or $R_1$ is substituted or unsubstituted 5- to 6-membered heteroaryl containing 1-2 heteroatoms independently selected from N, O, and S, or $R_1$ is substituted or unsubstituted 8- to 10-membered heteroaryl containing 1-2 heteroatoms independently selected from N, O, and S, said 8- to 10-membered heteroaryl is a fused-cyclic group; wherein, said substituted cycloalkyl, heterocycloalkyl, phenyl, 5- to 6-membered heteroaryl, 10-membered aryl, and 8- to 10-membered heteroaryl are each independently substituted with one, two, or more substituents independently selected from Cl, Br, F, I, oxo, carboxyl, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc), preferably, each independently substituted with one, two, or more substituents independently selected from Cl, Br, F, I, oxo, hydroxyl, methyl, hydroxymethyl, methoxy, amino, dimethylamino, cyano, nitro, carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc);

preferably, $R_1$ is unsubstituted or substituted 4-membered monocycloalkyl, 5-membered monocycloalkyl, 6-membered monocycloalkyl, 7-membered spiro-cycloalkyl, 8-membered spiro-cycloalkyl, 9-membered spiro-cycloalkyl, 10-membered spiro-cycloalkyl, 11-membered spiro-cycloalkyl, 8-membered fused-cycloalkyl, 9-membered fused-cycloalkyl, or 10-membered fused-cycloalkyl; or $R_1$ is 4-membered monocyclic heterocycloalkyl, 5-membered monocyclic heterocycloalkyl, 6-membered monocyclic heterocycloalkyl, 7-membered spiro-cyclic heterocycloalkyl, 8-membered spiro-cyclic heterocycloalkyl, 9-membered spiro-cyclic heterocycloalkyl, 10-membered spiro-cyclic heterocycloalkyl, 11-membered spiro-cyclic heterocycloalkyl, 8-membered fused-cyclic heterocycloalkyl, 9-membered fused-cyclic heterocycloalkyl, or 10-membered fused-cyclic heterocycloalkyl containing 1-2 heteroatoms independently selected from N, O, and S; or said substituted cycloalkyl and heterocycloalkyl are each independently substituted with one, two, or more substituents independently selected from Cl, Br, F, I, oxo, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc), preferably, each independently substituted with one, two, or more substituents independently selected from Cl, Br, F, I, oxo, hydroxy, methyl, hydroxymethyl, methoxy, amino, dimethylamino, cyano, nitro, carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc).

In one of the embodiments, $R_1$ is H, -$CH_3$, or -$C_4H_9$;
or $R_1$ is selected from the following groups optionally substituted with one or more substituents:

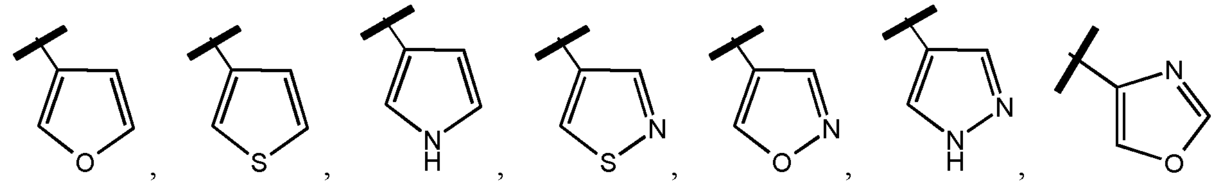
, , , , , , ,

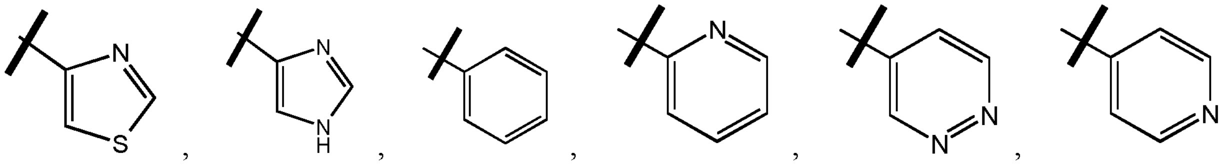
, , , , , ,

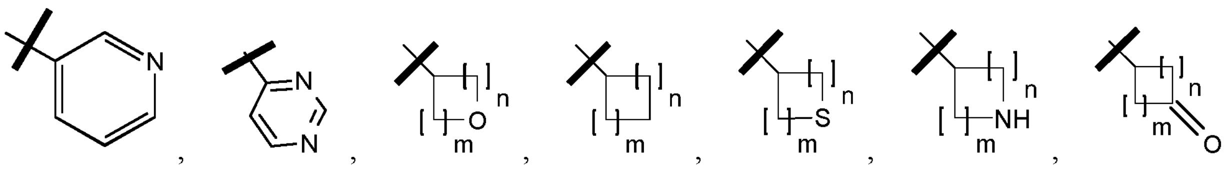
, , , , , , ,

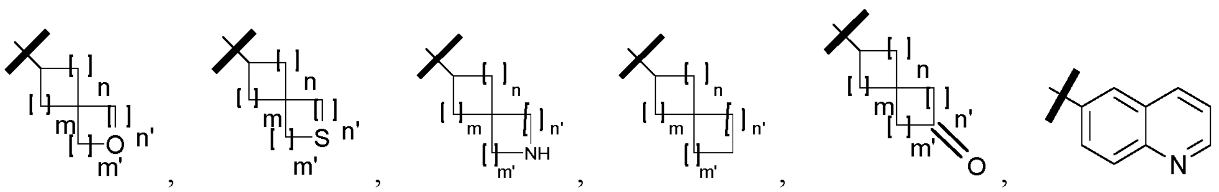
, , , , , ,

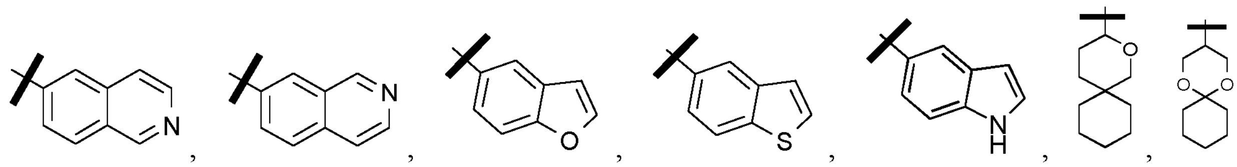
, , , , , , ,

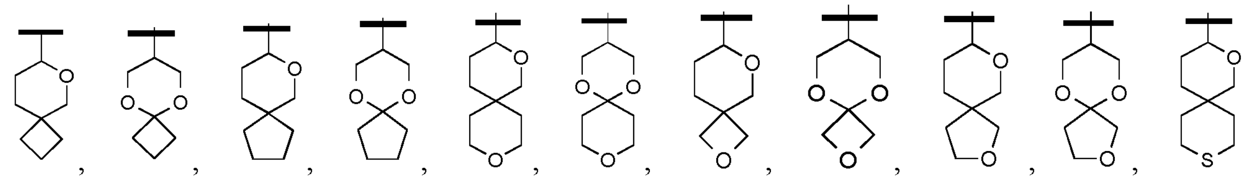
, , , , , , , , , , ,

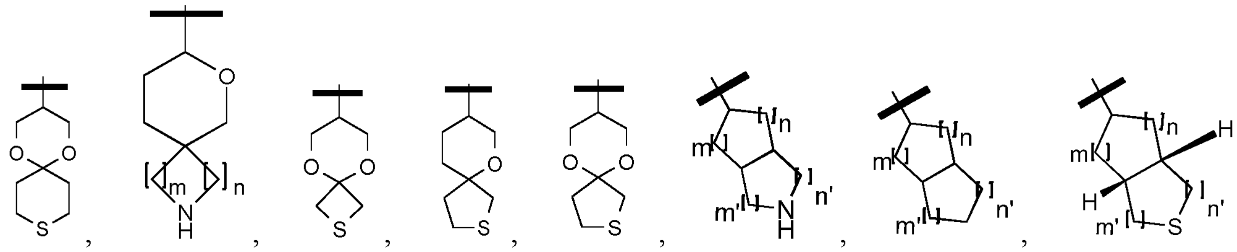
, , , , , , , ,

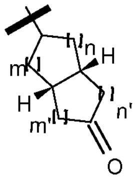, 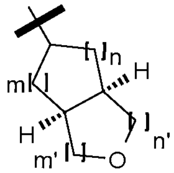, 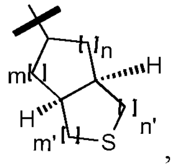, 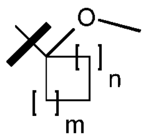, 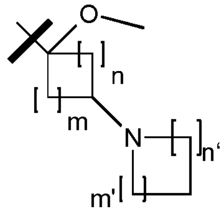, 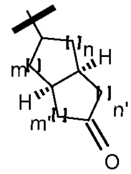, 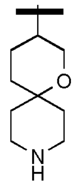,

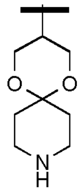, 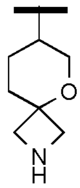, 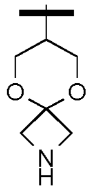, 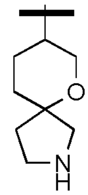 and 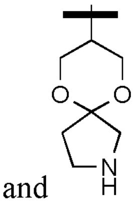;

wherein m, n, m', and n', at each occurrence, are each independently 1 or 2;

said one or more substituents are each independently selected from Cl, Br, F, I, oxo, carboxyl, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc); preferably, each independently selected from Cl, Br, F, I, oxo, hydroxy, methyl, hydroxymethyl, methoxy, amino, dimethylamino, cyano, nitro carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc); and

the bold bar line denotes the attachment point.

In one of the embodiments, $R_1$ is selected from the following groups optionally substituted with one or more substituents:

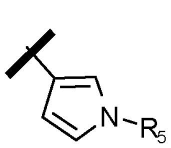, 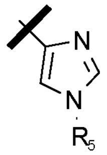, 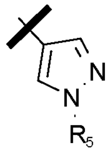, 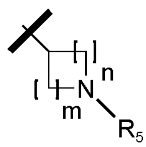, 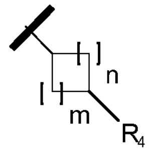, 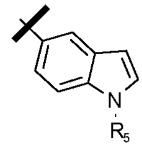, 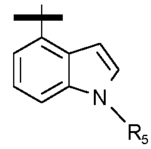,

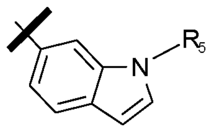, 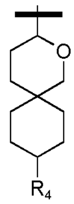, 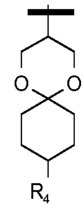, 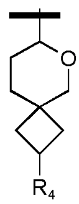, 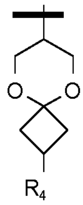, 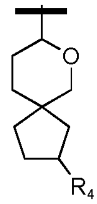, 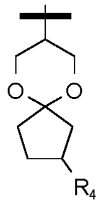, 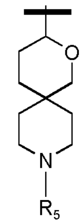, 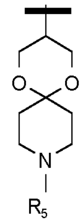,

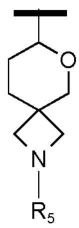, 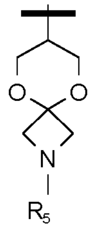, 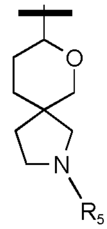, 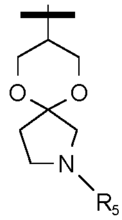, 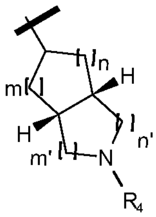, 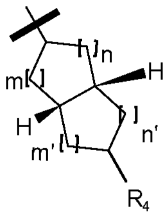, 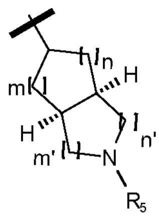

and

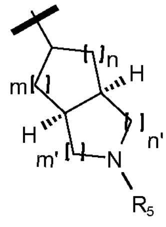 ;

wherein m, n, m', and n', at each occurrence, are each independently 1 or 2;
said one or more substituents are each independently selected from Cl, Br, F, I, oxo, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc); preferably, said one or more substituents are each independently selected from Cl, Br, F, I, oxo, hydroxy, methyl, hydroxymethyl, methoxy, amino, dimethylamino, cyano, nitro, carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc);
$R_4$ is, at each occurrence, independently selected from H, Cl, Br, F, I, oxo, carboxyl, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc); preferably, $R_4$ is, at each occurrence, independently selected from H, F, Cl, Br, I, -OH, oxo, methyl, amino (-$NH_2$), carboxyl (-COOH), methoxy, hydroxymethyl, dimethylamino, -(CO)H, -NH(CO)-$CH_3$, 5- to 6-membered heterocycloalkyl containing one nitrogen atom;
$R_5$ is, at each occurrence, independently selected from H, Cl, Br, F, I, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc); preferably, $R_5$ is, at each occurrence, independently selected from H, $C_1$-$C_3$ alkyl, acetyloxy (AcO), benzyl (Bn), benzyloxycarbonyl (Cbz), -(CO)H, tert-butoxycarbonyl (Boc), or 9-fluorenylmethoxycarbonyl (Fmoc); and
the bold bar line denotes the attachment point.

In one of the embodiments, said compound is selected from the following compounds:

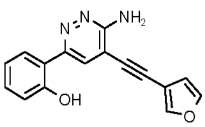 , 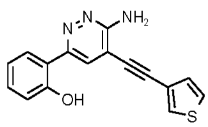 , 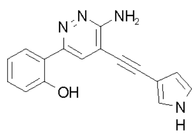 , 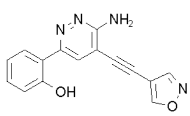 , 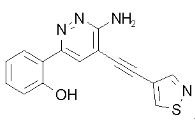 ,

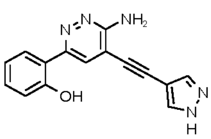 , 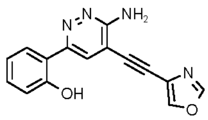 , 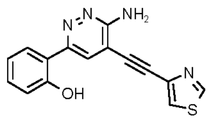 , 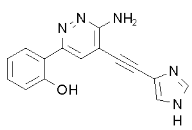 , 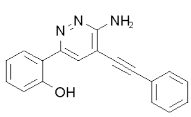 ,

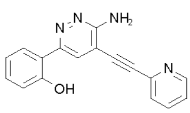 , 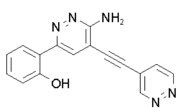 , 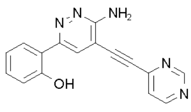 , 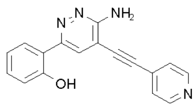 , 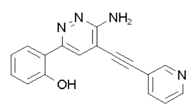 ,

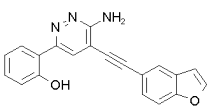 , 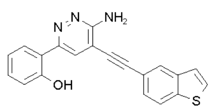 , 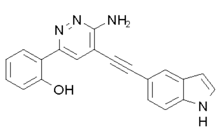 , 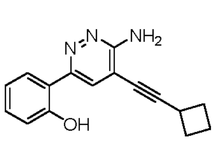 , 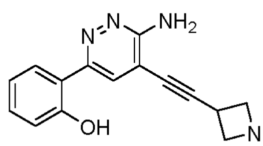 ,

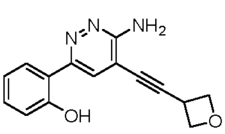, 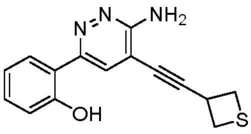, 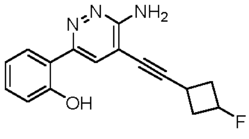, 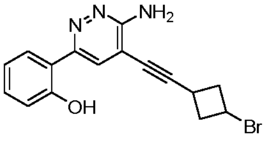,

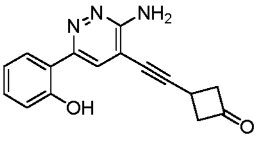, 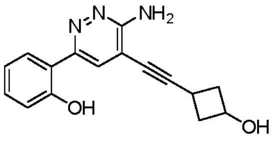, 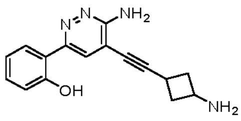, 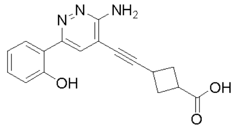,

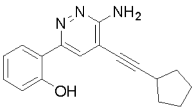, 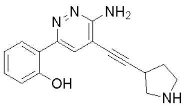, 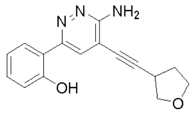, 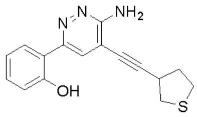, 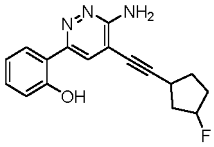,

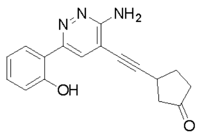, 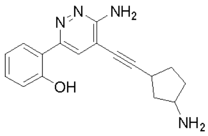, 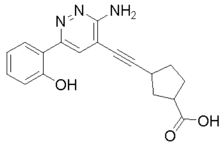, 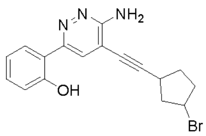, 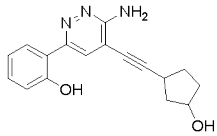,

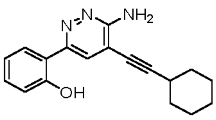, 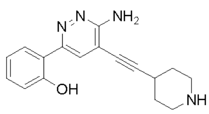, 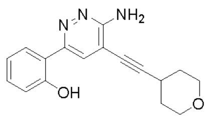, 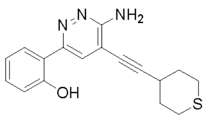, 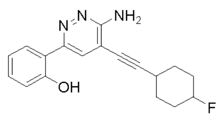,

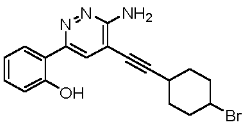, 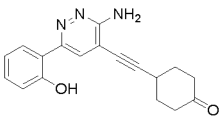, 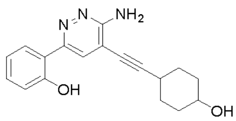, 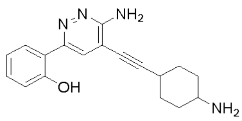,

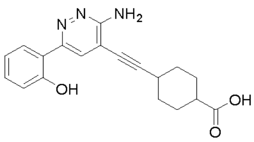, 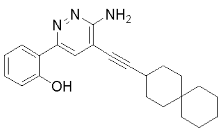, 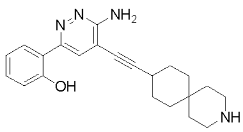, 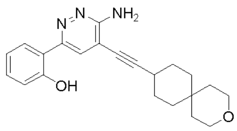,

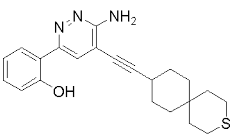, 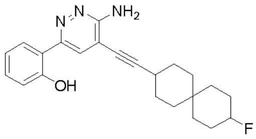, 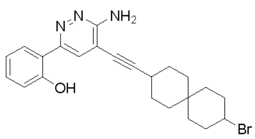, 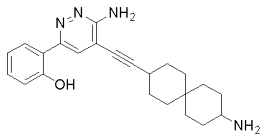,

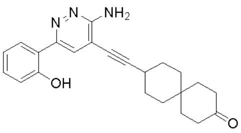, 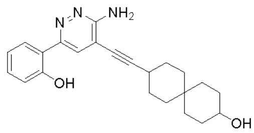, 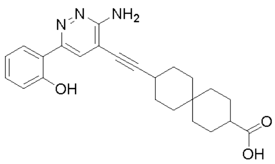, 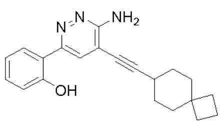,

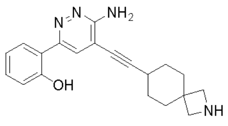 , 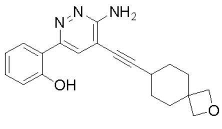 , 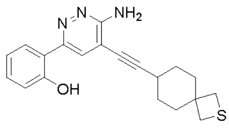 , 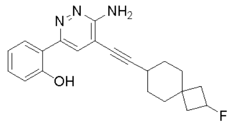 , 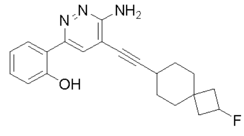 ,

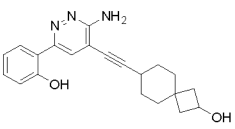 , 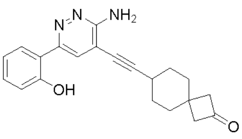 , 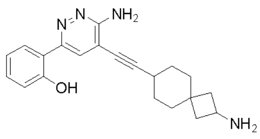 , 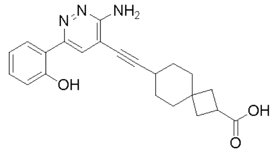 ,

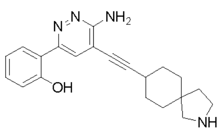 , 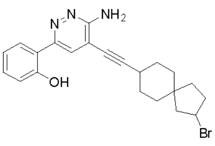 , 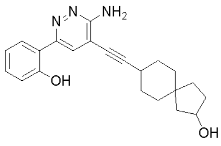 , 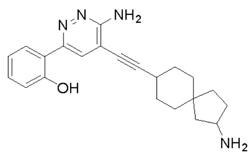 , 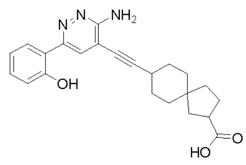 ,

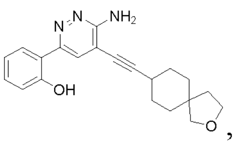 , 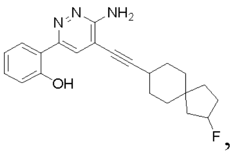 , 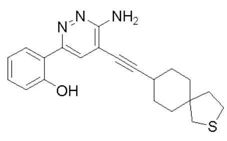 , 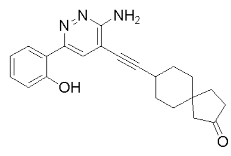 , 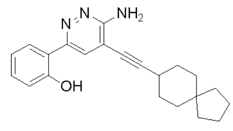 ,

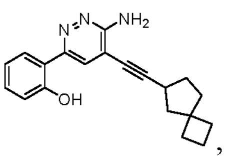 , 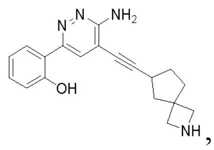 , 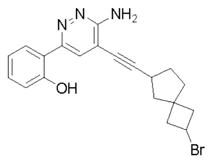 , 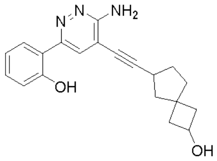 , 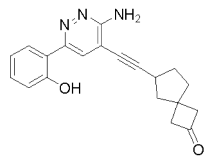 ,

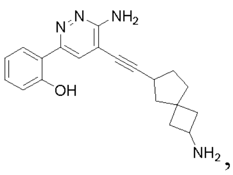 , 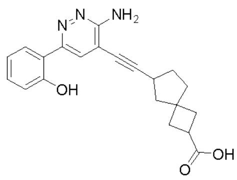 , 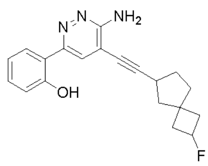 , 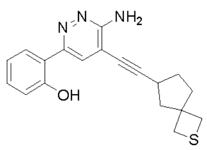 , 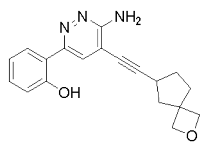 ,

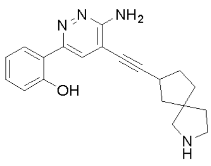 , 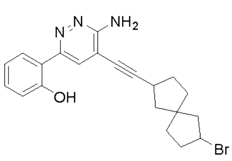 , 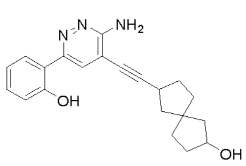 , 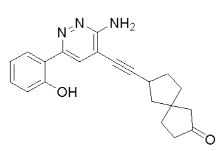 , 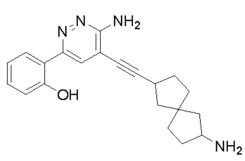 ,

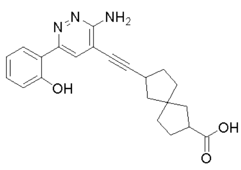 , 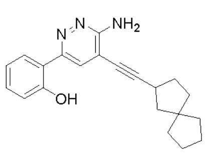 , 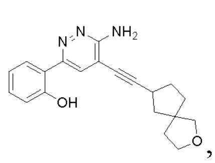 , 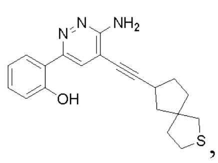 , 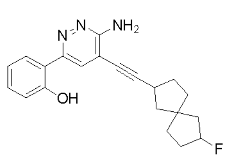 ,

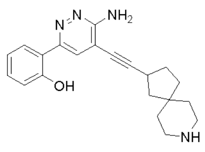 ,
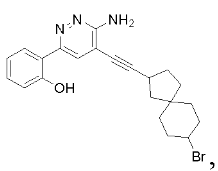 ,
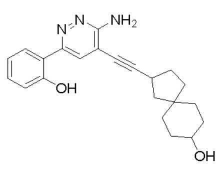 ,
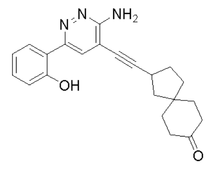 ,
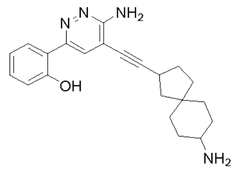 ,

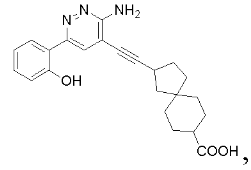 ,
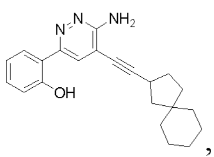 ,
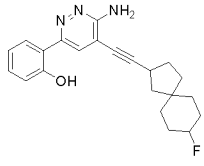 ,
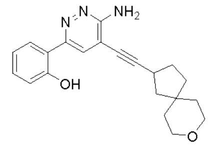 ,
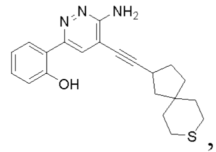 ,

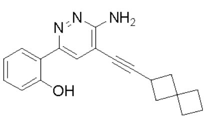 ,
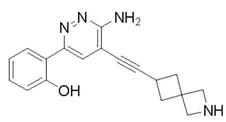 ,
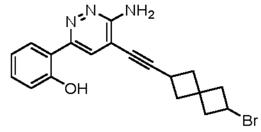 ,
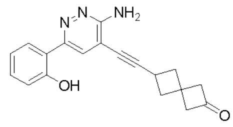 ,
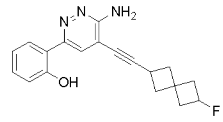 ,

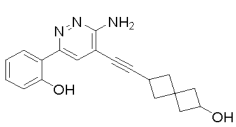 ,
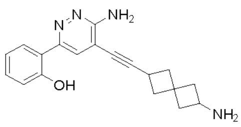 ,
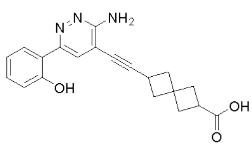 ,
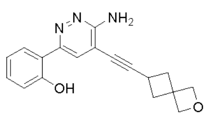 ,
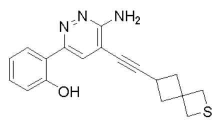 ,

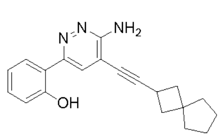 ,
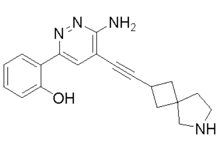 ,
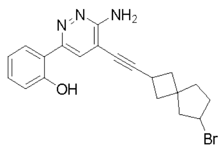 ,
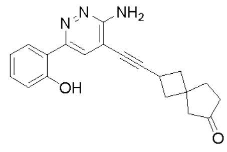 ,
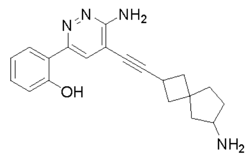 ,

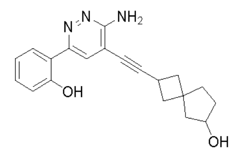 ,
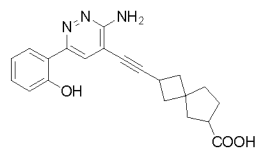 ,
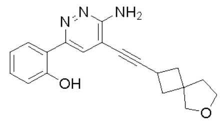 ,
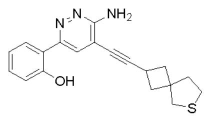 ,
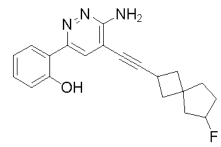 ,

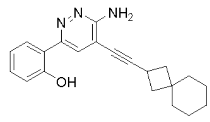 ,
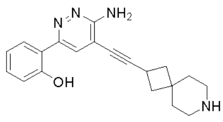 ,
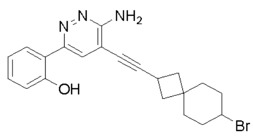 ,
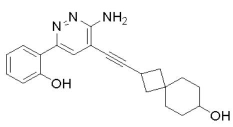 ,
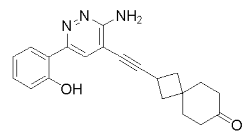 ,

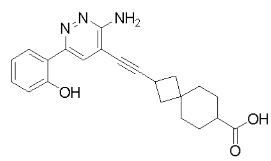 ,
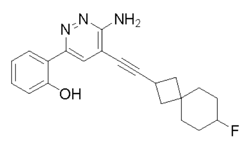 ,
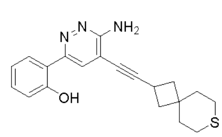 ,
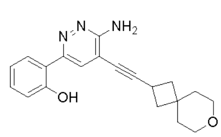 ,

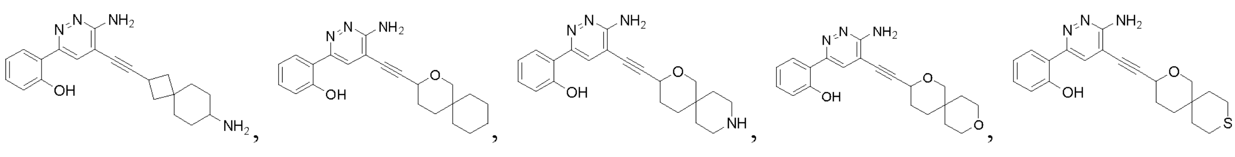 , , , , ,

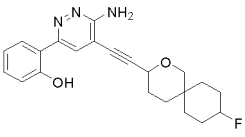, 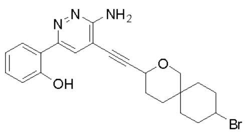, 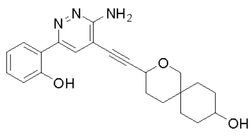, 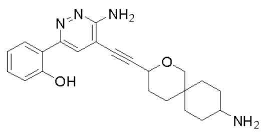,

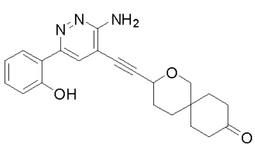, 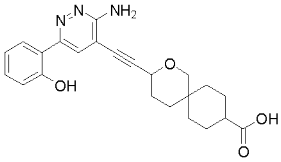, 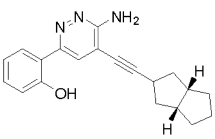, 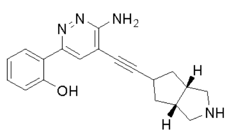,

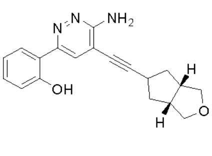, 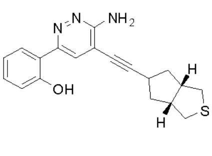, 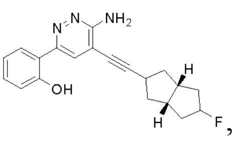, 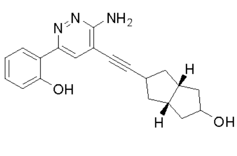, 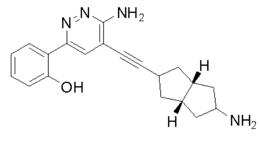,

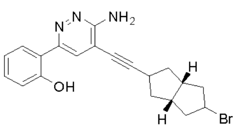, 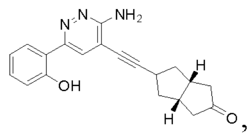, 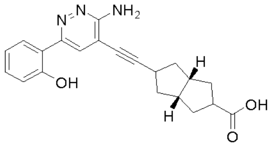, 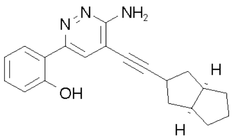,

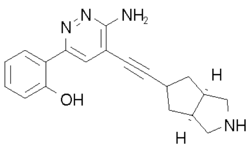, 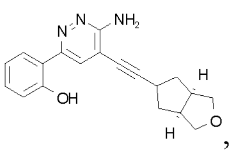, 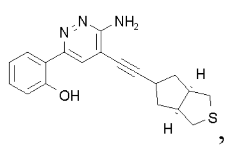, 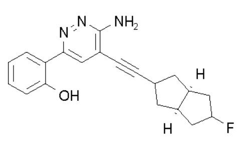,

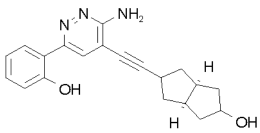, 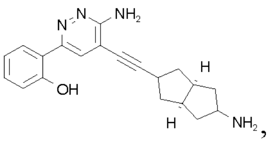, 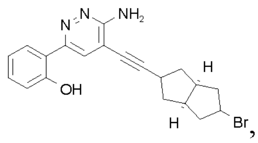, 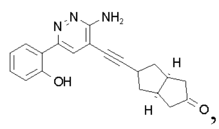,

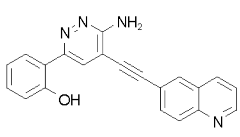, 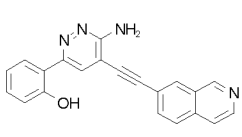, 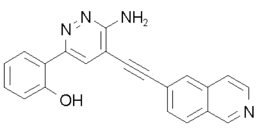, 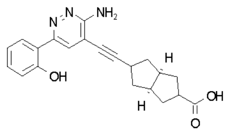,

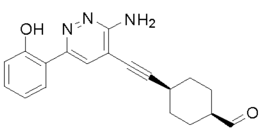, 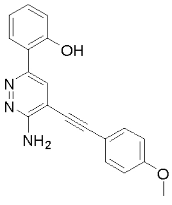, 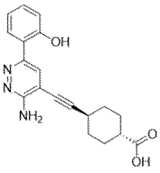, 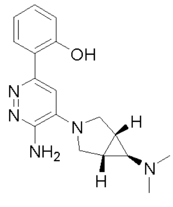, 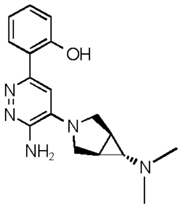,

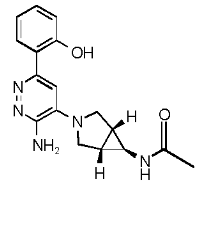, 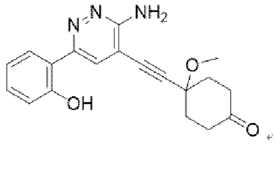, 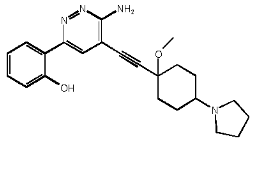, 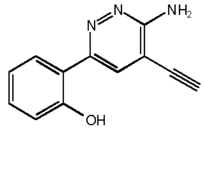,

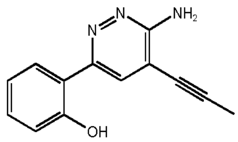, 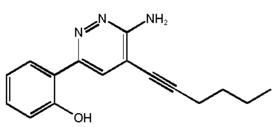,

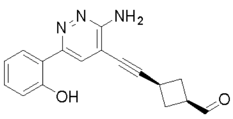, 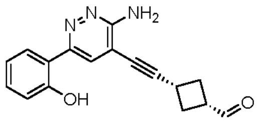, 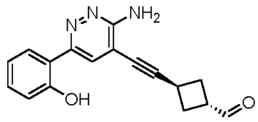, 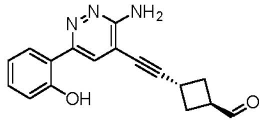,

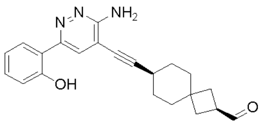, 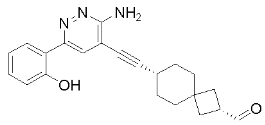, 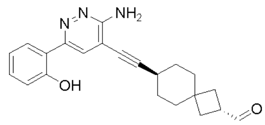, 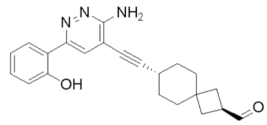,

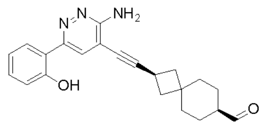, 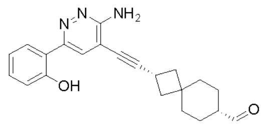, 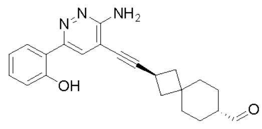, 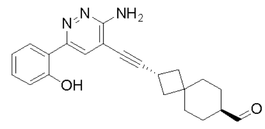

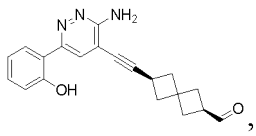, 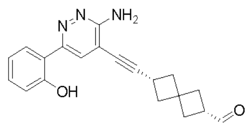, 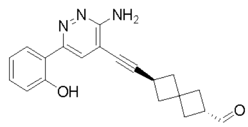, 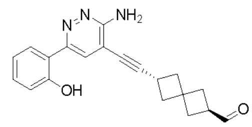,

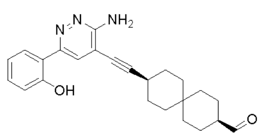, 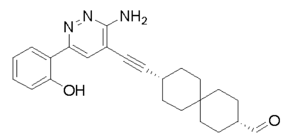, 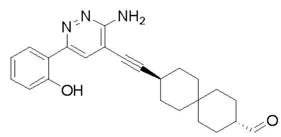, 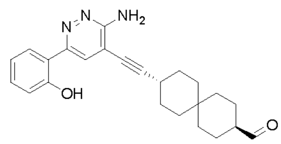,

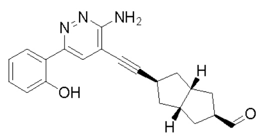, 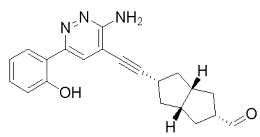, 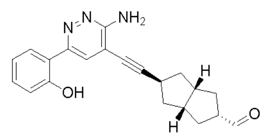, 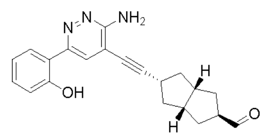,

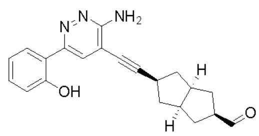, 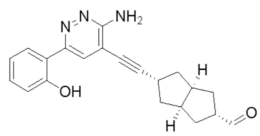, 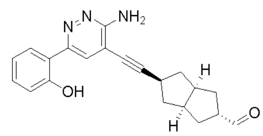, 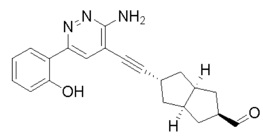,

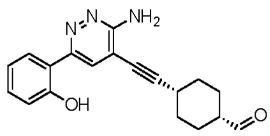, 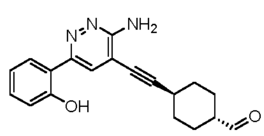 and 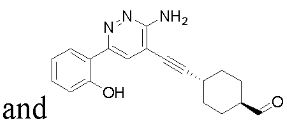.

The present invention also provides a pharmaceutical composition, characterized in that comprising a therapeutically effective amount of the compound of any one of claims 1-8 or a pharmaceutically acceptable salt thereof, and a pharmaceutically acceptable carrier.

The present invention also provides a use of any one of the compounds described hereinbefore or a pharmaceutically acceptable salt thereof, or the pharmaceutical composition described hereinbefore, in the preparation of a medicament for treating or preventing a disease or disorder mediated by SMARCA2/4.

In one of the embodiments, the disease or disorder mediated by SMARCA2/4 is cancer; preferably, the cancer is lung cancer, cervical cancer, and/or breast cancer.

The present invention also provides a compound or a pharmaceutically acceptable salt thereof described hereinbefore, or a pharmaceutical composition described herein before, for use in the treatment or prevention of a disease or disorder mediated by SMARCA2/4, preferably the disease or disorder is cancer; preferably the cancer is lung cancer, cervical cancer, and/or breast cancer.

The present invention also provides a method for treating or preventing a disease or disorder mediated by SMARCA2/4, comprising administering to a subject in need thereof a therapeutically effective amount of a compound described hereinbefore or a pharmaceutically acceptable salt thereof, or a pharmaceutical composition described hereinbefore; preferably the disease or disorder is cancer, and more preferably the cancer is lung cancer, cervical cancer, and/or breast cancer.

**Definitions and Detailed Description**

The compounds described in the present invention include stereoisomers, isotopic-markers, solvates, polymorphs or prodrugs thereof.

Certain compounds of the present invention can exist in non-solvated or solvated forms, including hydrated form. In general, solvated forms are comparable to non-solvated forms and both of them are included within the scope of the present invention. Solvated forms are generally equivalent to non-solvated forms and should be included within the scope of the present invention. Certain compounds of the present invention can exist in polymouphous or amorphous forms. In general, all physical forms are equivalent for the uses contemplated by the present invention and intended to be within the scope of the present invention.

The compounds of the present invention can exist in specific geometric or stereoisomeric forms. The present invention contemplates all such compounds, including cis and trans isomers, (-)- and (+)-enantiomers, (R)- and (S)-enantiomers, diastereoisomers, (D)-isomers, (L)-isomers, atropisomers (or also known as rotamers), etc., as well as racemic mixtures and other mixtures thereof, such as enriched in enantiomers or diastereomers, all of which are within the scope of the present invention.

The compounds of the present disclosure containing asymmetric carbon atoms can be isolated in optically active pureform or in racemic form. The optically active pure form can be resolved from the racemic mixture or synthesized by using chiral raw materials or chiral reagents.

The term "alkyl" as used herein refers to saturated aliphatic hydrocarbon group which is a straight or branched chain group containing 1 to 20 carbon atoms, preferably an alkyl group containing 1 to 12 (e.g., 1, 2, 3, 4, 5, 6, 7, 8, 9, 10, 11, and 12) carbon atoms, more preferably an alkyl group containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1, 2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 2,3-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl,2-methyl-3-ethylhexyl, 2,2-diethylpentyl, n-decyl, 3,3-diethylhexyl, 2,2-diethylhexyl, and various branched isomers thereof. More preferred are lower alkyl groups having 1 to 6 carbon atoms, and non-limiting embodiments of which include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group can be substituted or unsubstituted, and when substituted, the substituent can be substituted at any available attachment point, the substituent is preferably independently selected from one or more substituents selected from hydrogen atom, deuterium atom, halogen, alkyl, alkoxy, haloalkyl, hydroxy, hydroxyalkyl, cyano, amino, nitro, cycloalkyl, heterocycloalkyl, aryl, and heteroaryl.

The term "heteroalkyl" refers to an alkyl group in which one or more $-CH_2-$ are replaced by heteroatoms selected from NH, O and S or one or more -CH- are replaced by N atoms; wherein the alkyl is as defined above; the heteroalkyl can be substituted or unsubstituted, and when substituted, the substituent can be substituted at any available attachment point, the substituent is preferably independently s selected from one or more substituents selected from hydrogen atom, deuterium atom, halogen, alkyl, alkoxy, haloalkyl, hydroxy, hydroxyalkyl, cyano, amino, nitro, cycloalkyl, heterocycloalkyl, aryl and heteroaryl.

The term "alkoxy" refers to -O-(alkyl) and -O-(unsubstituted cycloalkyl), wherein the alkyl or cycloalkyl is as defined herein. Non-limiting examples of alkoxy include: methoxy, ethoxy, propoxy, butoxy, cyclopropyloxy, cyclobutyloxy, cyclopentyloxy, cyclohexyloxy. The alkoxy group can be optionally substituted or unsubstituted, and when substituted, the

substituent is preferably one or more of the following groups, which are independently selected from one or more substituents substituted with one or more of the following: hydrogen atom, deuterium atom, halogen, alkyl, alkoxy, haloalkyl, hydroxy, hydroxyalkyl, cyano, amino, nitro, cycloalkyl, heterocycloalkyl, aryl, heteroaryl.

The term "alkenyl" refers to an alkyl compound containing a carbon-carbon double bond in the molecule, wherein the alkyl is as defined above. Alkenyl can be substituted or unsubstituted, and when substituted, the substituent is preferably one or more of the following groups, which are independently selected from one or more of the substituents of hydrogen, alkyl, alkoxy, halogen, haloalkyl, hydroxy, hydroxyalkyl, cyano, amino, nitro, cycloalkyl, heterocycloalkyl, aryl and heteroaryl.

The term "alkynyl" refers to an alkyl compound containing a carbon-carbon triple bond in the molecule, wherein the alkyl is as defined above. The alkynyl group can be substituted or unsubstituted, and when substituted, the substituent is preferably one or more of the following groups, which are independently selected from one or more of the substituents of hydrogen, alkyl, alkoxy, halogen, haloalkyl, hydroxy, hydroxyalkyl, cyano, amino, nitro, cycloalkyl, heterocycloalkyl, aryl and heteroaryl.

The term "cycloalkyl" refers to saturated or partially unsaturated monocyclic, bicyclic or polycyclic hydrocarbon substituents, including, for example, monocyclic, fused-cyclic, bridged-cyclic, or spiro-cyclic group, and the cycloalkyl ring may contain 3 to 20 carbon atoms, preferably 3 to 12 carbon atoms, and more preferably 3 to 8 (e.g., 3, 4, 5, 6, 7,and 8) carbon atoms, more preferably 4 to 7 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.

The cycloalkylcan be substituted or unsubstituted. When substituted, the substituent may be substituted at any available attachment point, the substituent is preferably independently optionally substituted with one or more substituents selected from hydrogen, halogen, alkyl, alkoxy, haloalkyl, hydroxyl, hydroxyalkyl, cyano, amino, nitro, cycloalkyl, heterocycloalkyl, aryl, and heteroaryl.

The term "heterocycloalkyl" refers to a non-aromatic cyclic group in which one or more atoms constituing the ring are heteroatoms and the rest are carbon stoms, the heteroatoms including, but are not limited to, nitrogen atom, oxygen atom, sulfur atoms and the like. Preferred heterocycloalkyl is 3-10 membered saturated heterocycloalkyl. Unless otherwise specifically indicated in this specification, the heterocycloalkyl can be monocyclic ("monocyclic heterocycloalkyl"), or a bicyclic, tricyclic, or polycyclic heterocyclic ring, which comprise a fused (condensed), bridged (bridged-cyclic), or spiro-cyclic ring system (e.g., a bicyclic system ("bicyclic heterocycloalkyl")). The ring system of the heterocycloalkyl bicyclic ring may include one or more heteroatoms in one or two rings; and is saturated. Heterocycloalkyl groups include, but are not limited to, azetidinyl, epoxypropyl, thietanyl, oxetanyl, tetrahydrofuranyl, tetrahydropyranyl, or isomers and stereoisomers thereof.

Heterocycloalkyl can be substituted or unsubstituted, and when substituted, the substituent can be substituted at any available attachment point, the substituent is preferably independently substituted with one or more substituents optionally selected from hydrogen, halogen, alkyl, alkoxy, haloalkyl, hydroxyl, hydroxyalkyl, cyano, amino, nitro, cycloalkyl, heterocycloalkyl, aryl, and heteroaryl. The term "aryl" refers to a 6 to 14-membered all-carbon monocyclic or fused polycyclic (fused polycyclic is a ring sharing adjacent carbon atom pairs) group having a conjugated $\pi$-electronic system, preferably 6 to 10-membered, e.g. phenyl and naphthyl. The aryl ring comprises an aryl ring as described above fused to a heteroaryl, heterocycloalkyl or cycloalkyl ring, wherein the ring attached to the parent structure is an aryl ring. The aryl group can be substituted or unsubstituted, and when substituted, the substituent can be substituted at any available attachment point, the substituent is preferably independently substituted with one or more substituents selected from hydrogen, halogen, alkyl, alkoxy, haloalkyl, hydroxyl, hydroxyalkyl, cyano, amino, nitro, cycloalkyl, heterocycloalkyl, aryl, and heteroaryl.

The term "aryl" refers to a 6 to 14-membered all-carbon monocyclic or fused polycyclic (fused polycyclic is a ring sharing adjacent carbon atom pairs) group having a conjugated $\pi$-electronic system, preferably 6 to 10-membered, e.g. phenyl and naphthyl. The aryl ring comprises an aryl ring as described above fused to a heteroaryl, heterocyclyl or cycloalkyl ring, wherein the ring attached to the parent structure is an aryl ring. The aryl group can be substituted or unsubstituted, and when substituted, the substituent group can be substituted at any available attachment point, the substituent group is preferably independently optionally substituted with one or more substituents selected from hydrogen, halogen, alkyl, alkoxy, haloalkyl, hydroxyl, hydroxyalkyl, cyano, amino, nitro, cycloalkyl, heterocyclyl, aryl, and heteroaryl.

The term "heteroaryl" refers to a heteroaromatic system containing 1 to 4 (e.g., 1, 2, 3 and 4) heteroatoms, 5 to 14 ring atom, wherein the heteroatoms are selected from oxygen, sulfur and nitrogen. The heteroaryl is preferably 5 to 10 membered (e.g. 5, 6, 7, 8, 9 or 10 membered), more preferably 5 or 6 membered, such as furanyl, thienyl, pyridyl, pyrrolyl, N-alkylpyrrolyl, pyrimidyl, pyrazinyl, pyridazinyl, imidazolyl, pyrazolyl, triazolyl, tetrazolyl, and the like. The heteroaryl ring comprises a heteroaryl group as described above fused to an aryl, heterocycloalkyl or cycloalkyl ring, wherein the ring attached to the parent structure is a heteroaryl ring. Heteroaryl may be substituted or unsubstituted, and when substituted, the substituent may be substituted at any available attachment point, the substituent is preferably independently selected from one or more substituents of hydrogen, halogen, alkyl, alkoxy, haloalkyl, hydroxyl, hydroxyalkyl, cyano, amino, nitro,

cycloalkyl, heterocycloalkyl, aryl, and heteroaryl. The term "haloalkyl" means an alkyl group substituted with one or more halogen, wherein the alkyl group is as defined above.

The term "monocyclic" refers to a monocyclic group having one ring therein. The monocycle can be saturated or partially saturated.

The term "bridged-cyclic" refers to a polycyclic system that shares two or more carbon atoms, which can be divided into bicyclic bridging rings and polycyclic bridging rings. The former is composed of two aliphatic rings sharing two or more carbon atoms; the latter is a bridged ring composed of three or more rings.

The term "spiro-cyclic" refers to a polycyclic system in which two rings share one carbon atom (called the spiro atom).

The term "fused-cyclic" refers to a polycyclic structure formed by two or more cyclic rings sharing two adjacent ring atoms (i.e., sharing one bond).

The term "hydroxyalkyl" refers to an alkyl group substituted with one or more hydroxyl groups, wherein the alkyl group is as defined above.

The term "halogen" refers to fluorine, chlorine, bromine or iodine.

The term "amino" refers to $-NH_2$.

The term "cyano" refers to -CN.

The term "nitro" refers to $-NO_2$.

The term "target protein" refers to proteins and peptides that have any biological function or activity (including structural, regulatory, hormonal, enzymatic, genetic, immunological, contractile, storage, transport and signal transduction). In some embodiments, target proteins include structural proteins, receptors, enzymes, cell surface proteins, proteins associated with the integrated function of a cell, including proteins involved in each of: catalytic activity, aromatase activity, motility activity, helicase activity, metabolic processes (anabolic and catabolic), antioxidant activity, protein hydrolysis, biosynthesis, proteins with kinase activity, Oxidoreductase activity, transferase activity, hydrolase activity, lyase activity, isomerase activity, ligase activity, enzyme regulator activity, signal transducer activity, structural molecule activity, binding activity (proteins, lipids carbohydrates), receptor activity, cell motility, membrane fusion, cell communication, regulation of biological processes, development, cell differentiation, stimuli response, behavioral proteins, cell adhesion proteins, white matter involved in cell death, proteins involved in transporter (including protein transporter activity, nuclear transporter, ion transporter activity, channel transporter activity, carrier activity), permease activity, secretory activity, electron transport activity, pathogenesis, concomitant protein regulator activity, nucleic acid binding activity, transcriptional regulator activity, extracellular constitutive and biogenesis activity, and translational regulator activity. The proteins include proteins from eukaryotic and prokaryotic organisms, the eukaryotic and prokaryotic organisms include microorganisms, viruses, fungi, and parasites, and numerous others, including humans, microorganisms, viruses, fungi, and parasites as targets of drug therapy, and other animals including domesticated animals, microorganisms and other anti-microbial agents used to assay the target of an antibiotic, plants, and even viruses, and numerous others.

"Optional" or "optionally" refers to the subsequently described event or circumstance may but need not occur, and the description includes scenario where the event or circumstance occurs or does not occur. For example, "optionally substituted cyclopropyl" means that cyclopropyl may but need not be substituted, and the description includes scenarios where cyclopropyl is substituted or not substituted.

"Substituted" refers to one or more hydrogen atoms in the group, preferably no more than 5, more preferably 1 to 3 hydrogen atoms in the group are substituted independently of each other by a corresponding number of substituents. It is self-evident that the substituents are only in their possible chemical positions and that those skilled in the art is able to determine possible or impossible substitutions (by experiment or theory) without undue effort.

The term "pharmaceutically acceptable salt" means that a compound can be converted by conventional methods into a corresponding salt, which is chemically or physically compatible with other components composing a pharmaceutical dosage form and physiologically compatible with receptor. The salt can be acidic and/or basic salts formed by the compound with inorganic and/or organic acids and/or with inorganic and/or organic bases, and also comprise amphoteric salts (inner salts), quaternary ammonium salts, such as alkyl ammonium salts. These salts can be obtained directly in the final separation and purification of the compound. They can also be obtained by suitably mixing the compounds of the invention, or their stereoisomers or solvates, with a certain amount of acid or base. These salts may form a precipitate in the solution and be collected by filtering, or be recovered after the solvent evaporates, or be obtained by cooling and drying after reaction in an aqueous medium. Specifically, the salts are preferably water-soluble, pharmaceutically acceptable, non-toxic acid addition salts, such as salts formed by amines with inorganic acids (e.g., hydrochloric acid, hydrobromic acid, phosphoric acid, sulfuric acid, and perchloric acid), or with organic acids (e.g., acetic, oxalic, maleic, tartaric, citric, succinic, or malonic acids), or by using other methods (e.g., ionexchange) traditional in the art. The term "pharmaceutical composition" refers to a mixture of one or more of the compounds of the present application or pharmaceutically acceptable salts thereof with a pharmaceutically acceptable carrier. The pharmaceutical compositions are intended to facilitate administration of the compounds of the present application to an organism. The pharmaceutical compositions of the invention comprise one or more pharmaceutically acceptable salts, antioxidants, aqueous and non-aqueous carriers,

and/or adjuvants such as preservatives, wetting agents, emulsifiers and dispersants.

As used herein, "pharmaceutical compositions" can also be administered to a patient or subject in need of such treatment by any suitable mode of administration, such as oral, parenteral, rectal, transpulmonary or topical administration. When used for oral administration, said pharmaceutical compositions can be made into oral formulations, such as oral solid formulations, e.g., tablets, capsules, pills, granules and the like; or, oral liquid formulations, e.g., oral solutions, oral suspensions, syrups and the like. When made into oral preparations, said pharmaceutical preparations also comprise suitable fillers, binders, disintegrating agents, lubricants.

The term "pharmaceutically acceptable carrier" refers to those excipients that do not have a significant irritant effect on the organism and do not impair the biological activity and properties of the active compound. Suitable excipients are well known to those skilled in the art, such as carbohydrates, waxes, water-soluble and/or water-swellable polymers, hydrophilic or hydrophobic materials, gelatin, oils, solvents, water, liposomes, polymeric micelles or inorganic nanocarriers.

The pharmaceutical compositions of the present invention can be made in any pharmaceutically acceptable dosage form for oral, nasal, topical (including oral and sublingual), transrectal, transvaginal and/or parenteral administration, e.g., as tablets, lozenges, capsules, pills, solutions, suspensions, syrups, injections, suppositories, inhalers or sprays.

The term "effective amount" or "therapeutically effective amount" refers to an amount of an active ingredient (e.g., a compound) that, when administered to a subject for the treatment of a disease or at least one clinical symptom of a disease or disorder, is sufficient to affect such treatment of said disease, disorder or symptom. "Therapeutically effective amount" can vary with the compound, the disease, disorder and/or symptom of the disease or disorder, the severity of the disease, disorder and/or symptom of the disease or disorder, the age of the subject to be treated and/or the weight of the subject to be treated. In the embodiment, the appropriate amount is clear to one of skill in the art or be determined by routine experimentation. In some embodiments, "therapeutically effective amount" is an amount of at least one compound disclosed herein and/or at least one stereoisomer thereof and/or at least one pharmaceutically acceptable salt thereof that is effective to "treat" (as defined above) a disease or disorder in a subject. In the case of combination therapy, "therapeutically effective amount" refers to the total amount of a combination subject used to effectively treat a disease, disorder or condition.

## Examples

The following examples are directed to the intermediate compounds and final products identified in the specification and synthesis schemes. The preparation of the compounds of the present invention is described in detail using the following examples, but the chemical reactions described are disclosed according to their general applicability to the preparation of the compounds of the present invention. At times, the described reactions may not be applicable to every compound within the scope of the present invention as described. Those skilled in the art can readily recognize compounds where this occurs. In these cases, the reaction successfully carried out by conventional improvements known to those skilled in the art. In all preparation methods, all starting materials are known or can be readily prepared using known raw materials. The starting materials, chemical reagents, and solvents used in the present disclosure are all commercially available and purchased from companies such as Anergy Chemical, Shanghai Bide Pharma, Beijing Innochem, Jiangsu Aikon Biopharmaceutical, Sinopharm, Beijing J&K Scientific, Yunnan Xinlanjing, and others.

The structures of compounds synthesized in this application were all determined by nuclear magnetic resonance (NMR) and/or mass spectrometry (MS).

Nuclear magnetic resonance (NMR) was determined on a Bruke AVANCE-400/600 NMR using deuterated solvents, including deuterated dimethylsulfoxide (DMSO-$d_6$), deuterated chloroform ($CDCl_3$), and deuterated methanol ($CD_3OD$), and the internal standard was tetramethylsilane (TMS). Mass spectrometry (MS) measurements were performed on a Waters Acquity UPLC® Plus device.

High performance liquid chromatography for preparation was a Waters 2489 device.

The preparative medium-pressure liquid chromatography was a COMBIFLASH NEXTGEN 300+ device.

Silica gel 60 plates (aluminum plates coated with fluorescence) were used for thin layer chromatography (TLC).

Silica gel (100-200 mesh, 200-300 mesh) used for silica gel column chromatography was purchased from Innochem.

Reaction process detection in the embodiments was performed by thin layer chromatography (TLC), and the systems for the developing solvent used to monitor the reaction and the eluent used to purify the compounds by column chromatography included: petroleum ether/ethyl acetate system, dichloromethane/methanol system.

### Example 1: Preparation of 2-(6-amino-5-(piperidin-4-ylethynyl)pyridazin-3-yl)phenol (Compound 1c)

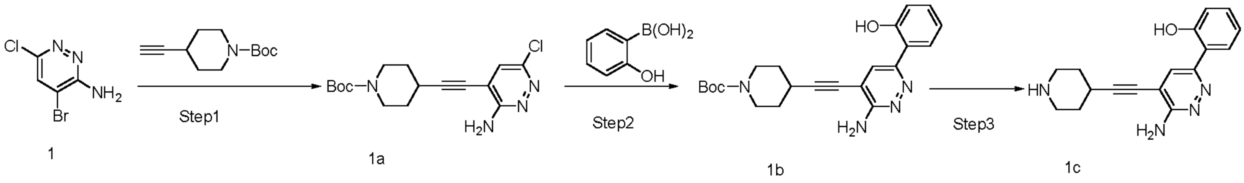


1) tert-Butyl 4-((3-amino-6-chloropyridazin-4-yl)ethynyl)piperidine-1-carboxylate

Under nitrogen protection, Compound 1 (100 mg, 1.0 eq), tert-butyl 4-ethynylpiperidine-1-carboxylate (120 mg, 1.2 eq), triethylamine (98 mg, 2.0 eq), cuprous iodide (9mg, 0.1 eq) and bis(triphenylphosphine)palladium(II) dichloride (34 mg, 0.1 eq) were dissolved in N,N-dimethylformamide (2 mL) and the reaction was raised to 115 °C for 4h. After the reaction was completed, the mixture was purified by preparative liquid phase to obtain a white solid 1a (102 mg, 63%).

$^1$H NMR (400 MHz, DMSO-$d_6$) δ 7.52 (s, 1H), 6.78 (s, 2H), 3.10 (s, 2H), 2.94 (tt, J = 8.3, 3.7 Hz, 1H), 1.99 (dq, J = 12.6, 6.3, 5.4 Hz, 1H), 1.83 (ddt, J = 12.5, 6.4, 3.3 Hz, 2H), 1.60 (ddt, J = 13.9, 9.2, 4.5 Hz, 2H), 1.40 (s, 9H), 1.24 (s, 1H).
LC-MS (ESI): $[M+H]^+$ = 337.31

2) tert-Butyl4-((3-amino-6-(2-hydroxyphenyl)pyridazin-4 yl)ethynyl)piperidine-1-carboxylate

Compound 1a (100 mg, 1.0eq), (2-hydroxyphenyl)boronic acid (123 mg, 3.0 eq), potassium carbonate (123 mg, 3.0eq), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II)(27 mg, 0.1eq) was dissolved in water/1,4-dioxane 1:5 (0.4 ml/2 ml) and warmed up to 80°C with stirring overnight. The resulting mixture was concentrated under vacuum and purified by preparative liquid phase to obtain white solid 1b (38 mg, 32%).

$^1$H NMR (600 MHz, Methanol-$d_4$) δ 8.09 (s, 1H), 7.73 (dd, J = 7.9, 1.4 Hz, 1H), 7.26 - 7.23 (m, 1H), 6.93 - 6.89 (m, 2H), 3.34 (s, 1H), 2.19 (t, J = 7.6 Hz, 1H), 2.03 (d, J = 6.0 Hz, 1H), 1.98 - 1.94 (m, 2H), 1.47 (s, 9H), 1.28 (s, 4H).
LC-MS (ESI): $[M+H]^+$ = 395.42

3) 2-(6-Amino-5-(piperidin-4-ylethynyl)pyridazin-3-yl)phenol

Compound 1b (20mg,1.0eq) was dissolved in 2 ml of dichloromethane, 2 ml of trifluoroacetic acid was added and stirred at room temperature for 2h. The mixture was cooled to room temperature and filtered. White solid Compound 1c (13mg, 87%) was obtained by preparative liquid phase purification.

$^1$H NMR (600 MHz, Methanol-$d_4$) δ 8.39 (s, 1H), 7.68 (d, J = 8.0 Hz, 1H), 7.39 (t, J = 7.8 Hz, 1H), 7.01 (dt, J = 7.2, 2.9 Hz, 2H), 3.46 (ddd, J = 12.2, 6.1, 3.7 Hz, 2H), 3.27 - 3.18 (m, 3H), 2.29 - 2.25 (m, 1H), 2.06 (dq, J = 14.1, 5.3 Hz, 2H), 1.34 - 1.31 (m, 2H).
LC-MS (ESI): $[M+H]^+$ = 295.33

**Example 2: Preparation of 2-6-amino-5-(pyrrolidin-3-ylethynyl)pyridazin-3-yl)phenol (Compound 2c)**

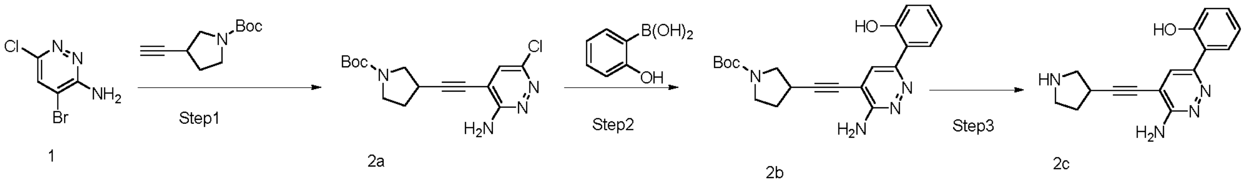


1) tert-Butyl 3-((3-amino-6-chloropyridazin-4-yl)ethynyl)pyrrolidine-1-carboxylate

Under nitrogen protection, Compound 1 (100mg,1.0eq), tert-butyl 3-ethynylpyrrolidine-1-carboxylate (112mg,1.2eq), triethylamine (97 mg,2.0 eq), cuprous iodide (9mg,0.1eq) and bis(triphenylphosphine)palladium(II) dichloride (34mg,0.1eq) were dissolved in N, N-dimethylformamide (2mL) and the reaction was raised to 115°C for 4h. After the reaction was completed, the mixture was purified by preparative liquid phase to obtain white solid 2a (77mg,

49%).
LC-MS(ESI): [M+H] + =323.28

2) tert-Butyl 3-((3-amino-6-(2-hydroxyphenyl)pyridazin-4-yl)ethynyl)pyrrolidine-1-carboxylate

Compound 2a (70mg,1.0eq), (2-hydroxyphenyl)boronic acid (89.73mg,3.0eq), potassium carbonate (89.91mg,3.0eq), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (27mg, 0.1eq) was dissolved in water/1,4-dioxane 1:5 (0.4ml/2 ml) and stirred at 80 °C overnight. The resulting mixture was concentrated under vacuum and purified by preparative liquid phase to obtain white solid 2b (27 mg, 32%).
LC-MS(ESI): [M+H] + =381.44

3) 2-(6-Amino-5-(pyrrolidin-3-ylethynyl)pyridazin-3-yl)phenol

Compound 2b (20mg, 1.0eq) was dissolved in 2ml of dichloromethane, 2ml of HCl/1,4-dioxane was added and stirred at room temperature, the mixture was cooled to room temperature and filtered and purified by preparative liquid phase to obtain the white solid (Compound 2c) (12mg,81%)

$^1$H NMR (600 MHz, Methanol-$d_4$) δ 8.36 (s, 1H), 7.70 - 7.68 (m, 1H), 7.37 (t, J = 7.3 Hz, 1H), 7.00 (dt, J = 7.1, 2.9 Hz, 2H), 3.69 (ddd, J = 19.0, 12.3, 7.2 Hz, 2H), 3.55 (ddt, J = 16.6, 11.2, 5.2 Hz, 2H), 3.47 - 3.42 (m, 1H), 2.50 (dt, J = 14.1, 6.9 Hz, 1H), 2.34 (dd, J = 13.6, 6.7 Hz, 1H).
LC-MS(ESI): [M+H]$^+$ = 281.29

**Example 3: 2-(6-amino-5-(azetidinyl-3-ethylenylidene)pyridazin-3-yl)phenol (Compound 3c)**

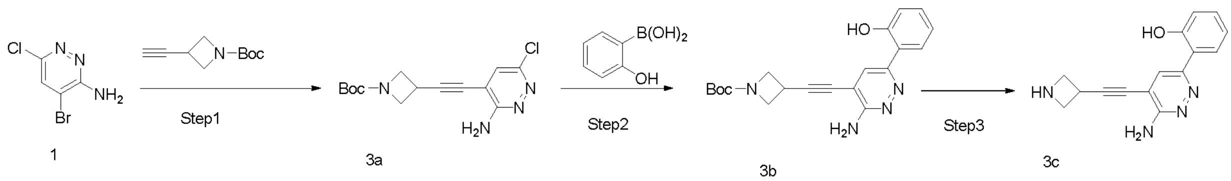

1 3a 3b 3c

1) tert-Butyl 3-((3-amino-6-chloropyridazin-4-yl)ethynyl)azetidine-1-carboxylate

Under nitrogen protection, Compound 1 (100mg, 1.0eq), tert-butyl 3-ethynylazetidine-1-carboxylate (104mg,1.2eq), triethylamine (97 mg, 2.0 eq), cuprous iodide (9mg, 0.1eq), and 1,1'-bis(diphenylphosphine)dichloropalladium ferrocene(II) (34mg,0.1eq) were dissolved in N, N-dimethylformamide (2mL) and the reaction was heated up to 115 °C for 4h. After the reaction was completed, the mixture was purified by preparative liquid phase to obtain the white solid 3a (60mg, 80%).

$^1$H NMR (600 MHz, DMSO-$d_6$) δ 7.56 (s, 1H), 7.01 (s, 2H), 4.15 - 4.11 (m, 2H), 4.00 - 3.95 (m, 2H), 3.75 (ddd, J = 8.8, 6.3, 2.6 Hz, 1H), 1.39 (s, 9H).
LC-MS(ESI): [M+H]$^+$ = 309.26

2) tert-Butyl 3-((3-amino-6-(2-hydroxyphenyl)pyridazin-4-yl)ethynyl)azetidine-1-carboxylate

Under nitrogen protection, Compound 3a (120mg,1.0eq), (2-hydroxyphenyl)boronic acid (161mg,3.0eq), potassium carbonate (161mg,3.0eq), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (35mg, 0.1eq) were dissolved in water/1,4-dioxane 1:5 (0.4/2 ml) and stirred at 80 °C overnight. The resulting mixture was concentrated under vacuum and purified by preparative liquid phase to obtain white solid 3b (40mg, 28%).
LC-MS(ESI): [M+H] + = 367.40

3) 2-(6-Amino-5-(azetidinyl-3-ethylenylidene)pyridazin-3-yl)phenol

Compound 1c (27mg,1.0eq) was dissolved in 2 ml of dichloromethane, HCl/1,4-dioxane (2ml) was added and

stirred at room temperature, the mixture was cooled to room temperature and filtered. After the reaction was completed, the white solid Compound 3c (12mg,61%) was obtained by preparative liquid phase purification.

$^1$H NMR (600 MHz, Methanol-$d_4$) δ 8.68 (s, 1H), 7.68 - 7.64 (m, 1H), 7.53 - 7.49 (m, 1H), 7.13 (q, J = 8.2, 7.8 Hz, 3H), 4.67 (p, J = 8.0 Hz, 1H), 4.60 (t, J = 9.8 Hz, 2H), 4.52 (t, J = 9.4 Hz, 2H).
LC-MS(ESI): $[M+H]^+$ = 267.26

**Example 4: (5-((2-azaspiro[3.3]hept-6-yl)ethynyl)-6-aminopyridazin-3-yl)phenol (Compound 4c)**

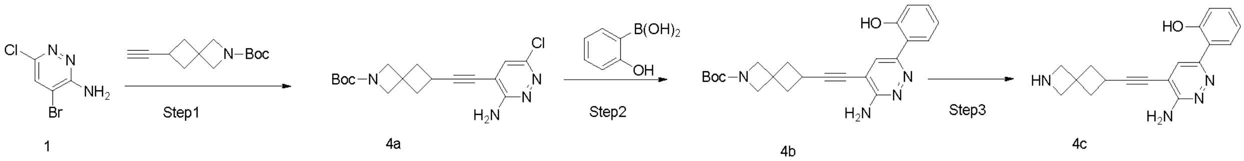


1) tert-Butyl 6-((3-amino-6-chloropyridazin-4-yl)ethynyl)-2-azaspiro[3.3]heptane-2-carboxylate

Under nitrogen protection, Compound 1a (128mg, 1.0eq), tert-butyl 6-ethynyl-2-azaspiro[3.3]heptane-2-carboxylate(163mg,1.2eq), triethylamine (124mg, 2.0eq), cuprous iodide (12mg, 0.1eq) and bis(triphenylphosphine)palladium(II) dichloride (41mg, 0.1eq) were dissolved in N,N-dimethylformamide (2mL) and the reaction was raised to 115 °C for 4h. After the reaction was completed, the mixture was purified by preparative liquid phase to obtain the white solid 4a (120mg, 56%).

$^1$H NMR (600 MHz, DMSO-$d_6$) δ 7.48 (s, 1H), 6.81 - 6.77 (m, 2H), 3.88 (s, 2H), 3.81 (s, 2H), 3.23 (q, J = 8.4 Hz, 1H), 2.54 (d, J = 3.0 Hz, 1H), 2.43 - 2.39 (m, 2H), 1.99 (dt, J = 18.6, 6.8 Hz, 1H), 1.36 (s, 9H).
LC-MS(ESI): $[M-H]^+$ = 349.33

2) tert-Butyl 6-((3-amino-6-(2-hydroxyphenyl)pyridazin-4-yl)ethynyl)-2-azaspiro[3.3]heptane-2-carboxylate

Under nitrogen protection, Compound 4a (80mg, 1.0eq), (2-hydroxyphenyl)boronic acid (95mg, 3.0eq), potassium carbonate (95mg, 3.0eq), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (21 mg, 0.1 eq) were dissolved in water/1,4-dioxane 1:5 (0.4/2 ml) and stirred at 80 °C overnight. The resulting mixture was concentrated under vacuum and purified by preparative liquid phase to obtain the white solid 4b (30 mg, 32%).

$^1$H NMR (600 MHz, DMSO-d6 ) δ 13.32 (s, 1H), 8.17 (s, 1H), 7.87 (dd, J = 8.0, 1.3 Hz, 1H), 7.26 - 7.23 (m, 1H), 6.92 - 6.84 (m, 4H), 3.87 ( d, J = 39.3 Hz, 4H), 3.27 (q, J = 8.3 Hz, 1H), 2.59 - 2.55 (m, 2H), 2.47 - 2.43 (m, 2H), 1.37 (s, 9H).
LC-MS(ESI): $[M-H]^+$ = 407.41

3) 2-(5-((2-Azaspiro[3.3]hept-6-yl)ethynyl)-6-aminopyridazin-3-yl)phenol

Compound 4b (30 mg, 1.0 eq) was dissolved in 2 ml of dichloromethane, 2 ml of trifluoroacetic acid was added and stirred at room temperature, the mixture was cooled to room temperature and filtered and purified by liquid phase to obtain the white solid Compound 4c (10mg, 44%).

$^1$H NMR (600 MHz, Methanol-d4) δ 8.32 (s, 1H), 7.69 - 7.65 (m, 1H), 7.41 - 7.36 (m, 1H), 7.03 - 6.97 (m, 2H), 4.19 (s, 2H), 4.12 (s, 2H), 3.44 - 3.39 (m, 1H), 2.82 - 2.77 (m, 2H), 2.63 (ddd, J = 10.7, 8.4, 2.2 Hz, 2H).
LC-MS(ESI): $[M-H]^+$ = 307.31

**Example 5: (6-(2-azaspiro[3.5]nonan-7-yl)ethynyl)-6-aminopyridazin-3-yl)phenol (Compound 5c)**

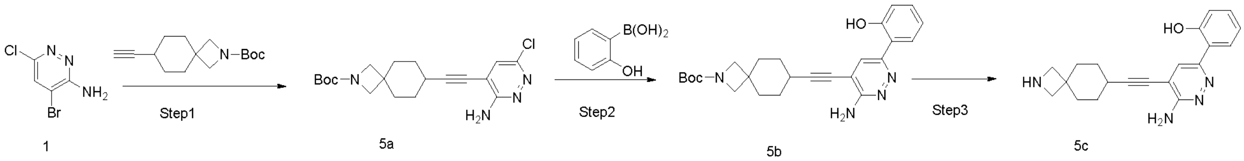


1) tert-Butyl 7-((3-amino-6-chloropyridazin-4-yl)ethynyl)-2-azaspiro[3.5]nonane-2-carboxylate

Under nitrogen protection, Compound 1a (100mg, 1.0eq), tert-butyl 7-ethynyl-2-azaspiro[3.5]nonane-2-carboxylate (144mg, 1.2eq), triethylamine (97mg, 2.0eq), cuprous iodide (9mg, 0.1eq), and bis(triphenylphosphine)palladium(II) dichloride (34 mg, 0.1eq) were dissolved in N,N-dimethylformamide (2mL) and the reaction was heated to 115 °C for 4h. After the reaction was completed, the mixture was purified by preparative liquid phase to obtain the white solid 5a (157mg, 86%).

$^1$H NMR (600 MHz, DMSO-$d_6$) δ 7.56 (s, 1H), 7.03 (s, 2H), 3.49 (d, J = 23.1 Hz, 4H), 2.70 (s, 1H), 1.81 (d, J = 9.5 Hz, 4H), 1.53 (q, J = 10.9, 10.4 Hz, 4H), 1.38 (s, 9H).
LC-MS(ESI): $[M+H]^+$ = 405.45

2) tert-Butyl7-((3-amino-6-(2-hydroxyphenyl)pyridazin-4-yl)ethynyl)-2-azaspiro[3.5]nonane-2-carboxylate

Compound 5a (90 mg, 1.0 eq), (2-hydroxyphenyl)boronic acid (99mg, 3.0eq), potassium carbonate (99 mg, 3.0eq), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (22 mg, 0.1eq) were dissolved in water/1,4-dioxane 1:5 (0.4/2.0 ml) and stirred at 80 °C overnight. The resulting mixture was concentrated under vacuum and purified by preparative liquid phase to obtain the white solid 5b (30mg, 28%).

$^1$H NMR (600 MHz, DMSO-$d_6$) δ 8.22 (s, 1H), 7.73 - 7.70 (m, 1H), 7.33 - 7.31 (m, 1H), 6.98 (d, J = 8.2 Hz, 1H), 6.93 (t, J = 7.5 Hz, 1H), 3.53 (s, 5H), 3.10 (qd, J = 7.3, 4.8 Hz, 1H), 1.84 (d, J = 10.1 Hz, 4H), 1.61 - 1.53 (m, 4H), 1.38 (s, 9H), 1.22 - 1.16 (m, 2H).
LC-MS(ESI): $[M+H]^+$ = 435.42

3) 2-(5-((2-Azaspiro[3.5]nonan-7-yl)ethynyl)-6-aminopyridazin-3-yl)phenol

Compound 5b (20 mg,1.0eq) was dissolved in 2ml of dichloromethane, HCl/1,4-dioxane (2ml) was added and stirred at room temperature, the mixture was cooled to room temperature and filtered and purified by liquid phase purification to obtain the white solid Compound 5c (10 mg, 64%).

$^1$H NMR (600 MHz, Methanol-$d_4$) δ 8.30 (s, 1H), 7.69 - 7.67 (m, 1H), 7.39 - 7.36 (m, 1H), 7.00 (dt, J = 7.3, 3.0 Hz, 2H), 3.88 (s, 2H), 3.82 (s, 2H), 2.86 (s, 1H), 2.13 - 2.09 (m, 2H), 2.03 - 1.97 (m, 2H), 1.73 (dt, J = 21.6, 10.2 Hz, 4H).
LC-MS(ESI): $[M+H]^+$ = 335.31

**Example 6: (5-((3-azaspiro[5.5]undecan-9-yl)ethynyl)-6-aminopyridazin-3-yl)phenol (Compound 6c)**

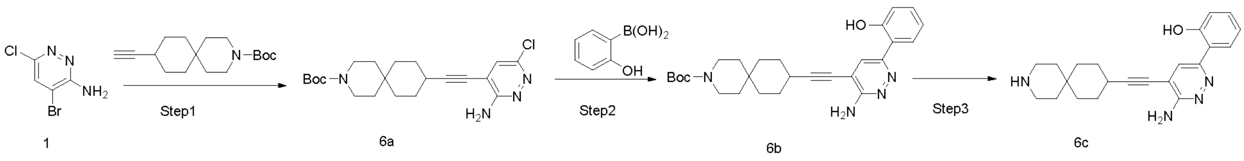


1) tert-Butyl 9-((3-amino-6-chloropyridazin-4-yl)ethynyl)-3-azaspiro[5.5]undecane-3-carboxylate

Under nitrogen protection, Compound 1 (60mg, 1.0eq), tert-butyl 9-ethynyl-3-azaspiro[5.5]undecane-3-carboxylate (96mg, 1.2eq), triethylamine (58.26mg, 2.0eq), cuprous iodide (5mg, 0.1eq) and bis(triphenylphosphine)dichloropalladium(II) (20mg, 0.1eq ) were dissolved in N,N-dimethylformamide (2mL) and the reaction was raised to 115 °C for 4h. After completion of the reaction, the mixture was purified by preparative liquid phase to obtain white solid 6a (84mg,

72%).

$^1$H NMR (600 MHz, DMSO-$d_6$) δ 7.48 (s, 1H), 6.73 (s, 2H), 3.29 (s, 4H), 2.70 - 2.67 (m, 1H), 1.80 - 1.74 (m, 4H), 1.62 (q, J = 10.3, 9.3 Hz, 6H), 1.40 (s, 2H), 1.39 (s, 9H).
LC-MS(ESI): $[M+H]^+$ = 405.45

2) tert-Butyl 9-((3-amino-6-(2-hydroxyphenyl)pyridazin-4-yl)ethynyl)-3-azaspiro[5.5]undecane-3-carboxylate

Under nitrogen protection, Compound 6a (65mg,1.0eq), (2-hydroxyphenyl)boronic acid (66mg,3.0eq), potassium carbonate (67mg,3.0eq), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (15mg, 0.1eq) were dissolved in water/1,4-dioxane 1:5 (0.4ml/2ml) and stirred at 80 °C overnight. The resulting mixture was concentrated under vacuum and purified by preparative liquid phase to obtain the white solid 6b (33mg, 44%).
LC-MS(ESI): $[M+H]^+$ = 463.44

3) 2-(5-((3-Azaspiro[5.5]undecan-9-yl)ethynyl)-6-aminopyridazin-3-yl)phenol

Compound 6b (30 mg,1.0eq) was dissolved in 2ml of dichloromethane, HCl/1,4-dioxane (2 ml) was added and stirred at room temperature, the mixture was cooled to room temperature and filtered. White solid Compound 6c (14 mg, 59%) was obtained by preparative liquid phase purification.

$^1$H NMR (600 MHz, Methanol-$d_4$) δ 8.32 (s, 1H), 7.67 (d, J = 7.9 Hz, 1H), 7.39 (t, J = 7.7 Hz, 1H), 7.02 - 6.99 (m, 2H), 3.21 - 3.18 (m, 4H), 2.86 (s, 1H), 2.05 (q, J = 6.3 Hz, 1H), 2.00 - 1.96 (m, 2H), 1.80 (d, J = 21.6 Hz, 5H), 1.67 (s, 2H), 1.43 (t, J = 11.7 Hz, 2H).
LC-MS(ESI): $[M+H]^+$ = 363.83

**Example 7: 2-(6-amino-5-(phenylethynyl)pyridazin-3-yl)phenol (Compound 7c)**

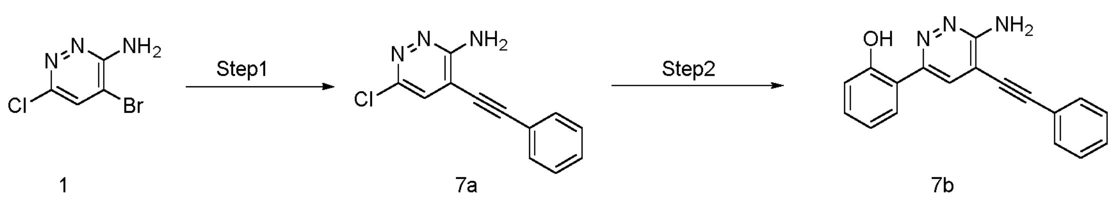


1 7a 7b

1) 6-Chloro-4-(phenylethynyl)pyridazin-3-amine

Compound 1 (200mg,1eq), phenylethynyl (117mg,1.2eq), bis(triphenylphosphine)palladium(II) dichloride (67 mg,0.1eq), cuprous iodide (18 mg,0.1eq), and triethylamine (194 mg,2 eq) were dissolved in N,N-dimethylformamide (2 ml) under nitrogen protection. The reaction was raised to 115 °C for 4 h. After the reaction was completed, the mixture was purified by preparative liquid phase to obtain the target Compound 7a (51 mg,23%)

LC-MS M/Z (M+H) 230.16/232.13.
$^1$H NMR (600 MHz, DMSO-$d_6$) δ 13.09 (s, 1H), 8.10 - 8.07 (m, 2H), 7.97 (s, 1H), 7.56 (t, J = 7.5 Hz, 2H), 7.53 - 7.50 (m, 1H), 7.05 (d, J = 1.9 Hz, 1H).

2) 2-(6-Amino-5-(phenylethynyl)pyridazin-3-yl)phenol

Compound 7a (46 mg,1.0eq), 2-hydroxyphenylboronic acid (82 mg,3.0eq), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (18 mg,0.1eq), potassium carbonate (82 mg,3.0eq) were dissolved in a solvent mixture of water/1,4-dioxane=0.6mL/3mL and stirred at 80°C overnight. The resulting mixture was concentrated under vacuum and purified by preparative liquid phase to obtain the target Compound 7b (15 mg, 26%).

LC-MS M/Z (M+H) 288.21.
$^1$H NMR (600 MHz, DMSO-$d_6$) δ 13.57 (d, J = 37.7 Hz, 2H), 8.64 (d, J = 1.2 Hz, 1H), 8.21 - 8.16 (m, 2H), 7.91 - 7.86 (m,

1H), 7.65 - 7.58 (m, 3H), 7.43 - 7.32 (m, 2H), 7.09 - 7.01 (m, 2H).

**Example 8: Preparation of 2-(6-amino-5-(pyridin-3-vinyl)pyridazin-3-yl)phenol** (**Compound 8b**)

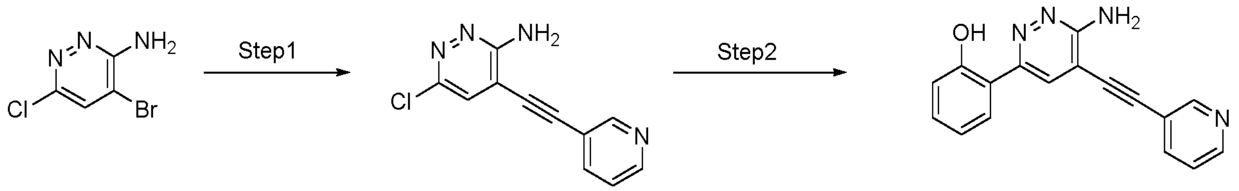


1 8a 8b

1) 6-Chloro-4-(pyridin-3-vinyl)pyridazin-3-amine

Compound 1 (200 mg, 1eq), 3-ethynylpyridine (118 mg, 1.2eq), bis(triphenylphosphine)palladium(II) dichloride (67 mg,0.1eq), cuprous iodide (18 mg,0.1eq), triethylamine (194 mg,2eq) were dissolved in N,N-dimethylformamide (2ml) under nitrogen protection. The reaction was raised to 115 °C for 4h. After the reaction was completed, the mixture was purified by preparative liquid phase to obtain the target Compound 8a (18 mg, 8%).

LCMS M/Z (M+H) 231.16/233.12.
$^1$H NMR (600 MHz, DMSO-$d_6$) δ 8.94 (s, 1H), 8.69 (d, J = 4.7 Hz, 1H), 8.18 (dt, J = 7.9, 1.9 Hz, 1H), 7.73 (s, 1H), 7.56 (dd, J = 7.9, 4.8 Hz, 1H), 7.23 (s, 2H).

2) 2-(6-Amino-5-(pyridin-3-vinyl)pyridazin-3-yl)phenol

Compound 8a (100 mg,1.0 eq), 2-hydroxyphenylboronic acid (180 mg,3.0 eq), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (36 mg, 0.1eq), potassium carbonate (180 mg, 3.0 eq) were dissolved in a solvent mixture of water/1,4-dioxane = 0.6 mL/3 mL and stirred at 80 °C overnight. The resulting mixture was concentrated under vacuum and purified by preparative liquid phase to obtain the target Compound 8b (15 mg ,26%).

LCMS M/Z (M+H) 289.19.
$^1$H NMR (600 MHz, DMSO-$d_6$) δ 13.62 (s, 2H), 9.39 (d, *J* = 2.4 Hz, 1H), 8.77 - 8.73 (m, 1H), 8.71 (s, 1H), 8.55 (dt, *J*= 8.1, 2.0 Hz, 1H), 7.92 (d, *J* = 7.8 Hz, 1H), 7.67 (dd, *J* = 8.0, 4.8 Hz, 1H), 7.46 (s, 1H), 7.42 - 7.35 (m, 1H), 7.07 - 7.00 (m, 2H).

**Example 9: Preparation of 2-(6-amino-5-ethynylpyridazin-3-yl)phenol (Compound 9b)**

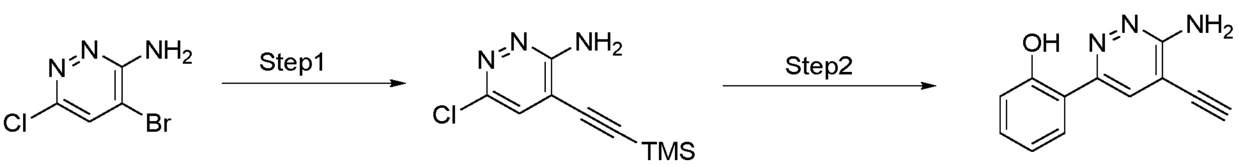


1 9a 9b

1) 6-Chloro-4-(pyridin-3-vinyl)pyridazin-3-amine

Compound 1 (200mg, 1eq), ethynyltrimethylsilane (113mg, 1.2eq), bis(triphenylphosphine)palladium(II) dichloride (67mg,0.1eq), cuprous iodide (18 mg,0.1eq), and triethylamine (194mg,2eq) were dissolved in N,N-dimethylformamide (2ml) under nitrogen protection. The reaction was raised to 115 °C for 4h. After reaction was completed, the mixture was purified by preparative liquid phase to obtain the target Compound 9a (20 mg ,6%).

LC-MS M/Z (M+H ) 226.20/228.15.
$^1$H NMR (600 MHz, DMSO-d6 ) δ 7.58 (s, 1H), 6.83 (s, 2H), 0.27 (s, 9H).

2) 2-(6-Amino-5-ethynylpyridazin-3-yl)phenol

Compound 9a (60 mg,1.0eq), 2-hydroxyphenylboronic acid (110 mg,3.0 eq), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (36 mg,0.1 eq), potassium carbonate (110 mg,3.0 eq) were dissolved in N,N-dimethylformamide (2ml). The mixture was stirred at 80°C overnight. The resulting mixture was concentrated under vacuum and purified by preparative liquid phase to obtain the target Compound 9b (5mg, 8%).

LC-MS M/Z (M+H) 212.20.
$^1$H NMR (600 MHz, DMSO-$d_6$) δ 8.72 (s, 1H), 7.37 (t, *J* = 7.8 Hz, 1H), 7.05 - 6.98 (m, 2H), 6.81 (s, 1H), 6.72 (q, *J* = 4.3 Hz, 2H), 6.60 (dd, *J* = 5.9, 3.7 Hz, 2H).

**Example 10: (5-((7-azaspiro[3.5]nonan-2-yl)ethynyl)-6-aminopyridazin-3-yl)phenol (Compound 10c)**

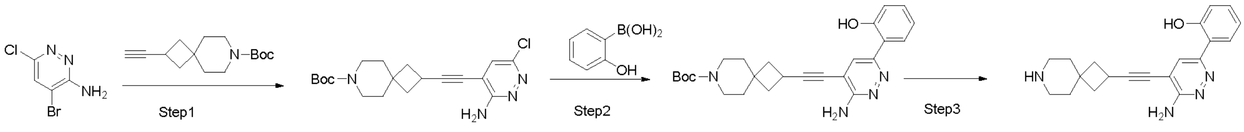


1 10a 10b 10c

1) tert-Butyl 2-((3-amino-6-chloropyridazin-4-yl)ethynyl)-7-azaspiro[3.5]nonane-7-carboxylate

Under nitrogen protection, Compound 1 (100 mg, 1.0eq), tert-butyl 2-ethynyl-7-azaspiro[3.5]nonane-7-carboxylate (143.55mg,1.2eq), triethylamine (97.09 mg,2.0 eq), cuprous iodide (9.14 mg,0.1 eq), and bis(triphenylphosphine) palladium(II) dichloride ( 33.67 mg,0.1eq) were dissolved in N,N-dimethylformamide (2 mL) and the reaction was raised to 115 °C for 4h. After the reaction was completed, the mixture was purified by preparative liquid phase to obtain the white solid 10a (157 mg, 86%).

$^1$H NMR (600 MHz, DMSO-$d_6$) δ 3.49 (d, J = 23.1 Hz, 4H), 1.81 (d, J = 9.5 Hz, 4H), 1.53 (q, J = 10.9, 10.4 Hz, 4H), 1.38 (s, 9H).
LC-MS(ESI): $[M+H]^+$ =377.34

2) tert-Butyl 2-((3-amino-6-(2-hydroxyphenyl)pyridazin-4-yl)ethynyl)-7-azaspiro[3.5]nonane-7-carboxylate

Compound 10a (100 mg,1.0 eq), (2-hydroxyphenyl)boronic acid (110 mg,3.0 eq), potassium carbonate (110 mg,3.0 eq), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (24 mg, 0.1 eq) were dissolved in water/1,4-dioxane 1:5 (0.4 ml/2 ml) and stirred at 80 °C overnight. The resulting mixture was concentrated under vacuum and purified by preparative liquid phase to obtain 10b (30 mg, 26%) as a white solid.
LC-MS(ESI): $[M+H]^+$ =435.43

3) 2-(5-((7-Azaspiro[3.5]nonan-2-yl)ethynyl)-6-aminopyridazin-3-yl)phenol

Compound 10b (25 mg,1.0 eq) was dissolved in 2 ml of dichloromethane, 2 ml of trifluoroacetic acid was added and stirred at room temperature for 2h. The mixture was cooled to room temperature and filtered. Purified by preparative liquid phase, white solid 10c (12 mg, 62%) was obtained.

$^1$H NMR (600 MHz, Methanol-$d_4$) δ 8.38 (s, 1H), 7.66 (dd, J = 8.0, 1.5 Hz, 1H), 7.41 - 7.38 (m, 1H), 7.01 (dt, J = 7.3, 3.0 Hz, 2H), 3.53 (p, J = 8.7 Hz, 1H), 3.19 - 3.17 (m, 2H), 3.14 - 3.11 (m, 2H), 2.46 - 2.42 (m, 2H), 2.27 - 2.23 (m, 2H), 1.93 (q, J = 4.8 Hz, 4H).
LC-MS(ESI): $[M+H]^+$ = 335.32.

**Example 11: 2-(5-(1H-indol-5-yl)ethynyl)-6-aminopyridazin-3-yl)phenol (Compound 11b)**

1) 4-((1H-indol-5-yl)ethynyl)-6-chloropyridin-3-amine

Compound 1 (50.0 mg, 1.0 eq), 5-ethynyl-1H-indole (41 mg, 1.2 eq), triethylamine (49 mg, 2.0 eq), cuprous iodide (5 mg, 0.1 eq) and bis(triphenylphosphine) palladium (II) dichloride (17 mg, 0.1 eq) were dissolved in N,N-dimethylformamide (2 mL) under nitrogen protection and the reaction was raised to 115 °C for 4 h. After the reaction was completed, the mixture was purified by preparative liquid phase to obtain the white solid 11a (48 mg, 74%).

$^1$H NMR (400 MHz, DMSO-$d_6$) δ 12.92 (s, 1H), 11.42 (d, J = 17.4 Hz, 1H), 8.32 (s, 1H), 7.84 (s, 1H), 7.79 (dd, J = 8.6, 1.5 Hz, 1H), 7.54 (d, J = 8.5 Hz, 1H), 7.48 - 7.42 (m, 2H), 6.92 (d, J = 1.7 Hz, 1H).
LC-MS (ESI): $[M+H]^+$ =269.22

2) 2-(5-(1H-indol-5-yl)ethynyl)-6-aminopyridazin-3-yl)phenol

Compound 11a (50.0 mg,1.0 eq), (2-hydroxyphenyl)boronic acid (77 mg,3.0 eq), potassium carbonate (77 mg,3.0 eq), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (17 mg, 0.1 eq) were dissolved in water/1,4-dioxane 1:5 (0.4 ml/2 ml) and stirred at 80 °C overnight. The resulting mixture was concentrated under vacuum and purified by preparative liquid phase to obtain the white solid 11b (12 mg, 20%).

$^1$H NMR (400 MHz, Methanol-$d_4$) δ 8.40 (s, 2H), 7.87 (dd, J = 8.6, 1.7 Hz, 1H), 7.65 - 7.59 (m, 2H), 7.48 (td, J = 8.2, 1.5 Hz, 1H), 7.41 (d, J = 3.2 Hz, 1H), 7.33 (s, 1H), 6.66 (d, J = 3.0 Hz, 1H).
LC-MS(ESI): $[M+H]^+$ =327.33

**Example 12: Synthesis of Intermediate 18**

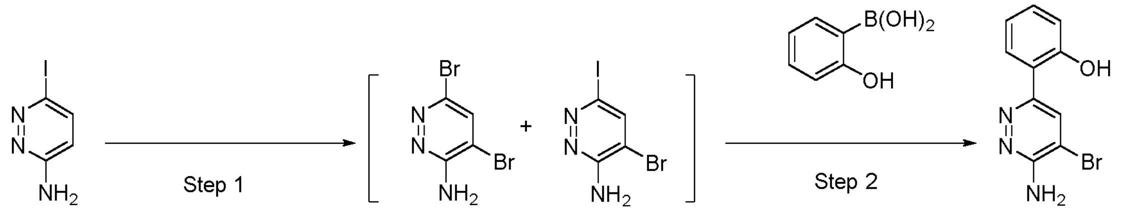


Intermediate 18a Intermediate 18b Intermediate 18

**Step 1: Synthesis of 4-bromo-6-iodopyridazin-3-amine and 4,6-dibromopyridazin-3-amine**

4-Bromo-6-iodopyridazin-3-amine (5.00 g, 22.62 mmol) was dissolved in a vial containing acetonitrile (100 mL), and N-bromosuccinimide (4.03 g, 22.62 mmol) were added slowly and stirred for 1h. After the reaction was completed, the reaction solution was quenched with aqueous sodium thiosulphate, and extracted with ethyl acetate three times (20 mL). The organic phase was washed with saturated saline and concentrated by drying and filtering with anhydrous sodium sulfate. The crude product was purified by column (petroleum ether: ethyl acetate = 1: 1) to obtain the mixture of Intermediate 18a and Intermediate 18b as a yellow solid Compound (2.02 g).

**Step 2: Synthesis of 2-(6-amino-5-bromopyridazin-3-yl)phenol**

A mixture of Intermediate 18a and Intermediate 18b (555.00 mg, 1.85 mmol), (2-hydroxyphenyl)boronic acid (204.20 mg, 1.48 mmol), palladium tetrakis(triphenylphosphine) (213.86 mg, 185.06 mmol), and potassium carbonate (639.41 mg, 4.63 mmol) were dissolved in 1,4-dioxane (7 mL) and purified water (1 mL), and after nitrogen protection, the reaction was stirred at 80 °C for 3h. At the end of the reaction, excess solvent was removed under reduced pressure, purified water was added, and the mixture was extracted with ethyl (20 mL)acetate three times . The organic phase was washed with saturated brine, dried and filtered with anhydrous sodium sulfate to concentrate the crude product. The crude product was purified by column (petroleum ether: ethyl acetate = 1:1) to obtain yellow solid Intermediate 18 (147 mg). $^1$H NMR (600 MHz, DMSO-$d_6$) δ 8.50 (s, 1H), 7.82 (dd, *J* = 7.8, 1.3 Hz, 1H), 7.29 - 7.23 (m, 1H), 6.92 (t, *J* = 8.2 Hz, 2H).

**Example 13 : Synthesis of 2-((3-amino-6-(2-hydroxyphenyl)pyridazin-4-yl)ethynyl)spiro[3.5]nonan-7-one (Compound 12)**

Synthesis Scheme

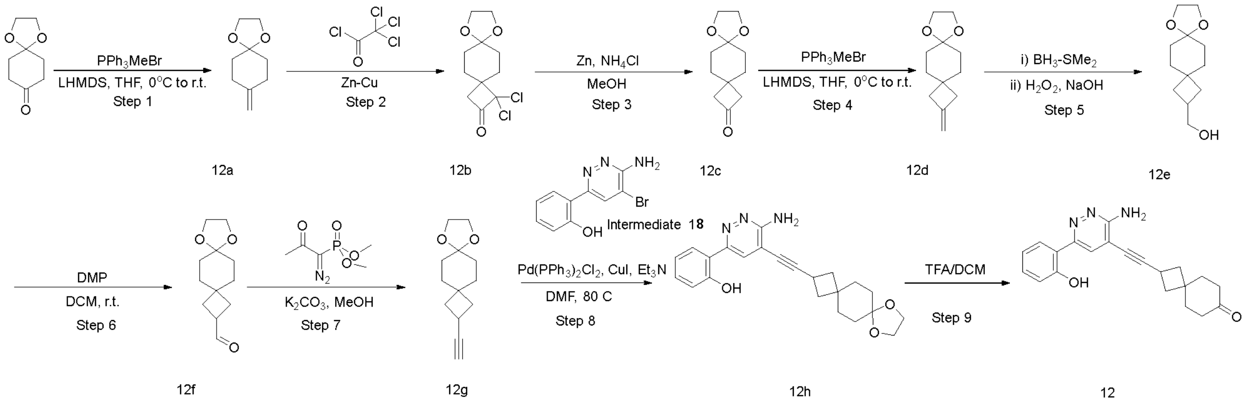


12a 12b 12c 12d 12e

12f 12g 12h 12

Step 1: Synthesis of 8-methylene-1,4-dioxaspiro[4.5]decane (Intermediate 12a)

Methyltriphenylphosphonium bromide (354.53 g, 992.44 mmol) was dissolved in a three-necked vial containing tetrahydrofuran (500 mL), cooled to 0 °C under nitrogen protection, and a tetrahydrofuran solution of LHMDS (992.44 mL, 992.44 mmol) was slowly added, maintained at 0 °C and stirred for 1 h. 1,4-Dioxaspiro[4.5]decan-8-one (50.00 g, 320.14 mmol) in tetrahydrofuran (100 mL) was added to a triplex vial and stirred at 0 °C for 0.5 h. The reaction was brought to room temperature and stirring was continued overnight. After the reaction was completed, the product was quenched with purified water and extracted with ethyl acetate three times (100 mL) and the organic phase was washed with saturated brine, dried with anhydrous sodium sulfate and concentrated by filtration to obtain the crude product. The crude product was purified by column (petroleum ether: ethyl acetate = 10:1) to obtain colorless oily Intermediate 12a (40.0 g, 11.43%).

Step 2: Synthesis of 1,1-dichloro-8,11-dioxadiazolo[3.2.4$^7$.2$^4$]tridecan-2-one (Intermediate 12b)

12a (20 g, 129.69 mmol) and Zn-Cu reagent (33.44 g, 259.39 mmol) were dissolved in a single-necked vial containing DME (200 mL), cooled to 0 °C, and trichloroacetyl chloride (28.95 mL, 259.39 mmol) was added slowly, and stirred at 0 °C for 0.5h. The reaction was transferred and stirred for 3h under room temperature. After completion, the reaction was cooled to 0 °C, quenched with ice water and extracted with ethyl acetate three times (100 mL) and the organic phase was washed with saturated brine, dried and filtered with anhydrous sodium sulfate and concentrated to obtain the crude product of Intermediate 12b. The crude product was used directly in the next step of the reaction without further purification.

Step 3: Synthesis of 8,11-dioxohexaspiro[3.2.4$^7$.2$^4$ ]tridecan-2-one (Intermediate 12c)

The crude product of Intermediate 12b (68.77 g, 259.39 mmol) and ammonium chloride (41.62 g, 778.17 mmol) were dissolved in a single-necked flask containing methanol (300 mL), cooled down to 0 °C, and zinc powder (67.84 g, 1.04 mol) was added slowly, and stirring was maintained at 0 °C for 0.5h. It was transferred to room temperature, and the stirring was continued for 3h. After the reaction was completed, it was cooled to 0 °C, quenched with ice water and extracted with ethyl acetate three times (100 mL), the organic phase was washed with saturated brine, dried with anhydrous sodium sulfate and filtered and concentrated to obtain the crude product. The crude product was purified by column (petroleum ether: ethyl acetate = 5:1) to obtain the yellow oily Intermediate 12c (16 g, 30.37%).

Step 4: Synthesis of 2-methyl-8,11-dioxohexaspiro[3.2.4$^7$.2$^4$ ]tridecane (Intermediate 12d)

Methyltriphenylphosphonium bromide (32.77 g, 91.72 mmol) was dissolved in a three-necked flask containing tetrahydrofuran (60 mL), cooled down to 0°C under nitrogen protection, and a tetrahydrofuran solution of LHMDS (91.72 mL, 91.72 mmol) was added slowly, and stirring was maintained at 0°C for 1h. A tetrahydrofuran (12 mL) solution of Intermediate 12c (6.00 g, 30.57 mmol) was added to a triplex vial and stirred at 0 °C for 0.5 h. The solution was brought to

room temperature and stirred overnight. After the reaction was completed, the product was quenched with purified water and extracted with ethyl acetate three times (100 mL) and the organic phase was washed with saturated saline, dried over anhydrous sodium sulfate and concentrated by filtration to obtain the crude product. The crude product was purified by column (petroleum ether: ethyl acetate = 10:1) to obtain the colorless oily Intermediate 12d (5.59 g, 94.11%).
$^1$H NMR (600 MHz, $CDCl_3$) δ 4.81 - 4.79 (m, 2H), 3.94 (s, 4H), 2.41 (t, J = 2.3 Hz, 4H), 1.69 - 1.65 (m, 4H), 1.59 - 1.57 (m, 4H).

Step 5: Synthesis of (8,11-dioxohexaspiro[3.2.4$^7$.2$^4$]tridecan-2-yl)methanol (Intermediate 12e)

Intermediate 12d (5.59 g, 28.77 mmol) was dissolved in a triplex vial containing tetrahydrofuran (50 mL), cooled down to 0 °C under nitrogen protection, slowly added tetrahydrofuran solution of dimethyl sulfide (17.26 mL, 34.53 mmol), and stirred for 1h. Sodium hydroxide (3.45 g, 86.32 mmol) was added to the triplex vial, and sodium hydroxide (3.45 g, 86.32 mmol) was added slowly. Sodium hydroxide (3.45 g, 86.32 mmol) was added into a three-necked flask, and hydrogen peroxide solution (8.90 g, 86.32 mmol) was added slowly and stirred at 0 °C for 0.5h. After the reaction was completed, it was cooled down to 0 °C, quenched with ice water, and the organic phase was extracted with ethyl acetate for three times (100 mL), and the organic phase was washed with saturated brine, dried with anhydrous sodium sulfate, filtered, and concentrated to obtain the crude product. The crude product was purified by column (petroleum ether: ethyl acetate = 10:1) to obtain the colorless oily Intermediate 12e (2.35 g, 38.47%).
$^1$H NMR (600 MHz, $CDCl_3$) δ 3.93 (s, 4H), 3.59 (d, J = 6.7 Hz, 2H), 2.43 (dt, J = 8.1, 6.9 Hz, 1H), 1.91 - 1.87 (m, 2H), 1.69 (dd, J = 7.7, 4.7 Hz, 2H), 1.63 - 1.57 (m, 4H), 1.54 - 1.47 (m, 4H).

Step 6: Synthesis of 8,11-dioxohexaspiro[3.2.4$^7$.2$^4$]tridecane-2-carbaldehyde (Intermediate 12f)

Intermediate 12e (2.35 g, 11.07 mmol) was dissolved in a single-necked vial containing dichloromethane (40 mL), cooled down to 0 °C, and Dess-Martin oxidizer (9.39 g, 22.14 mmol) was slowly added. The reaction was transferred to room temperature and stirred for 1h. After the reaction was completed, the reaction was quenched with aqueous ammonium chloride, the excess solvent was removed under reduced pressure and the organic phase was extracted three times with ethyl acetate (100 mL), washed with saturated brine, dried with anhydrous sodium sulfate, filtered, and concentrated to obtain the crude product. The crude product was purified by column (petroleum ether: ethyl acetate = 2:1) to obtain the colorless oily Intermediate 12f (2.20 g, 94.54%).
$^1$H NMR (600 MHz, $CDCl_3$) δ 9.75 (d, J = 1.8 Hz, 1H), 3.93 (s, 4H), 3.12 - 3.09 (m, 1H), 2.24 - 2.20 (m, 2H), 2.16 (dd, J = 7.3, 4.8 Hz, 2H), 2.05 - 1.99 (m, 8H).

Step 7: Synthesis of 2-ethynyl-8,11-dioxadiazolo[3.2.4$^7$.2$^4$]tridecane (Intermediate 12g)

Intermediate 12f (2.20 g, 10.46 mmol) and dimethyl (1-diazo-2-oxopropyl)phosphonate (3.01 g, 15.69 mmol) were dissolved in a single-necked vial containing methanol (20 mL), cooled down to 0 °C, potassium carbonate (2.89 g, 20.93 mmol) was added slowly, and stirred at 0 °C for 0.5h. The reaction was transferred to room temperature, and stirring was continued for 1h. After the reaction was completed, the excess solvent was removed under reduced pressure, purified water was added, and the organic phase was extracted with ethyl acetate for three times (20 mL) and washed with saturated brine, dried with anhydrous sodium sulfate, filtered, and concentrated to obtain the crude product. The crude product was purified by column (petroleum ether: ethyl acetate = 5:1) to obtain 12g (510 mg, 22.74%) of colorless oily intermediate.
$^1$H NMR (600 MHz, $CDCl_3$) δ 3.92 (s, 4H), 3.13 (s, 1H), 2.97 - 2.91 (m, 1H), 2.18 - 2.14 (m, 2H), 1.91 - 1.87 (m, 2H), 1.68 (dd, J = 7.6, 4.9 Hz, 2H), 1.63 (dd, J = 7.6, 4.3 Hz, 2H), 1.59 - 1.56 (m, 2H), 1.53 (s, 2H).

Step 8: Synthesis of 2-(5-((8,11-dioxadispiro[3.2.4$^7$.2$^4$]tridecan-2-yl)ethynyl)-6-aminopyridazin-3-yl)phenol (Intermediate 12h)

Intermediate 12g (217.00 mg, 815.49 μmol), SM-I-CH-007 (185.05 mg, 897.04 μmol), bis(triphenylphosphine) dichloropalladium (57.24 mg, 81.55, μmol), cuprous iodide (15.53 mg, 81.55 μmol) and triethylamine (226.71 μL, 1.63 mmol) were dissolved in a three-necked vial containing DMF (2 mL), protected by nitrogen, and heated to 80 °C with stirring overnight. After the reaction was completed, purified water was added and the organic phase was extracted with ethyl acetate for three times (20 mL) and washed with saturated saline, dried with anhydrous sodium sulfate, filtered and concentrated to obtain a crude product. The crude product was purified by column (petroleum ether: ethyl acetate = 1:1) to obtain the yellow solid Intermediate 12h (140 mg, 43.85%).

$^1$H NMR (600 MHz, DMSO-$d_6$) δ 13.36 (s, 1H), 8.17 (s, 1H), 7.88 (dd, J = 8.0, 1.4 Hz, 1H), 7.26 - 7.22 (m, 1H), 6.92 - 6.87 (m, 2H), 6.82 (s, 2H), 3.83 (s, 4H), 3.37 (d, J = 8.7 Hz, 1H), 2.21 (dd, J = 11.6, 9.3 Hz, 2H), 2.02 (dd, J = 11.7, 8.5 Hz,

2H), 1.63 (dd, J = 11.7, 4.9 Hz, 4H), 1.52 (d, J = 6.4 Hz, 2H), 1.48 - 1.45 (m, 2H).
LC-MS (ESI) : $[M+H]^+$ = 392.36

Step 9: Synthesis of 2-((3-amino-6-(2-hydroxyphenyl)pyridazin-4-yl)ethynyl)spiro[3.5]nonan-7-one (Compound 12)

Intermediate 12h (140 mg, 357.63 μmol) was dissolved in a three-necked vial containing dichloromethane (2.5 mL), trifluoroacetic acid (2.5 mL) was added and stirred for 1h. After the reaction was completed, the excess solvent was removed under reduced pressure, purified water was added and the organic phase was extracted with ethyl acetate for three times (20 mL) was washed with saturated brine, dried with anhydrous sodium sulfate, filtered and concentrated. A crude product was obtained. The crude product was purified by column (petroleum ether:ethyl acetate=1:1) to obtain yellow solid Compound 12 (61 mg, 49.09%). $^1$H NMR (600 MHz, DMSO-$d_6$) δ 8.19 (s, 1H), 7.86 (d, J = 7.5 Hz, 1H), 7.27 - 7.24 (m, 1H), 6.98 (s, 2H), 6.92 (d, J = 8.1 Hz, 1H), 6.90 (d, J = 6.8 Hz, 1H), 2.62 - 2.60 (m, 1H), 2.36 (d, J = 9.4 Hz, 2H), 2.26 (d, J = 5.9 Hz, 2H), 2.20 (dd, J = 13.0, 7.6 Hz, 4H), 1.91 - 1.87 (m, 4H).
LC-MS (ESI) : $[M+H]^+$ = 348.3

**Example 14: Synthesis of 7-((3-amino-6-(2-hydroxyphenyl)pyridazin-4-yl)ethynyl)spiro[3.5]nonan-2-one (Compound 13)**

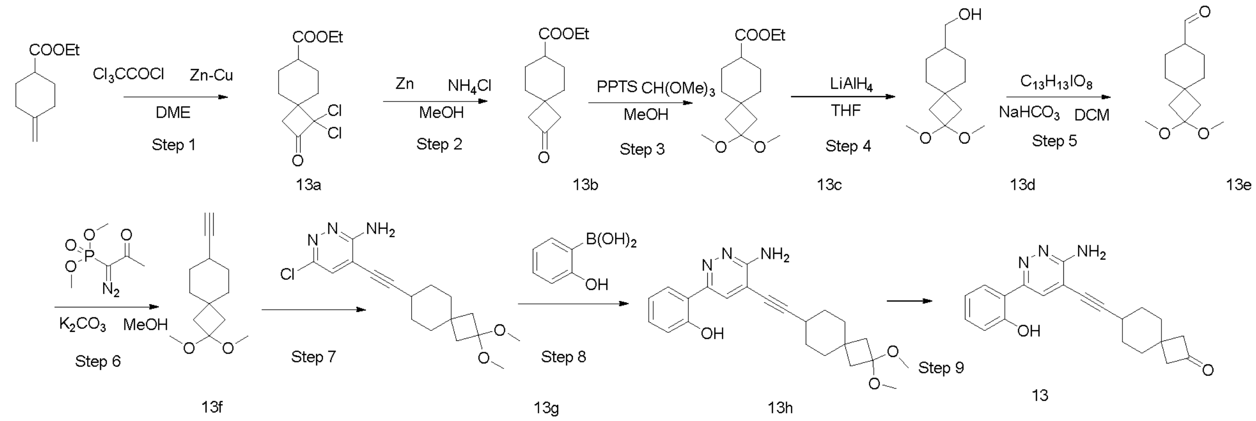


Step 1: Synthesis of ethyl 1,1-dichloro-2-oxospiro[3.5]nonane-7-carboxylate (Intermediate 13a)

Ethyl 4-methylenecyclohexane-1-carboxylate (10 g, 59.44 mmol) was dissolved in ethylene glycol dimethyl ether (100 mL), zinc-copper reagent (10 g, 154 mmol) was added, and trichloroacetyl chloride (25 mL, 224 mmol) was added slowly dropwise to the reaction system under the cooling of ice-water bath, and the reaction was carried out at room temperature overnight. After the reaction was completed, saturated aqueous sodium bicarbonate was added slowly under an ice bath to quench the reaction system, the solid was filtered through diatomaceous earth and the filtrate was extracted with ethyl acetate for three times (100 mL), the organic phase was washed with saturated brine, dried with anhydrous sodium sulfate, filtered and concentrated to obtain a brown oily crude Intermediate 13a (16.6 g). The crude product was directly subjected to the next step without purification.

Step 2: Synthesis of ethyl 2-oxospiro[3.5]nonane-7-carboxylate (Intermediate 13b)

Intermediate 13a (16.6 g, 59.46 mmol), ammonium chloride solid (12.72 g, 237.86 mmol) was dissolved in methanol (160 mL), and zinc powder (38 g, 594 mmol) was added slowly and batchwise in ice-water bath, and the reaction was carried out overnight. After the reaction was completed, the solid was filtered through diatomaceous earth, and the filtrate was concentrated and purified by column chromatography (petroleum ether: ethyl acetate = 10:1) to obtain a colorless oily liquid Intermediate 13b (10.45 g, 83.57%).
$^1$H NMR (600 MHz, DMSO-$d_6$) δ 4.06 (q, J = 7.1 Hz, 2H), 2.74 (s, 4H), 2.36 - 2.31 (m, 1H), 1.83 - 1.79 (m 2H), 1.76 - 1.65 (m, 2H), 1.60 - 1.55 (m, 2H), 1.44 - 1.38 (m, 2H), 1.18 (t, J = 7.1 Hz, 3H).

Step 3: Synthesis of ethyl 2,2-dimethoxyspiro[3.5]nonane-7-carboxylate (Intermediate 13c)

Intermediate 13b (10.45 g, 50 mmol), trimethyl orthoformate (67 mL, 612 mmol)and pyridinium p-toluenesulfonate(3.75 g, 15 mmol) were dissolved in methanol (100 mL) and reacted at 75 °C overnight. After the reaction was completed, the solvent was evaporated, 5mL of water was added, extracted with 30mL of ethyl acetate for three times, dried and concentrated, and then purified by column chromatography (petroleum ether: ethyl acetate=10:1) to obtain the yellow oily liquid Intermediate 13c (12g, 94.19%).
$^1$H NMR (600 MHz, DMSO-$d_6$) δ 4.03 (q, J = 7.1 Hz, 2H), 3.01 (s, 6H), 2.28 - 2.16 (m, 1H), 1.80 (d, J = 7.7 Hz, 4H), 1.70 - 1.65 (m, 2H), 1.64 - 1.54 (m, 2H), 1.40 - 1.29 (m, 4H), 1.16 (t, J = 7.1 Hz, 3H).

Step 4: Synthesis of 2,2-dimethoxyspiro[3.5]nonane-7-methanol (Intermediate 13d)

Intermediate 13c (12 g, 46 mmol), was dissolved in tetrahydrofuran (120 mL) and lithium tetrahydroaluminum (2.67 g, 70 mmol) was added slowly and batchwise in an ice bath. The reaction was carried out for 4h at room temperature. After the reaction was completed, the reaction system was quenched by adding a little water under ice-water bath, filtered through diatomaceous earth, dried and concentrated the filtrate, and purified by column chromatography (petroleum ether: ethyl acetate=5:1) to obtain the Intermediate 13d (7.7 g, 76.75%) as a colorless transparent liquid.
$^1$H NMR (600 MHz, DMSO-$d_6$) δ 4.33 (t, J = 5.3 Hz, 1H), 3.17 (dd, J = 6.4, 5.3 Hz, 2H), 3.00 (s, 6H), 1.79 - 1.77 (m, 4H), 1.65 - 1.48 (m, 4H), 1.31 - 1.20 (m, 3H), 0.95 - 0.79 (m, 2H).

Step 5: Synthesis of 2,2-dimethoxyspiro[3.5]nonane-7-carbaldehyde (Intermediate 13e)

Intermediate 13d (15.4 g, 71.86 mmol), sodium bicarbonate (30 g, 355 mmol) and Desmartin's reagent (45.72 g, 107.8 mmol) were dissolved in dichloromethane (150 mL), and the reaction was carried out at room temperature for 2h. After the reaction was completed, the reaction system was quenched by adding a little water under ice-water bath and the reaction system was filtered through diatomaceous earth and the filtrate was dried and concentrated. The filtrate was purified by column (petroleum ether:ethyl acetate=10:1) to obtain the colorless transparent liquid Intermediate 13e (10.35 g, 67.85%).
$^1$H NMR (600 MHz, DMSO-$d_6$) δ 9.55 (s, 1H), 3.01 (s, 6H), 2.22 (tt, J = 9.9, 3.9 Hz, 1H), 1.82 - 1.76 (m, 4H), 1.73 - 1.66 (m, 2H), 1.59 - 1.53 (m, 2H), 1.40 - 1.34 (m, 2H), 1.33 - 1.25 (m, 2H).

Step 6: Synthesis of 7-ethynyl-2,2-dimethoxyspiro[3.5]nonane (Intermediate 13f)

Intermediate 13e (6.33 g, 29.82 mmol) was dissolved in methanol (60 mL), potassium carbonate (12.36 g, 89.45 mmol) was added, and dimethyl (1-diazido-2-oxopropyl)phosphonate (7.45 g, 38.76 mmol) was added dropwise under ice bath The reaction was carried out at room temperature overnight, until the reaction was completed, the reaction solution was concentrated and purified by column (petroleum ether: ethyl acetate = 10:1) to obtain colorless transparent liquid Intermediate 13f (3.02 g, 48.62%).
$^1$H NMR (600 MHz, DMSO-d6 ) δ 3.00 (s, 6H), 2.83 (d, J = 2.4 Hz, 1H), 2.32 (s, 1H), 1.79 (dd, J = 15.7, 1.5 Hz, 4H), 1.65 - 1.57 (m, 4H), 1.37 - 1.31 (m, 4H).

Step 7: Synthesis of 6-chloro-4-(2,2-dimethoxyspiro[3.5]nonan-7-yl)ethynyl)pyridazin-3-amine (Intermediate 13g)

Intermediate 13f (3 g, 14.4 mmol), 3-amino-4-bromo-6-chloropyridazine (2.5 g, 12 mmol), bis(triphenylphosphine)dichloropalladium (842 mg, 1.2 mmol), cuprous iodide (228 mg, 1.2 mmol), and triethylamine (6.26 mL, 4.8 mmol) were dissolved in a three-necked vial containing DMF (100 mL), protected by nitrogen. The reaction was carried out in a three-necked flask containing DMF (100 mL), protected by nitrogen, and heated to 110 °C for 2h. After the reaction was completed, the diatomaceous earth was filtered, 400 mL of purified water was added to the filtrate, and the organic phase was extracted with ethyl acetate three times (30 mL x 3) and washed with saturated saline, and then dried with anhydrous sodium sulfate and filtered and concentrated to obtain the crude product. The crude product was purified by column (petroleum ether:ethyl acetate=2:1) to obtain 13g (2.37 g,58.84%) of yellow solid Intermediate 13g.

$^1$H NMR (600 MHz, DMSO-$d_6$) δ 7.48 (s, 1H), 6.71 (s, 2H), 3.02 (s, 6H), 2.67 (s, 1H), 1.85 - 1.81 (m, 4H), 1.79 - 1.72 (m, 2H), 1.68 - 1.61 (m, 2H), 1.57 - 1.47 (m, 2H), 1.41 - 1.37 (m, 2H).
LC-MS (ESI) : $[M+H]^+$ = 336.35

Step 8: Synthesis of 2-(6-amino-5-((2,2-dimethoxyspiro[3.5]nonan-7-yl)ethynyl)pyridazin-3-yl)phenol (Intermediate 13h)

Intermediate 13g (3.5 g, 10.42 mmol), 2-hydroxyphenylboronic acid (4.31 g, 31.27 mmol), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (94.5 mg, 1.04 mmol), and potassium carbonate (4.32 g, 31.2 mmol) were dissolved in 1,4-dioxane (40 mL) and water (4 mL), protected by nitrogen, and heated to 80 °C with stirring overnight. After the reaction was completed, the diatomaceous earth was filtered, the filtrate was concentrated, and the crude product was purified by column (petroleum ether: ethyl acetate=2:1) to obtain the yellow solid Intermediate 13h (2.23 g, 58.84%).

$^1$H NMR (600 MHz, DMSO-d6 ) δ 13.32 (s, 1H), 8.15 (s, 1H), 7.88 (dd, J = 8.0, 1.6 Hz, 1H), 7.26 - 7.22 (m, 1H), 6.93 - 6.86 (m, 2H), 6.78 ( s, 2H), 3.02 (s, 6H), 2.71 (s, 1H), 1.88 - 1.82 (m, 4H), 1.80 (s, 2H), 1.70 - 1.63 (m, 2H), 1.60 - 1.51 (m, 2H), 1.45 - 1.38 (m, 2H).
LC-MS (ESI) : $[M+H]^+$ = 394.39

Step 9: Synthesis of 7-((3-amino-6-(2-hydroxyphenyl)pyridazin-4-yl)ethynyl)spiro[3.5]nonan-2-one (Compound 13)

Intermediate 13h (100 mg, 254.14 μmol) was dissolved in dichloromethane (2 mL), and trifluoroacetic acid (375μL, 5.08 mmol) was added and reacted for 2 h. After the reaction was completed, the reaction solution was concentrated directly to obtain Compound 13 (76 mg, 91.83%) as a yellow solid.

$^1$H NMR (600 MHz, Methanol-$d_4$) δ 8.34 (d, J = 8.6 Hz, 1H), 7.67 (dd, J = 8.1, 1.8 Hz, 1H), 7.39 (td, J = 7.7, 7.3, 1.6 Hz, 1H), 7.01 (dd, J = 7.9, 6.5 Hz, 2H), 2.82 (dt, J = 13.0, 1.9 Hz, 4H), 2.12 - 1.85 (m, 5H), 1.82 - 1.66 (m, 4H).
LC-MS (ESI) : $[M+H]^+$ = 348.35

**Example 15: Synthesis of 6-((3-amino-6-(2-hydroxyphenyl)pyridazin-4-yl)ethynyl)spiro[3.3]heptan-2-one (Compound 14)**

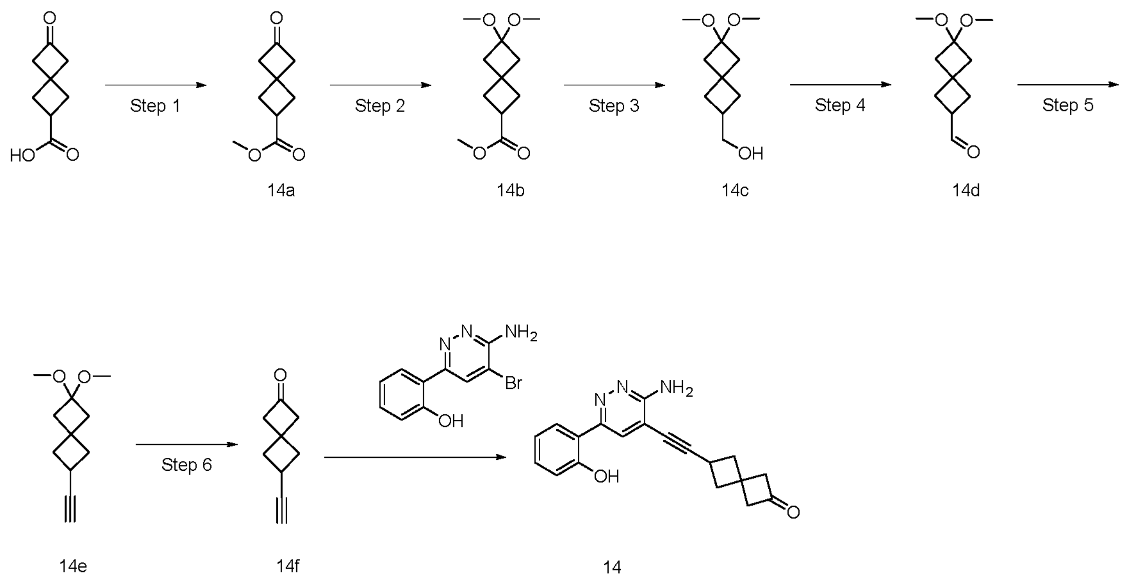


14a 14b 14c 14d

14e 14f 14

Synthesis scheme:

Step 1: Synthesis of compound methyl 6-oxospiro[3.3]heptane-2-carboxylate (Intermediate 14a)

6-Oxospiro[3.3]heptane-2-carboxylic acid (5.00 g, 32.43 mmol) was dissolved in a single-necked vial containing DMF (50 mL), and iodomethane (4.60 g, 32.43 mmol) and potassium carbonate (4.48 g, 32.43 mmol) were added sequentially, and the reaction was stirred at room temperature for 5h. After the reaction was completed, the reaction was quenched by addition of water, and extracted three times with ethyl acetate (50 mL), the crude product was concentrated

by drying and filtering with anhydrous sodium sulfate. The crude product was purified by column (0-20% ethyl acetate) to obtain colorless oily Intermediate 14a (3.83 g).Step 2: Synthesis of compound 6,6-dimethoxyspiro[3.3]heptane-2-carboxylic acid methyl ester (Intermediate 14b)

Intermediate 14a (3.00 g, 17.84 mmol) was dissolved in a single-necked vial containing MeOH (30 mL), trimethyl orthoformate (5.68 g, 53.51 mmol) and pyridinium p-toluenesulfonate (896.48 mg, 3.57 mmol) were added sequentially, and the reaction was stirred at room temperature for 5h. After the reaction was completed, the reaction was quenched by addition of water, and extracted three times with ethyl acetate (50 mL) was concentrated by drying and filtering with anhydrous sodium sulfate to obtain the crude product. The crude product was purified by column (0-30% ethyl acetate) to obtain the colorless oily Intermediate 14b (2.93 g).

Step 3: Synthesis of compound 6,6-dimethoxyspiro[3.3]heptane-2-methanol (Intermediate 14c)

Methyl 6,6-dimethoxyspiro[3.3]heptane-2-carboxylate (3.00g, 14.00 mmol) was dissolved in a single-necked flask containing tetrahydrofuran (30 mL), cooled down to 0 °C, and lithium tetrahydroaluminate (1.06 g, 28.00 mmol) was slowly added, and stirred at 0 °C for 0.5 h. The reaction was transferred to room temperature, and the stirring was continued for 5 h. After the reaction was completed, it was cooled down to 0 °C, quenched with ice water and extracted with ethyl acetate three times (40 mL), the organic phase was washed with saturated brine, dried with anhydrous sodium sulfate, filtered and concentrated to obtain a crude product. The crude product was purified by column (0-50% ethyl acetate) to obtain colorless oily Intermediate 14c (2.35 g).

Step 4: Synthesis of compound 6,6-dimethoxyspiro[3.3]heptane-2-carbaldehyde (Intermediate 14d)

Methyl 6,6-dimethoxyspiro[3.3]heptane-2-carboxylate (2.17 g, 11.65 mmol) was dissolved in a single-necked vial containing acetonitrile (30 mL), and 2-iodoylbenzoic acid (6.52 g, 23.30 mmol) was slowly added, and the reaction was stirred for 2h at 80 °C under the atmosphere of $N_2$. After the reaction was completed, it was cooled down to room temperature, and then concentrated by filtration to obtain the crude product. The crude product was purified by column (0-20% ethyl acetate) to obtain a colorless oily Intermediate 14d (1.63 g).

Step 5: Synthesis of compound 6-ethynyl-2,2-dimethoxyspiro[3.3]heptane (Intermediate 14e)

6,6-Dimethoxyspiro[3.3]heptane-2-carboxaldehyde (2.15 g, 11.67 mmol) was dissolved in a single-necked vial containing methanol (20 mL), cooled down to 0 °C, and potassium carbonate (3.23 g, 23.34 mmol) was slowly added, and stirring was maintained at 0 °C for 10 min. 1-Diazo-2-oxopropyl)phosphonic acid dimethyl ester (3.36 g, 17.50 mmol) was added, and continue stirring for 4h. After the reaction was completed, the crude product was filtered and concentrated. The crude product was purified by column (0-20% ethyl acetate) to obtain colorless oily Intermediate 14e (1.03 g).

Step 6: Synthesis of compound spiro[3.3]heptan-2-one, 6-ethynyl (Intermediate 14f)

6-Ethynyl-2,2-dimethoxyspiro[3.3]heptane (1.00 g, 5.55 mmol) was dissolved in a glass vial containing dichloromethane (12 mL). Trifluoroacetic acid (3 ml) was slowly added, and stirred at room temperature for 2h. After the reaction was completed, the crude product was filtered and concentrated. The crude product was purified by column (0-20% ethyl acetate) to obtain the colorless oily compound Intermediate 14f (836.00 mg).
$^1$H NMR (400 MHz, DMSO) δ 3.11 (d, *J* = 2.1 Hz, 2H), 3.06 (t, *J* = 2.8 Hz, 2H), 3.01 (d, *J* = 2.2 Hz, 1H), 2.99 (dd, *J* = 8.2, 2.4 Hz, 1H), 2.50 - 2.46 (m, 3H), 2.30 - 2.24 (m, 1H).

Step 7: Synthesis of 6-((3-amino-6-(2-hydroxyphenyl)pyridazin-4-yl)ethynyl)spiro[3.3]heptan-2-one Intermediate 18 (100 mg, 375 μmol), 6-ethynylspiro[3.3]heptan-2-one (60.51 mg, 451 μmol), bis(triphenylphosphine)dichloropalladium (26.38 mg, 37.5 μmol), cuprous iodide (7.16 mg, 37.5 μmol) and triethylamine (208 μL, 1.5 mmol) were dissolved in a solution with DMF (4mL), protected by nitrogen, and then the reaction was raised to 110 °C for 2h. After the reaction was completed, the diatomaceous earth was filtered, 16 mL of purified water was added to the filtrate, and the organic phase was extracted with ethyl acetate three times (4 mL x 3) and washed with saturated saline, and then dried with anhydrous sodium sulfate, filtered, and concentrated to obtain a crude product. The crude product was purified on column (petroleum ether: ethyl acetate = 2:1) to obtain the yellow solid Compound 14 (42 mg, 35%).

$^1$H NMR (600 MHz, Methanol- *$d_4$*) δ 8.09 (d, J = 12.8 Hz, 1H), 7.80 - 7.69 (m, 1H), 7.31 - 7.23 (m, 1H), 6.94 (t, J = 7.1 Hz, 2H), 3.45 (p, J = 8.4 Hz, 1H), 3.24 - 3.13 (m, 3H), 2.86 - 2.46 (m, 4H), 1.51 - 1.19 (m, 4H).
LC-MS (ESI) : $[M+H]^+$ = 320.30

**Example 16: Synthesis of 2-(6-amino-5-((1-methoxy-4-(pyrrolidin-1-yl)cyclohexyl)ethynyl)pyridazin-3-yl)phenol (Compound 15)**

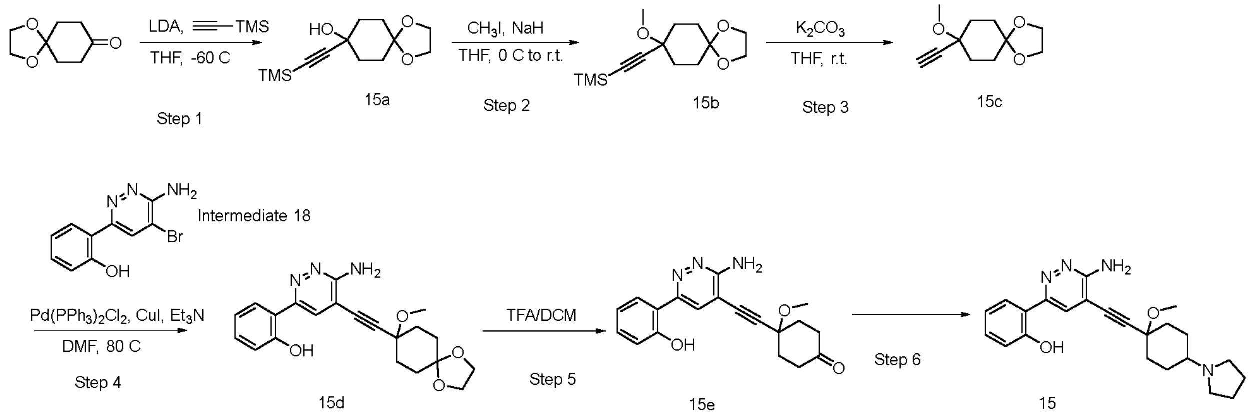


Step 1: Synthesis of 8-((trimethylsilyl)ethynyl)-1,4-dioxaacetyl[4.5]decan-8-ol (Intermediate 15a)

1,4-Dioxaspiro[4.5]decan-8-one (5.00 g, 32.01 mmol) and trimethylsilylacetylene (4.72 g, 48.02 mmol) were dissolved in a triplex vial containing tetrahydrofuran (20 mL) protected by nitrogen, then cooled down to 0 °C, and LDA (22.41 mL, 44.41 mmol) was slowly added, and stirring was maintained at 0 °C for 0.5h. The reaction was transferred to room temperature and continued stirring. After the reaction was completed, the temperature was lowered to 0 °C, quenched by adding purified water, the excess solvent was removed under reduced pressure, and the organic phase was extracted with ethyl acetate for three times (20 mL), washed with saturated brine, dried with anhydrous sodium sulfate, filtered and concentrated to obtain the crude product. The crude was purified by column (petroleum ether: ethyl acetate = 5:1) to obtain the colorless oily Intermediate 15a (5.4 g, 66.3%).
$^1$H NMR (600 MHz, $CDCl_3$) δ 3.94 (s, 4H), 2.04 (s, 1H), 1.97 - 1.94 (m, 2H), 1.91 - 1.86 (m, 2H), 1.78 (t, J = 4.9 Hz, 4H), 0.16 (s, 9H).

Step 2: Synthesis of ((8-methoxy-1,4-dioxaspiro[4.5]dec-8-yl)ethynyl)trimethylsilane (Intermediate 15b)

Intermediate 15a (5.00 g, 20.63 mmol) was dissolved in a three-necked flask containing tetrahydrofuran (20 mL), cooled down to 0 °C, slowly added sodium hydrogen (907.54 mg, 22.69 mmol), maintained at 0 °C and stirred for 0.5 h. Iodomethane (5.86 g, 41.26 mmol) was slowly added, maintained at 0 °C and stirred for 0.5 h, then transferred to room temperature, and continued stirring for 1 h. After the reaction was completed, the temperature was lowered to 0 °C, quenched by adding purified water, the excess solvent was removed under reduced pressure, and the organic phase was extracted three times (20 mL) with ethyl acetate, washed with saturated brine, dried with anhydrous sodium sulfate, filtered, and concentrated to obtain a crude product. The crude product was purified by column (petroleum ether:ethyl acetate=5:1) to obtain colorless oily Intermediate 15b (4.8 g, 90.98%).

Step 3: Synthesis of 8-ethynyl-8-methoxy-1,4-dioxaspiro[4.5]decane (Intermediate 15c)

Intermediate 15b (4.80 g, 17.88 mmol) and potassium carbonate (4.94 g, 35.76 mmol) were dissolved in a three-necked flask containing methanol (20 mL) and stirred for 1h. After the reaction was completed, the excess solvent was removed under reduced pressure, purified water was added, and the organic phase was extracted three times with ethyl acetate (20 mL), washed with saturated brine, and dried with anhydrous sodium sulfate, filtered, and concentrated to obtain a crude product. The crude product was purified by column (petroleum ether:ethyl acetate=5:1) to obtain the colorless oily Intermediate 15c (2.7 g, 76.94%).
$^1$H NMR (600 MHz, Chloroform-d) δ 3.94 (s, 4H), 2.73 (s, 1H), 1.94 (dd, *J* = 12.3, 6.2 Hz, 2H), 1.91 - 1.83 (m, 2H), 1.80 - 1.73 (m, 4H), 0.15 (s, 9H).

Step 4: Synthesis of 2-(6-amino-5-((8-methoxy-1,4-dioxaspiro[4.5]dec-8-yl)ethynyl)pyridazin-3-yl)phenol (Intermediate 15d)

Intermediate 15c (2.35 g, 8.83mmol), SM-I-XL-006 (1.73 g, 8.83mmol), bis(triphenylphosphine)dichloropalladium (619.87 mg, 883.13μmol), cuprous iodide (168.19 mg, 883.13μmol) and triethylamine (1.79g, 17.66mmol) were dissolved in a three-necked flask containing DMF (20 mL), protected by nitrogen, and heated to 80 °C with stirring overnight. After the reaction was completed, purified water was added and the organic phase was extracted with ethyl acetate three times (20 mL) and washed with saturated brine, dried with anhydrous sodium sulfate, filtered, and concentrate to obtain a crude product. The crude product was purified by column (petroleum ether: ethyl acetate = 1:1) to obtain a yellow solid Intermediate 15d (630 mg, 13.79%).

$^1$H NMR (400 MHz, Chloroform-d) δ 7.92 (s, 1H), 7.61 (dd, *J* = 8.0, 1.5 Hz, 1H), 7.33 (ddd, *J* = 8.5, 7.2, 1.5 Hz, 1H), 7.08 (dd, *J* = 8.3, 1.1 Hz, 1H), 6.98 - 6.92 (m, 1H), 5.37 (s, 2H), 4.00 (d, *J* = 1.9 Hz, 4H), 3.47 (s, 3H), 2.21 - 2.08 (m, 4H), 1.89 (ddd, *J* = 13.6, 7.9, 5.8 Hz, 2H), 1.82 - 1.71 (m, 2H).
LC-MS (ESI) : $[M+H]^+$ = 382.19

Step 5: Synthesis of 4-((3-amino-6-(2-hydroxyphenyl)pyridazin-4-yl)ethynyl)-4-methoxycyclohexan-1-one (Intermediate 15e)

Intermediate 15d (630 mg, 1.65mmol) was dissolved in dichloromethane (2.5 mL) in a three-necked vial, trifluoroacetic acid (2.5mL) was added, and the reaction was stirred for 1h. After the reaction was completed, the excess solvent was removed under reduced pressure, purified water was added, and the organic phase was extracted three times with ethyl acetate (20mL), washed with saturated brine, and dryied with anhydrous sodium sulfate, filtered, and concentrated to obtain a crude product. The crude product was concentrated by drying and filtration using anhydrous sodium sulfate. The crude product was purified by column (petroleum ether:ethyl acetate=1:1) to obtain the yellow solid Compound 15e (425 mg, 76.26%).

$^1$H NMR (400 MHz, Chloroform-d) δ 7.94 (s, 1H), 7.61 (dd, *J* = 8.0, 1.6 Hz, 1H), 7.33 (ddd, *J* = 8.5, 7.2, 1.6 Hz, 1H), 7.09 (dd, *J* = 8.2, 1.2 Hz, 1H), 6.99 - 6.91 (m, 1H), 5.39 (s, 2H), 3.57 (s, 3H), 2.72 - 2.58 (m, 2H), 2.46 (ddt, *J* = 15.7, 9.9, 4.5 Hz, 4H), 2.32 - 2.21 (m, 2H).
LC-MS (ESI) : $[M+H]^+$ = 338.29

Step 6: Synthesis of 2-(6-amino-5-((1-methoxy-4-(pyrrolidin-1-yl)cyclohexyl)ethynyl)pyridazin-3-yl)phenol (Compound 15)

15e (40 mg, 118.58 μmol, 1.0 eq), pyrrole (10.12 mg, 142.27 μmol), AcOH (6 d) and $Et_3N$ (6d) were dissolved in a reaction vial containing DCE/DMSO (1/1 mL) and reacted for 2h at room temperature. $NaBH(OAc)_3$ ( 75.38 mg, 355.68 μmol, 3.0 eq) was then added and reacted overnight at room temperature. After the reaction was completed, the final product 15 (16 mg, 36.10%) was purified by HPLC.

$^1$H NMR (400 MHz, Methanol-*$d_4$*) δ 8.48 (s, 1H), 7.94 (d, J = 8.2 Hz, 1H), 7.29 (t, J = 8.4 Hz, 1H), 6.98 (t, J = 7.3 Hz, 2H), 6.59 (s, 1H), 3.41 (s, 3H), 3.12 (s, 3H), 2.51 (d, J = 10.1 Hz, 2H), 2.16 (d, J = 7.0 Hz, 2H), 2.06 (d, J = 26.5 Hz, 5H), 1.92 (t, J = 9.5 Hz, 4H).
LC-MS (ESI): $[M+H]^+$ = 393.40

**Example 17: Synthesis of (1s,4s)-4-((3-amino-6-(2-hydroxyphenyl)pyridazin-4-yl)ethynyl)cyclohexane-1-carbaldehyde (Compound 16)**

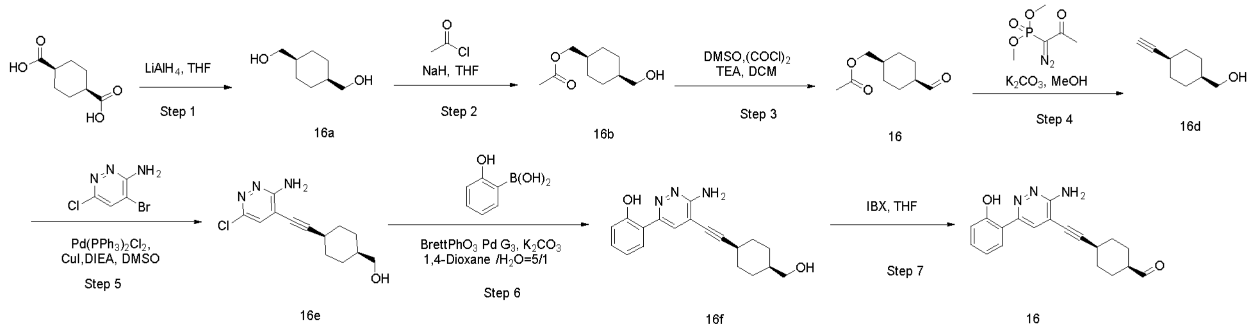


16a 16b 16 16d 16e 16f 16

Step 1: Synthesis of cis-1,4-cyclohexanedimethanol (Intermediate 16a)

Cis-1,4-cyclohexanedicarboxylic acid (15.0 g, 87.12 mmol) was dissolved in a reaction vial containing THF (500 mL), LiAlH4 (9.92 g, 261.35 mmol) was added dropwise to the reaction vial under ice-water bath. After the reaction was completed, it was quenched by the addition of $Na_2SO_4$-$10H_2O$ solution and the organic phase was extracted with ethyl acetate, dried with anhydrous sodium sulfate, filtered, and concentrated to obtain a crude product. The crude product was purified by column (petroleum ether: ethyl acetate=1:2) to obtain colorless oily Intermediate 16a (9.15 g, 72.83%).
$^1$H NMR (400 MHz, $CDCl_3$) $\delta$ 3.55 (d, J = 6.9 Hz, 4H), 1.74 - 1.67 (m, 2H), 1.55 (ddt, J = 13.0, 6.8, 2.6 Hz, 6H), 1.43 (dd, J = 11.6, 5.7 Hz, 4H).

Step 2: Synthesis of cis-4-acetic acid methyl ester-cyclohexane-1-methanol (Intermediate 16b)

Cis-1,4-cyclohexanedimethanol (9.10 g, 63.10 mmol) was dissolved in a reaction flask containing THF (150 mL), NaH (2.52 g, 60% dispersion in mineral oil) was added to the reaction flask under ice-water bath, and the reaction was carried out at 0 °C for 0.5h followed by the gradual dropwise addition of acetochlorine. After the reaction was completed, the reaction was quenched by the addition of purified water and the organic phase was extracted with ethyl acetate, dried with anhydrous sodium sulfate, filtered, and concentrated and concentrated to obtain a crude product. The crude product was purified by column (petroleum ether: ethyl acetate = 2:1) to obtain the light yellow oily Intermediate 16b (6.30 g, 53.61%).
$^1$H NMR (600 MHz, $CDCl_3$) $\delta$ 3.99 (d, J = 7.3 Hz, 2H), 3.53 (d, J = 6.9 Hz, 2H), 2.06 (d, J = 1.2 Hz, 3H), 1.87 (ddq, J = 10.1, 7.0, 2.9 Hz, 1H), 1.68 (pt, J = 7.1, 4.1 Hz, 1H), 1.54 (ddp, J = 13.5, 9.5, 4.8 Hz, 4H), 1.42 (tdd, J = 12.6, 10.7, 8.8, 5.3 Hz, 4H), 1.25 (s, 1H).

Step 3: Synthesis of cis-4-acetic acid methyl ester-cyclohexane-1-carbaldehyde (Intermediate 16c)

DMSO (6.61 g, 84.56 mmol) was dissolved in a three-necked reaction vial containing DCM (50 mL), oxalyl chloride (5.51 g, 40.59 mmol) was added dropwise to the reaction vial at -78 °C, followed by cis-4-acetic acid methyl ester-cyclohexane-1-carboxaldehyde (6.30 g, 33.83 mmol) dissolved in DCM (30 mL) and added dropwise to the reaction vial at -78 °C. After reaction for 1h, triethylamine (17.11 g, 17.11 g, 17.11 g) was added to the reaction vial. After 1h, triethylamine (17.11 g, 169.13 mmol) was added to the reaction vial for 0.5h under nitrogen atmosphere. After the reaction was completed, the reaction vial was removed from the cold bath and quenched by the addition of hydrochloric acid solution (1 M) at -10 °C. The reaction was carried out in cold bath. The organic phase was extracted with ethyl acetate, and concentrated to obtain a crude yellow oily intermediate 16c (6.2 g). The compound is not stable and does and could be directly used in the next reaction without purification.

Step 4: Synthesis of cis-4-acetylene-cyclohexane-1-methanol (Intermediate 16d)

Cis-4-acetylmethyl-cyclohexane-1-carboxaldehyde (6.20 g, 33.82 mmol) was dissolved in a reaction vial containing methanol (60 mL), dimethyl (1-diazido-2-oxopropyl)phosphonate (8.45 g, 43.96 mmol) was added dropwise to the reaction vial at 0 °C, followed by potassium carbonate (14.02 g, 101.45 mmol) was added to the reaction solution and reacted at room temperature overnight. After the reaction was completed, the organic phase was extracted with ethyl acetate and concentrated to obtain the crude product, which was purified by column (petroleum ether: ethyl acetate=1:5) to obtain the colorless oily Intermediate 16d (3.86 g, 62.59%).
$^1$H NMR (600 MHz, $CDCl_3$) $\delta$ 3.49 (d, J = 6.1 Hz, 3H), 3.45 (d, J = 6.3 Hz, 1H), 2.81 - 2.76 (m, 1H), 2.05 (d, J = 2.4 Hz, 3H), 1.82 (d, J = 12.9 Hz, 4H), 1.63 (dt, J = 12.0, 3.7 Hz, 4H), 1.25 (s, 6H).

Step 5: Synthesis of cis-4-((3-amino-6-chloropyridazin-4-yl)ethynyl)cyclohexyl)methanol (Intermediate 16e)

3-Amino-4-bromo-6-chloropyridazine (5.10 g, 24.47 mmol), cis-4-ethynyl-cyclohexane-1-methanol (4.06 g, 29.36 mmol), Pd $(PPh3)_2Cl_2$ (1.72 g, 2.45 mmol), CuI (1.40 g, 7.34 mmol) and DIEA (15.81 g, 122.34 mmol) were dissolved in a reaction vial containing DMSO (100 mL) and reacted at 80 °C for 3 h under nitrogen atmosphere. After the reaction was completed, the organic phase was washed with saturated saline and extracted with ethyl acetate and concentrated to obtain a crude product, which was purified by column (petroleum ether: ethyl acetate=1:2) to obtain the colorless oily Intermediate 16e (3.14 g, 48.29%).
LC-MS (ESI): $[M+H]^+$ = 266.37

Step 6: Synthesis of 2-(6-amino-5-((cis-4-(hydroxymethyl)cyclohexyl)ethynyl)pyridazin-3-yl)phenol (Intermediate 16f)

Intermediate 16e (3.14 g, 11.82 mmol), 2-hydroxyphenylboronic acid (4.89 g, 35.45 mmol), BrettPhO3 Pd G3 (1.07 g, 1.18 mmol) and $K_2CO_3$ (4.90 g, 35.45 mmol) were dissolved in a reaction flask containing 1,4-dioxane /$H_2O$=5/1 (84 mL) in a reaction flask, and the reaction was carried out overnight at 80 °C under nitrogen atmosphere. After the reaction was completed, the organic phase was washed with saturated saline and extracted with ethyl acetate and concentrated to obtain a crude product, which was purified by column (petroleum ether:ethyl acetate=1:3) to obtain colorless oily Intermediate 16f (1.40 g, 36.64%).

$^1$H NMR (400 MHz, DMSO-$d_6$) δ 8.17 (s, 1H), 7.80 (dd, J = 7.9, 1.7 Hz, 1H), 7.30 - 7.24 (m, 1H), 6.96 - 6.86 (m, 2H), 4.25 (d, J = 6.1 Hz, 1H), 3.22 (d, J = 6.2 Hz, 2H), 2.08 (dt, J = 13.0, 7.4 Hz, 2H), 1.86 - 1.82 (m, 1H), 1.80 - 1.72 (m, 2H), 1.49 (ddd, J = 15.6, 10.1, 3.9 Hz, 2H), 1.37 (tq, J = 8.8, 3.4, 2.8 Hz, 1H), 0.96 (qd, J = 13.1, 3.5 Hz, 2H).
LC-MS (ESI): $[M+H]^+$ = 324.02

Step 7: Synthesis of cis-4-((3-amino-6-(2-hydroxyphenyl)pyridazin-4-yl)ethynyl)cyclohexane-1-carbaldehyde (Compound 16)

Intermediate 16f (100 mg, 309.22 μmol) and IBX (173.17 g, 618.44 μmol) were dissolved in a reaction vial containing THF (10 mL) and reacted at 50 °C for 2h. After the reaction was completed, Compound 16 was obtained by filtration.
LC-MS (ESI): $[M+H]^+$ = 322.30

**Example 18: Synthesis of 2-(6-amino-5-((4-methoxyphenyl)ethynyl)pyridazin-3-yl)phenol (Compound 17)**

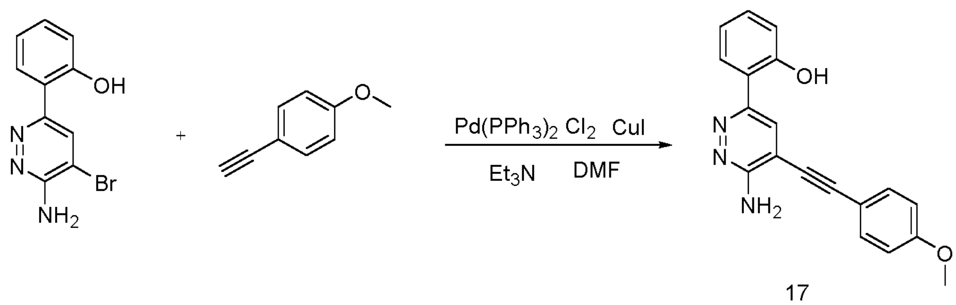


17

Step 1: synthesis of 6-((3-amino-6-(2-hydroxyphenyl)pyridazin-4-yl)ethynyl)spiro[3.3]heptan-2-one

Intermediate 18 (33 mg, 125 μmol), 1-ethynyl-4-methoxybenzene (20 mg, 150 μmol), bis(triphenylphosphine) dichloropalladium (8.78 mg, 12.5 μmol), cuprous iodide (2.38 mg, 12.5 μmol), and triethylamine (70 μL, 0.5 mmol) were dissolved in DMF (1 mL) under nitrogen. After protection, the reaction was heated up to 110 °C for 40mins. After the reaction was completed, the diatomaceous earth was filtered, 4mL of purified water was added to the filtrate, and the organic phase was extracted with ethyl acetate three times (4 mL x 3) and washed with saturated saline, and then dried with anhydrous sodium sulfate, filtered, and concentrated to obtain a crude product. The crude product was purified on a column (petroleum ether: ethyl acetate = 2:1) to obtain Compound 17 as a solid (10 mg, 25%).

$^1$H NMR (600 MHz, Methanol- $d_4$) δ 8.41 (s, 1H), 7.72 (d, J = 8.6 Hz, 3H), 7.40 (s, 1H), 7.12 - 6.87 (m, 4H), 3.88 (s, 3H).

LC-MS (ESI) : $[M+H]^+$ = 318.28

**Example 19: Synthesis of 4-((3-amino-6-(2-hydroxyphenyl)pyridazin-4-yl)ethynyl)cyclohexan-1-one (Compound 18)**

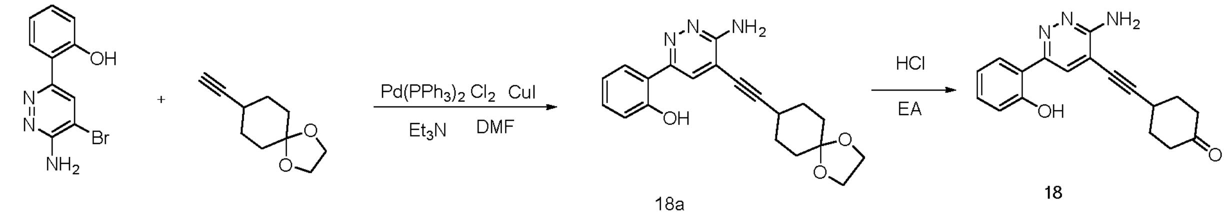


18a

18

Step 1: Synthesis of 2-(5-(1,4-dioxaspiro[4.5]decan-8-yl)ethynyl)-6-aminopyridazin-3-yl)phenol

Intermediate 18 (500mg, 1.88mmol), 8-ethynyl-1,4-dioxaspiro[4.5]decane (374.8 mg, 2.25 mmol), bis(triphenylphosphine)dichloropalladium (131.89 mg,187.90 μmol), cuprous iodide (35.8 mg, 187.90μmol) and triethylamine (970 μL, 7.52 mmol) were dissolved in DMF (20 mL), protected by nitrogen, and the temperature was raised to 110 °C for 2h. After the reaction was completed, the diatomaceous earth was filtered, 80 mL of purified water was added to the filtrate, and the organic phase was extracted three times (20 mL x 3) with ethyl acetate, washed with saturated saline, and concentrated by drying and filtration using anhydrous sodium sulfate to obtain the crude product. The crude product was purified by column (petroleum ether: ethyl acetate = 2:1) to obtain solid Compound 18a (404 mg, 61.18%).

$^1$H NMR (600 MHz, Methanol- *d$_4$*) δ 8.08 (s, 1H), 7.78 - 7.73 (m, 1H), 7.27 (td, J = 7.7, 1.5 Hz, 1H), 6.94 (t, J = 7.4 Hz, 2H), 3.97 (d, J = 2.7 Hz, 4H), 2.87 (dp, J = 8.4, 3.7 Hz, 1H), 2.08 - 2.01 (m, 2H), 1.88 (tdd, J = 12.6, 8.4, 3.7 Hz, 4H), 1.66 (ddd, J = 13.6, 10.3, 4.0 Hz, 2H).
LC-MS (ESI) : $[M+H]^+$ = 352.30

Step 2: Synthesis of 4-((3-amino-6-(2-hydroxyphenyl)pyridazin-4-yl)ethynyl)cyclohexan-1-one Intermediate 18a (404mg, 1.15mmol) was dissolved in 6mL of tetrahydrofuran and reacted overnight with 2mL of concentrated hydrochloric acid and 1mL of water. The tetrahydrofuran in the system was spun dry and the pH was adjusted to a weak base by adding appropriate amount of water and sodium bicarbonate solid. The organic phase was extracted three times with ethyl acetate (20 mL x 3) and dried with anhydrous sodium sulfate, filtered, and concentrated to obtain the crude product. The crude product was purified by column (petroleum ether: ethyl acetate = 2;1) to obtain solid Compound 18 (250 mg, 79.24%).

$^1$H NMR (400 MHz, Methanol- *d$_4$*) δ 8.18 - 8.10 (m, 1H), 7.75 (dt, J = 8.2, 2.1 Hz, 1H), 7.28 (td, J = 7.7, 1.6 Hz, 1H), 6.98 - 6.91 (m, 2H), 3.29 (dq, J = 7.9, 3.9 Hz, 1H), 2.62 (dt, J = 13.5, 6.1 Hz, 1H), 2.47 (ddd, J = 14.5, 9.0, 5.4 Hz, 1H), 2.29 (ddt, J = 12.9, 9.6, 5.1 Hz, 1H), 2.19 - 2.08 (m, 1H), 1.97 (ddt, J = 16.5, 13.1, 4.8 Hz, 2H), 1.88 - 1.74 (m, 1H), 1.67 (dq, J = 19.1, 9.6, 8.3 Hz, 1H).
LC-MS (ESI) : $[M+H]^+$ = 308.38

**Example 20: Synthesis of (1R,4R)-4-(3-amino-6-(2-hydroxyphenyl)pyridazin-4-yl)ethynyl)cyclohexane-1-carboxylic acid (Compound 19)**

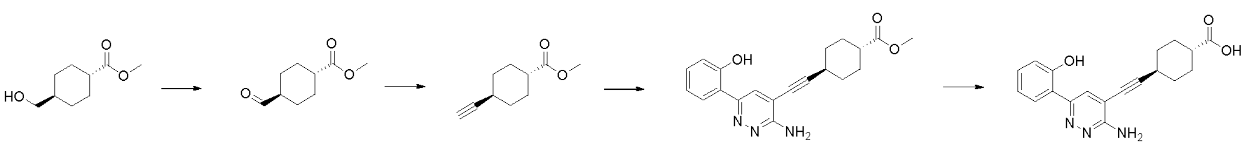

19a

19b

19c

19

Step 1: Synthesis of methyl (1R,4R)-4-formylcyclohexane-1-carboxylate

Methyl (1R,4R)-4-formylcyclohexane-1-carboxylate (500 mg, 2.9 mmol), 2-iodoacylbenzoic acid (1.63 g,5.81 mmol) were dissolved in acetonitrile (5 mL), protected by nitrogen, and then the reaction was warmed up to 80 °C for 1h. After the reaction was completed, the crude product 19a (506 mg, 102.4%) was obtained by filtration and concentration.

Step 2: Synthesis of methyl (1R,4R)-4-ethynylcyclohexane-1-carboxylate

19a (506 mg, 2.97mmol) was dissolved in methanol (5mL), potassium carbonate (821.72 mg, 5.95 mmol) was added, and dimethyl (1-diazido-2-oxopropyl)phosphonate (742.45 mg, 3.86 mmol) was added dropwise under ice bath, the reaction was carried out at room temperature overnight. After the reaction was completed, the reaction solution was concentrated and purified by column (petroleum ether: ethyl acetate=10:1) to obtain the white crystalline solid 19b (389.4 mg, 78.80%).

$^1$H NMR (600 MHz, Methanol- *$d_4$*) δ 3.66 (d, J = 0.7 Hz, 3H), 2.39 - 2.34 (m, 1H), 2.33 (dd, J = 2.4, 0.7 Hz, 1H), 2.30 - 2.24 (m, 1H), 2.04 - 1.96 (m, 4H), 1.49 - 1.38 (m, 4H).

Step 3: Synthesis of methyl (3-amino-6-(2-hydroxyphenyl)pyridazin-4-yl)ethynyl)cyclohexane-1-carboxylate

Intermediates 18 (50 mg, 188 μmol), 19b (37.38mg,225.5μmol), bis(triphenylphosphine)dichloropalladium (13mg,18.8μmol), cuprous iodide (3.58 mg, 18.80 μmol), and triethylamine (104μL, 751.6μmol) were dissolved in DMF (2 mL) with nitrogen protection and then the reaction was carried out at 110 °C for 1h. After the reaction was completed, the diatomaceous earth was filtered, 8 mL of purified water was added to the filtrate, and the organic phase was extracted three times (2 mL x 3) with ethyl acetate, washed with saturated saline, and dried with anhydrous sodium sulfate, filtered and concentrated to obtain the crude product. The crude product was purified by column (petroleum ether: ethyl acetate = 2;1) to obtain solid 19c (32 mg, 48.46%).

$^1$H NMR (600 MHz, Methanol- *$d_4$*) δ 8.08 (s, 1H), 7.75 (d, J = 8.0 Hz, 1H), 7.27 (t, J = 7.2 Hz, 1H), 6.94 (t, J = 7.8 Hz, 2H), 3.78 - 3.60 (m, 3H), 2.69 (t, J = 11.5 Hz, 1H), 2.42 (d, J = 11.6 Hz, 1H), 2.19 (d, J = 12.7 Hz, 2H), 2.12 - 1.99 (m, 2H), 1.58 (dq, J = 44.3, 12.2 Hz, 4H).
LC-MS (ESI) : $[M+H]^+$ = 352.39

Step 4 : Synthesis of (1R,4R)-4-((3-amino-6-(2-hydroxyphenyl)pyridazin-4-yl)ethynyl)cyclohexane-1-carboxylic acid

19c (118.2 mg, 336 μmol), LiOH (80.55 mg,3.36 mmol) were dissolved in THF/H2O=1:1 (4 mL) and stirred for 1h at room temperature. After the reaction was completed, the pH was adjusted to 4 by dropwise addition of dilute hydrochloric acid and a solid was precipitated, which was filtered to obtain yellow solid Compound 19 (113.8 mg, 100.28%).

$^1$H NMR (600 MHz, Methanol- *$d_4$*) δ 8.10 (d, J = 24.5 Hz, 1H), 7.76 (dd, J = 15.1, 7.9 Hz, 1H), 7.32 - 7.23 (m, 1H), 6.94 (t, J = 7.5 Hz, 2H), 3.76 - 3.72 (m, 1H), 2.20 (dd, J = 13.1, 4.1 Hz, 1H), 2.08 (ddd, J = 14.3, 9.4, 5.0 Hz, 1H), 2.04 - 1.92 (m, 2H), 1.89 (td, J = 11.3, 9.0, 5.7 Hz, 2H), 1.80 (ddt, J = 14.3, 10.8, 3.7 Hz, 1H), 1.66 - 1.48 (m, 3H).
LC-MS (ESI) : $[M+H]^+$ = 338.29

**Example 21: Synthesis of 2-(6-amino-5-((1R,5S,6s)-6-(dimethylamino)-3-azabicyclo[3.1.0]hexan-3-yl)pyridazin-3-yl)phenol (Compound 20)**

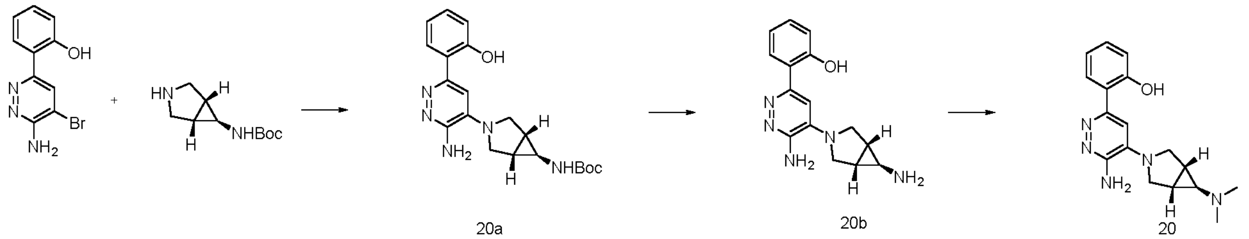

20a 20b 20

Step 1 : Synthesis of tert-butyl (1R, 5S, 6S)-3-(3-amino-6-(2-hydroxyphenyl)pyridazin-4-yl)-3-azabicyclo[3.1.0]hexan-6-yl)carbamate

Intermediate 18 (200mg, 751.60μmol), tert-butyl (1R, 5S, 6S)-rel-3-azabicyclo[3.1.0]hexan-6-ylcarbamate (447mg, 2.25mmol), and DIEA (398mg, 3.08mmol) were dissolved in a reaction vial containing acetonitrile (5mL), and the reaction was carried out at 80 °C for 12h. After the reaction was completed, 20a (148 mg, 51.35%) was purified by column chromatography.

$^1$H NMR (400 MHz, DMSO-$d_6$) δ 14.52 (s, 1H), 7.98 - 7.87 (m, 1H), 7.23 (td, J = 7.7, 1.5 Hz, 1H), 7.12 (d, J = 8.3 Hz, 2H), 6.87 (t, J = 7.3 Hz, 2H), 6.01 (s, 2H), 4.11 (q, J = 5.3 Hz, 1H), 3.82 (d, J = 10.1 Hz, 2H), 3.17 (d, J = 5.0 Hz, 2H), 1.74 (d, J = 2.6 Hz, 2H), 1.40 (s, 9H).
LC-MS (ESI): $[M+H]^+$=384.49

Step 2: Synthesis of 2-(6-amino-5-((1R,5S,6s)-6-amino-3-azabicyclo[3.1.0]hexan-3-yl)pyridazin-3-yl)phenol

20a (100mg, 260.8μmol) was dissolved in a reaction flask containing tetrahydrofuran (5 mL), to which HCl (1mL, 4M in dioxane) was added and carried out for 12h at room temperature. After the reaction was completed, 20b (72 mg, 97.43%) was concentrated under reduced pressure. The crude product was not purified and directly subjected to the next step of the reaction.
LC-MS (ESI): $[M+H]^+$=284.28

Step 3: Synthesis of 2-(6-amino-5-((1R,5S,6s)-6-(dimethylamino)-3-azabicyclo[3.1.0]hex-3-yl)pyridazin-3-yl)phenol

20b (70mg, 247.06μmol) was dissolved in a reaction vial containing methanol (6mL), to which DIEA (141mg, 1.09mmol) and aqueous formaldehyde solution (70μL, 741.18μmol) were added and reacted for 2 h at room temperature. Then $NaBH_3CN$ (49mg, 741.18μmol) was added to the reaction system and the reaction was continued for 12h at room temperature. After the reaction was completed, the crude product was concentrated under reduced pressure and purified by HPLC to obtain Compound 20 (39 mg, 50.69%).

$^1$H NMR (400 MHz, Methanol-$d_4$) δ 7.55 (dd, J = 7.7, 1.6 Hz, 1H), 7.48 - 7.43 (m, 1H), 7.08 - 7.03 (m, 3H), 4.23 (d, J = 11.3 Hz, 2H), 3.87 - 3.82 (m, 2H), 3.05 (s, 6H), 2.92 (d, J = 2.4 Hz, 1H), 2.48 (q, J = 2.2 Hz, 2H).
LC-MS (ESI): $[M+H]^+$=312.28

**Example 22: Synthesis of N-((1R,5S,6s)-3-(3-amino-6-(2-hydroxyphenyl)pyridazin-4-yl)-3-azabicyclo[3.1.0]hexan-6-yl)acetamide**

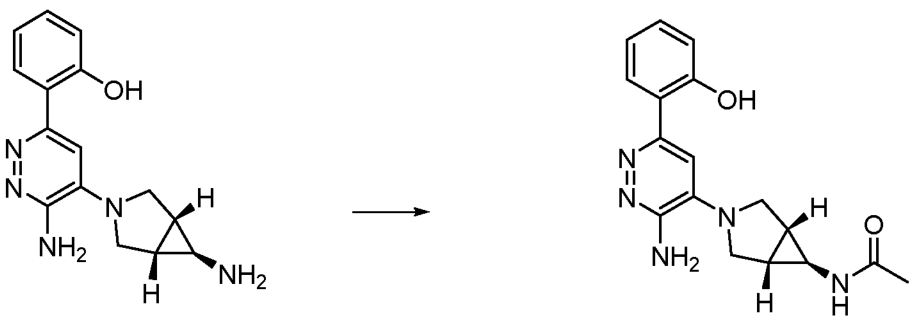

20b

21

20b (50 mg, 176.47μmol), HATU (87.23 mg, 229.41μmol, 1.3eq), AcOH (13.78 mg, 229.41 μmol, 1.3 eq) and DIEA (114.04 mg, 882.35 μmol, 5.0 eq) were dissolved in a reaction vial containing DCM (2 mL). The reaction was carried out at room temperature for 3h. After the reaction was completed, the final product 21 (5 mg, 8.71%) was purified by HPLC.

$^1$H NMR (600 MHz, Methanol-$d_4$) δ 7.71 (d, J = 7.3 Hz, 1H), 7.27 (t, J = 7.5 Hz, 1H), 7.17 (s, 1H), 6.93 (t, J = 7.8 Hz, 2H), 3.99 (d, J = 10.1 Hz, 2H), 3.39 (d, J = 9.7 Hz, 2H), 2.77 (s, 1H), 1.94 (s, 3H), 1.86 (s, 2H).
LC-MS (ESI): $[M+H]^+$=326.28

**Test Example 1: Fluorescence polarization detection**

1. Perform a fluorescence polarization assay on with the target protein, which has been expressed and purified from prokaryotic cells to a purity of >90%.

His-SMARCA2$^{BD}$(S1377-Q1486;Swiss Prot P51531-2):

mhhhhhhgslvpr\gsSPNPPKLTKQMNAIIDTVINYKDSSGRQLSEVFIQLPSRKELPEYYELIRKPVDFKKIKERIRNHKYRSLGDLEKDVMLLCHNAQTFNLEGSQIYEDSIVLQSVFKSARQ (SEQ ID NO:1)

His-SMARCA4$^{BD}$(A1448-S1575;Swiss Prot P51532):

mhhhhhhgslvpr\gsAEKLSPNPPNLTKKMKKIVDAVIKYKDSSSGRQLSEVFIQLPSRKELPEYYELIRKPVDFKKIKERIRNHKYRSLNDLEKDVMLLCQNAQTFNLEGSLIYEDSIVLQSVFTSVRQKIEKEDDSEGEES (SEQ ID NO:2)

2. The fluorescence polarization assay was conducted in a 384-well plate (3820, Corning) protected from light, with an excitation wavelength of 485 nm and an emission wavelength of 520 nm. Fluorescent probe was used in the experiment, and the buffer solution was 25 mM Bis-Tris, 0.01% Triton X-100, at pH 7.5. The final concentration of DMSO after the addition of the compound was less than 1%. The final detection volume per well was 125 μl, containing 50 μl of the fluorescent probe (final concentration 10 nM), 50 μl of SMARCA2$^{BD}$ protein (final concentration 50 nM) or 50 μl of SMARCA4$^{BD}$ protein (final concentration 120 nM), and 25 μl of the compound at various concentrations (final concentration ranging from 0 to 100 μM with a 3-fold dilution, a total of 11 concentrations). A control group (probe + protein), a probe control group (probe only), and a blank control group (buffer only) were set up. The fluorescence values were read by an ELISA reader r (SPARK m1000 pro, TECAN) after incubation for 2 h at room temperature on a shaker. The polarization value mP is calculated based on the fluorescence values in parallel and perpendicular directions of each well, and then the inhibition rate is calculated based on the mP value:

$$\text{Inhibition} = 1 - \frac{\text{mP(sample)} - \text{mP(tracer } group)}{\text{mP(control)} - \text{mP(tracer } group)} \times 100\%$$

The inhibition rate data was plotted in GraphPad Prism 8.0 software and the $IC_{50}$ value was calculated.

**Test Example 2: Cell proliferation assay**

The antiproliferative activity of the compounds on the cells was determined by CCK-8 kit (C0039, Biotronik). A549 (CCL-185™, ATCC) and MCF-7 (HTB-22™ ATCC) cells were incubated with F-12K medium (30-2004, ATCC) containing 10% FBS (10099141C, Gibco) and DMEM medium (11995065, Gibco) containing 10% FBS, respectively, at 37°C with 5% $CO_2$ until entering the logarithmic growth phase. In 96-well plates, 2800 cells (A549) or 1000 cells (MCF-7) was added in a volume of 100 μL, the blank control group was 100 uL of medium. After one day of incubation at 37°C, 5% $CO_2$, 100 μL of compounds of different concentrations were added (final concentration of 0-100 μM, 3-fold dilution, a total of 9 concentrations), and the control group was added to 100 μL of medium and continued to be incubation for 4 days (A549) or 7 days (MCF-7). According to the manufacturer's instructions, 20 μL of CCK-8 solution was added. Finally, the absorbance at 450 nm was measured using a microplate reader (SPARK m1000 pro, TECAN) after incubation at 37°C for 2 h (A549) or 4 h (MCF-7).

The inhibition rate of the compounds on the cells at different concentrations was calculated based on the OD values of each well, and the inhibition rate data were plotted in GraphPad Prism 8.0 software to calculate the $IC_{50}$ values.

$$\text{Inhibition} = [1 - \frac{\text{OD(sample)} - \text{OD(the blank group)}}{\text{OD(DMSO)} - \text{OD(the blank group)}}] \times 100\%$$

Compounds 1c, 2c, 3c, 4c, 5c, 6c, 10c prepared according to the above embodiments were used as experimental compounds. The control compounds were prepared with reference to Example 4 in PCT patent application WO2016138114. The structural formula of the control compounds is shown below:

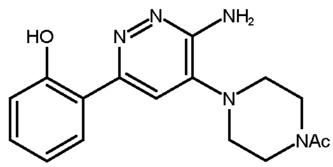

The experimental results are shown in Table 1:

**Table 1 Binding affinity (FP) and activity (CCK8) data of inhibitors**

| name | SMARCA2 FP $IC_{50}$ (μM) | SMARCA4 FP $IC_{50}$ (μM) | A549 cell line CCK8 $IC_{50}$(μM) | MCF-7 cell line CCK8 $IC_{50}$(μM) |
|---|---|---|---|---|
| Compound 1c | 0.19 | 0.30 | 25.38 | 12.02 |
| Compound 2c | 0.33 | 0.28 | 7.17 | 4.73 |
| Compound 3c | 0.48 | 0.57 | 73.28 | 30.98 |
| Compound 4c | 0.19 | 0.15 | 15.67 | 12.36 |
| Compound 5c | 0.13 | 0.13 | 15.38 | 7.65 |
| Compound 6c | 0.20 | 0.15 | 13.59 | 3.70 |
| Compound 10c | 0.19 | 0.14 | 14.63 | 7.03 |
| Control compound | 0.70 | 0.53 | >100 | 73.88 |

As shown in Table 1, the compounds provided by the present invention have significantly smaller $IC_{50}$ values for SMARCA2/4 relative to the control compound, demonstrating that the compounds provided by the present invention have better binding affinity for SMARCA2/4. Additionally, as can be seen from Table 1, the compounds provided by the present invention have significantly lower $IC_{50}$ values for A549 and MCF-7 cells than the control compound, proving that the compounds provided by the present invention have better inhibitory capabilities. At the same time, the $IC_{50}$ values of the compounds provided by the present invention against SMARCA2 and SMARCA4 were highly consistent, which means that the compounds provided by the present invention have good inhibitory activity against both SMARCA2 and SMARCA4, and can be used as dual inhibitors targeting the bromodomain of SMARCA2/4.

The antiproliferative activity of the compounds on the cells was determined by CCK-8 kit (C0039, Biotronik). Hela (CCL-2™, ATCC) and SW1573 (CRL-2170™, ATCC) cells were incubated with DMEM medium (11995065, Gibco) containing 10% FBS (10099141C, Gibco) at 37°C with 5% $CO_2$ until entering the logarithmic growth phase. In 96-well plates, 2500 cells (Hela) or 800 cells (SW1573) were added in a volume of 100 μL, the blank control group was 100 μL of medium, and after one day of incubation at 37°C, 5% $CO_2$, 100 μL of compounds of different concentrations were added (final concentration of 0-100 μM, 3-fold dilution, a total of 9 concentrations), and the control group was added to 100 μL of medium and continued to incubate for 3 days (Hela) or 7 days (SW1573). Then 20 μL of CCK-8 solution were added. Finally, the absorbance at 450 nm was measured using a microplate reader (SPARK m1000 pro, TECAN) after incubation at 37°C for 1.5 h (Hela) or 2.5 h (SW1573).

The inhibition rate of the compounds on the cells at different concentrations was calculated based on the OD values of each well, and the inhibition rate data were plotted in GraphPad Prism 8.0 software to calculate the $IC_{50}$ values.

$$\text{Inhibition} = [1 - \frac{\text{OD(sample)} - \text{OD(the blank group)}}{\text{OD(DMSO)} - \text{OD(the blank group)}}] \times 100\%$$

Compounds 1c, 2c, 3c, 4c, 5c, 6c, 10c prepared according to the above Examples were used as experimental compounds. The control compounds were prepared with reference to Example 4 in PCT patent application WO2016138114, and the structural formula of the control compound is shown below:

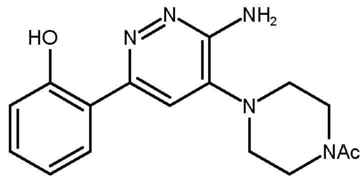

The experimental results are shown in Table 2:

**Table 2 Binding affinity (FP) and activity (CCK8) data of inhibitors**

| name | SMARCA2 FP $IC_{50}$ (μM) | SMARCA4 FP $IC_{50}$ (μM) | Hela cell line CCK8 $IC_{50}$(μM) | SW1573 cell line CCK8 $IC_{50}$(μM) |
|---|---|---|---|---|
| Compound 15 | 0.19 | 0.21 | 25.27 | 13.10 |
| Compound 17 | 1.1 | >3.7 | 66.26 | 99.00 |
| Compound 20 | 0.17 | 0.12 | 38.95 | 91.16 |
| Compound 21 | 0.13 | 0.25 | 68.41 | >100 |
| Control compound | 0.70 | 0.53 | >100 | >100 |

As shown in Table 2, the compounds provided by the present invention have significantly smaller $IC_{50}$ values for SMARCA2/4 relative to the control compound, demonstrating that the compounds provided by the present invention have better binding affinity for SMARCA2/4. Additionally, as can be seen from Table 2, the compounds provided by the present invention have significantly lower $IC_{50}$ values for Hela and SW1573 cells than the control compound, proving that the compounds provided by the present invention have better inhibitory capabilities. At the same time, the $IC_{50}$ values of the compounds provided by the present invention against SMARCA2 and SMARCA4 were highly consistent, which means that the compounds provided by the present invention have good inhibitory activity against both SMARCA2 and SMARCA4, and can be used as dual inhibitors targeting the bromodomain of SMARCA2/4.

The present invention has been illustrated through the aforementioned examples, but it should be understood that these examples are for illustrative and explanatory purposes only and are not intended to limit the scope of the invention to the specific examples described. Furthermore, those skilled in the art will appreciate that the invention is not limited to the above examples, and that various modifications and variations can be made in light of the teachings of the invention, all of which fall within the scope of the protection sought by the invention. The scope of protection of the invention is defined by the claims and their equivalents.

## Claims

**1.** A compound having a structure shown by formula $I_A$ or a pharmaceutically acceptable salt thereof,

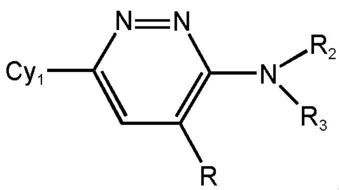

;

$I_A$

wherein,

$Cy_1$ is saturated or unsaturated cycloalkyl, saturated or unsaturated heteroalkyl containing one or more heteroatoms selected from N, O, and S, aryl, or heteroaryl containing one or more heteroatoms selected from N, O, and S, which is unsubstituted or substituted by one or more substituents each independently selected from halo, cyano, haloalkyl, haloalkoxy, alkyl, hydroxy, amino, and alkyl amino; preferably, $Cy_1$ is saturated or unsaturated 3- to 10-membered cycloalkyl, saturated or unsaturated 3- to 10-membered heteroalkyl containing 1 to 3 heteroatoms each independently selected from N, O, and S, 6- to 10-membered aryl, or 5- to 10-membered

heteroaryl containing 1 to 3 heteroatoms each independently selected from N, O, and S, which is unsubstituted or substituted by one or more substituents each independently selected from halo, cyano, $C_1$-$C_6$ haloalkyl, $C_1$-$C_6$ haloalkoxy, $C_1$-$C_6$ alkyl, hydroxy, amino, and $C_1$-$C_6$ alkyl amino;

Ris

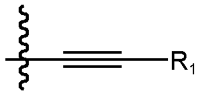

or

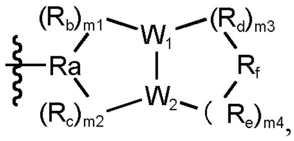

and the wavy line denotes the attachment point;

$R_a$ is $CR_{aa}$ or N;

$R_f$ is selected from $C(R_{aa})_2$, CO, O, S, and $NR_{aa}$;

$W_1$ and $W_2$ are each independently $CR_{aa}$ or N;

$R_d$, $R_c$, $R_d$, and $R_e$ are each independently, at each occurrence, selected from $C(R_{aa})_2$, CO, O, S, and $NR_{aa}$;

$R_{aa}$ is each independently, at each occurrence, selected from H, deuterium, halo, alkyl, deuterated alkyl, heteroalkyl, alkenyl, alkynyl, alkoxy, hydroxy, cycloalkyl, heterocyclyl, aryl, heteroaryl, - $N(R_g)_2$, cyano, -C(O)-$N(R_g)_2$, -S(O)-$N(R_g)_2$, -$S(O)_2$-$N(R_g)_2$, -O-$R_g$, -S-$R_g$, -O-C(O)-$R_g$, -C(O)-$R_g$, - C(O)-$OR_g$, -S(O)-$R_g$, -$S(O)_2$-$R_g$, -$N(R_g)$-C(O)-$R_g$, -$N(R_g)$-S(O)-$R_g$, -$N(R_g)$-C(O)-$N(R_g)_2$, and -$N(R_g)$-$S(O)_2$-$R_g$, and nitro, wherein optionally, said alkyl, heteroalkyl, alkenyl, alkynyl, alkoxy, cycloalkyl, heterocyclyl, aryl, and heteroaryl are each independently substituted with one or more substituents selected from halo, oxo (=O), alkyl, alkenyl, alkynyl, alkoxy, haloalkyl, haloalkoxy, hydroxyalkyl, cycloalkyl, heterocyclyl, aryl, heteroaryl, -$N(R_g)_2$, cyano, -C(O)-$N(R_g)_2$, -S(O)-$N(R_g)_2$, -$S(O)_2$-$N(R_g)_2$, -O-$R_g$, -S-$R_g$, -O-C(O)-$R_g$, -C(O)-$R_g$, -C(O)-$OR_g$, -S(O)-$R_g$, -$S(O)_2$-$R_g$, -$N(R_g)$-C(O)-$R_g$, -$N(R_g)$-S(O)-$R_g$, -$N(R_g)$-C(O)-$N(R_g)_2$, -$N(R_g)$-$S(O)_2$-$R_g$, and nitro; preferably, $R_{aa}$ is, at each occurrence, each independently selected from H, deuterium, halo, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxy, $C_1$-$C_6$ hydroxyalkyl, -C(O)-$C_1$-$C_6$ alkyl, nitro, cyano, $C_3$-$C_{10}$ cycloalkyl, $C_3$-$C_{10}$ heterocyclyl, $C_6$-$C_{10}$ aryl, $C_5$-$C_{10}$ heteroaryl, - $NHR_g$, benzyloxycarbonyl (Cbz), carboxyl (-COOH), -$N(R_g)_2$, -NH(CO)$R_g$, acetyloxy (AcO), and-$N(R_g)_2$, wherein optionally, said $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_{10}$ cycloalkyl, $C_3$-$C_{10}$ heterocyclyl, $C_6$-$C_{10}$ aryl, and $C_5$-$C_{10}$ heteroaryl are each independently substituted with one or more substituents selected from halo, $C_1$-$C_6$ alkyl, $C_1$-$C_6$ heteroalkyl, $C_1$-$C_6$ alkoxy, $C_1$-$C_6$ haloalkyl, hydroxy, $C_1$-$C_6$ hydroxyalkyl, cyano, nitro, $C_3$-$C_{10}$ cycloalkyl, $C_3$-$C_{10}$ heterocyclyl, $C_6$-$C_{10}$ aryl, $C_5$-$C_{10}$ heteroaryl, carboxyl (-COOH), -NH(CO)$R_g$, acetyloxy (AcO), and -$N(R_g)_2$; preferably, $R_{aa}$ is, at each occurrence, independently selected from H, deuterium, halo, hydroxy, $C_1$-$C_6$ alkyl, $C_1$-$C_6$ hydroxyalkyl, $C_1$-$C_6$ alkoxy, $C_1$-$C_6$ haloalkyl, $C_1$-$C_6$ haloalkoxy, cyano, nitro, benzyloxycarbonyl (Cbz), carboxyl (-COOH), NH(CO)$R_g$, acetyloxy (AcO), and -$N(R_g)_2$, wherein optionally, said $C_1$-$C_6$ alkyl and $C_1$-$C_6$ alkoxy are each independently substituted with one or more substituents selected from halo, $C_1$-$C_6$ alkyl, $C_1$-$C_6$ heteroalkyl, $C_1$-$C_6$ alkoxy, $C_1$-$C_6$ haloalkyl, hydroxy, $C_1$-$C_6$ hydroxyalkyl, cyano, nitro, $C_3$-$C_8$ cycloalkyl, $C_3$-$C_{10}$ heterocyclyl, $C_6$-$C_{10}$ aryl, $C_5$-$C_{10}$ heteroaryl, carboxyl (-COOH), -NH(CO)$R_g$, acetyloxy (AcO), and -$N(R_g)_2$;

m1, m2, m3, and m4 are each independently, at each occurrence, selected from 0, 1, 2, 3, 4, and 5;

$R_1$ is selected from H, deuterium, halo, alkyl, deuterated alkyl, heteroalkyl, alkenyl, alkynyl, alkoxy, hydroxy, cycloalkyl, heterocyclyl, aryl, heteroaryl, -$N(R_g)_2$, cyano, -C(O)-$N(R_g)_2$, -S(O)-$N(R_g)_2$, -$S(O)_2$-$N(R_g)_2$, -O-$R_g$, -S-$R_g$, -O-C(O)-$R_g$, -C(O)-$R_g$, -C(O)-$OR_g$, -S(O)-$R_g$, -$S(O)_2$-$R_g$, - $N(R_g)$-C(O)-$R_g$, -$N(R_g)$-S(O)-$R_g$, -$N(R_g)$-C(O)-$N(R_g)_2$, -$N(R_g)$-$S(O)_2$-$R_g$, and nitro, wherein optionally, said alkyl, heteroalkyl, alkenyl, alkynyl, alkoxy, cycloalkyl, heterocyclyl, aryl, and heteroaryl are each independently substituted with one or more substituentsselected from halo, oxo (=O), alkyl, alkenyl, alkynyl, alkoxy, haloalkyl, haloalkoxy, hydroxyalkyl, cycloalkyl, heterocycloalkyl, aryl, heteroaryl, -$N(R_g)_2$, cyano, -C(O)-$N(R_g)_2$, -S(O)-$N(R_g)_2$, -$S(O)_2$-$N(R_g)_2$, -$OR_g$, -S-$R_g$, -O-C(O)-$R_g$, -C(O)-$R_g$, -C(O)-$OR_g$, -S(O)-$R_g$, -$S(O)_2$-$R_g$, -$N(R_g)$-C(O)-$R_g$, -$N(R_g)$-S(O)-$R_g$, -$N(R_g)$-C(O)-$N(R_g)_2$, -$N(R_g)$-$S(O)_2$-$R_g$, and nitro; preferably, $R_1$ is selected from H, deuterium, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, 3- to 15-membered cycloalkyl, 3- to 15-membered heterocycloalkyl, 6- to 14-membered aryl, and 5-to 14-membered heteroaryl,

wherein said 3- to 15-membered cycloalkyl and 3- to 15-membered heterocycloalkyl are each independently monocyclic, fused-cyclic, bridged-cyclic, or spiro-cyclic group, said 6- to 14-membered aryl and 5- to 14-membered heteroaryl are each independently monocyclic or fused-cyclic group, wherein optionally, said $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, 3- to 15-membered cycloalkyl, 3- to 15-membered heterocycloalkyl, 6- to 14-membered aryl, and 5- to 14-membered heteroaryl are each independently substituted with one, two, or more substituents selected from halo, oxo (=O), $C_1$-$C_6$ alkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, $C_1$-$C_6$ hydroxyalkyl, 3- to 15-membered cycloalkyl, 3- to 15-membered heterocycloalkyl, 6- to 14-membered aryl, 5- to 14-membered heteroaryl, -N($R_g$)$_2$, cyano, -C(O)-N($R_g$)$_2$, -S(O)-N($R_g$)$_2$, -S(O)$_2$-N($R_g$)$_2$, -O-$R_g$, -S-$R_g$, -O-C(O)-$R_g$, -C(O)-$R_g$, -C(O)-O$R_g$, -S(O)-$R_g$, -S(O)$_2$-$R_g$, -N($R_g$)-C(O)-$R_g$, - N($R_g$)-S(O)-$R_g$, -N($R_g$)-C(O)-N($R_g$)$_2$, -N($R_g$)-S(O)$_2$-$R_g$, and nitro;

$R_g$ is, at each occurrence, each independently selected from H, deuterium, halo, alkyl, deuterated alkyl, heteroalkyl, alkenyl, alkynyl, alkoxy, haloalkyl, haloalkoxy, hydroxy, hydroxyalkyl, -C(O)-alkyl, cycloalkyl, heterocyclyl, aryl, heteroaryl, benzyloxycarbonyl (Cbz), wherein optionally, said alkyl, heteroalkyl, alkenyl, alkynyl, alkoxy, cycloalkyl, heterocyclyl, aryl, and heteroaryl are each independently substituted with one or more substituents selected from halo, alkyl, heteroalkyl, alkoxy, haloalkyl, hydroxy, hydroxyalkyl, cyano, amino, nitro, cycloalkyl, heterocyclyl, aryl, heteroaryl, carboxyl (-COOH), alkyl amino, -NH(CO)H, -NH(CO)-alkyl, and acetyloxy (AcO);

preferably, $R_g$ is each independently, at each occurrence, selected from H, deuterium, $C_1$-$C_6$ alkyl, $C_1$-$C_6$ heteroalkyl, $C_1$-$C_6$ alkoxy, $C_2$-$C_6$ alkenyl, $C_2$-$C_6$ alkynyl, $C_1$-$C_6$ haloalkyl, $C_1$-$C_6$ haloalkoxy, $C_1$-$C_6$ hydroxyalkyl, -C(O)-$C_1$-$C_6$ alkyl, $C_6$-$C_{10}$ aryl, $C_5$-$C_{10}$ heteroaryl, $C_3$-$C_{15}$ cycloalkyl, and $C_3$-$C_{15}$ heterocycloalkyl, wherein optionally, said $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_{15}$ cycloalkyl, $C_3$-$C_{15}$ heterocycloalkyl, $C_6$-$C_{10}$ aryl, and $C_5$-$C_{10}$ heteroaryl are each independently substituted with one or more substituents selected from halo, $C_1$-$C_6$ alkyl, $C_1$-$C_6$ heteroalkyl, $C_1$-$C_6$ alkoxy, $C_1$-$C_6$ haloalkyl, hydroxy, $C_1$-$C_6$ hydroxyalkyl, cyano, amino, nitro, $C_3$-$C_{15}$ cycloalkyl, $C_3$-$C_{15}$ heterocycloalkyl, $C_6$-$C_{10}$ aryl, $C_5$-$C_{10}$ heteroaryl, carboxyl (-COOH), $C_1$-$C_6$ alkyl amino, -NH(CO)H, -NH(CO)-$C_1$-$C_6$ alkyl, and acetyloxy (AcO); and

$R_2$ and $R_3$ are each independently selected from H, alkyl, deuterium, F, Cl, Br, I, hydroxy, haloalkyl, hydroxyalkyl, alkoxy, and -C(=O)-alkyl; preferably, $R_2$ and $R_3$ are each independently selected from H, $C_1$-$C_6$ alkyl, deuterium, $C_1$-$C_6$ haloalkyl, $C_1$-$C_6$ hydroxyalkyl, $C_1$-$C_6$ alkoxy, and - C(=O)-$C_1$-$C_6$ alkyl.

**2.** The compound of claim 1, **characterized in that** $Cy_1$ is aryl or heteroaryl, said aryl and heteroaryl are substituted with hydroxy, amino, or alkyl amino, and optionally each independently being substituted with one or more substituents selected from halo, cyano, $C_1$-$C_6$ haloalkyl, $C_1$-$C_6$ haloalkoxy, and $C_1$-$C_6$ alkyl; preferably, $Cy_1$ is 6- to 10-membered aryl or 5- to 10-membered heteroaryl containing 1-3 heteroatoms each independently selected from N, O, and S, said 6- to 10-membered aryl and 5- to 10-membered heteroaryl are substituted with hydroxy, amino, or alkyl amino, and optionally each independently being further substituted with one or more substituents selected from halo, cyano, $C_1$-$C_6$ haloalkyl, $C_1$-$C_6$ haloalkoxy, and $C_1$-$C_6$ alkyl; preferably, $Cy_1$ is phenyl or 5-to 6-membered heteroaryl containing 1-3 heteroatoms each independently selected from N, O, and S, said phenyl and 5- to 6-membered heteroaryl are substituted with hydroxy, amino, or alkyl amino, and optionally each independently being substituted with one or more substituents selected from halo, cyano, $C_1$-$C_6$ haloalkyl, $C_1$-$C_6$ haloalkoxy, and $C_1$-$C_6$ alkyl; preferably, $Cy_1$ is phenyl or 5- to 6-membered heteroaryl containing 1-3 heteroatoms each independently selected from N, O, and S, said phenyl and 5- to 6-membered heteroaryl are substituted with hydroxy, amino, or alkyl amino, and optionally each independently being further substituted with one or more substituents selected from F, Cl, Br, cyano, trifluoromethyl, trifluoromethoxy, $C_1$-$C_3$ alkyl, and $C_1$-$C_3$ alkoxy; preferably, $Cy_1$ is phenyl or 5- to 6-membered heteroaryl containing 1-3 heteroatoms independently selected from N, O, and S, said phenyl and 5- to 6-membered heteroaryl are substituted with at least one hydroxy, amino, or alkyl amino, preferably with one hydroxy, amino, or alkyl amino; and/or

$R_1$ is selected from H, deuterium, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, 3- to 15-membered cycloalkyl, 3- to 15-membered heterocycloalkyl, 6- to 14-membered aryl, and 5- to 14-membered heteroaryl, wherein said 3- to 15-membered cycloalkyl and 3- to 15-membered heterocycloalkyl are each independently monocyclic, fused-cyclic, bridged-cyclic, or spiro-cyclic group, said 6- to 14-membered aryl and 5- to 14-membered heteroaryl are each independently monocyclic or fused-cyclic group, and said 3- to 15-membered heterocycloalkyl and 5- to 14-membered heteroaryl are each independently containing 1, 2, 3, 4, or 5 heteroatoms independently selected from N, O, and S, optionally, said $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, 3- to 15-membered cycloalkyl, 3- to 15-membered heterocycloalkyl, 6- to 14-membered aryl, and 5- to 14-membered heteroaryl are each independently substituted with one, two, or more substituents independently selected from F, Cl, Br, I, oxo

(=O), hydroxy, $C_1$-$C_6$ alkyl, $C_1$-$C_6$ hydroxyalkyl, $C_1$-$C_6$ alkoxy, $C_1$-$C_6$ haloalkyl, $C_1$-$C_6$ haloalkoxy, amino, $C_1$-$C_6$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_6$ alkyl, 5- to 8-membered heterocycloalkyl containing 1, 2, or 3 heteroatoms independently selected from N, O, and S, and 9-fluorenylmethoxycarbonyl (Fmoc); and/or

$R_{aa}$ is each independently, at each occurrence, selected from H, deuterium, halo, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_6$ haloalkoxy, cyano, nitro, benzyloxycarbonyl (Cbz), carboxyl (-COOH), NH(CO)$R_g$, acetyloxy (AcO), and -N($R_g$)$_2$, wherein optionally, said $C_1$-$C_3$ alkyl and $C_1$-$C_3$ alkoxy are each independently substituted with one or more substituents selected from halo, $C_1$-$C_3$ alkyl, $C_1$-$C_3$ heteroalkyl, $C_1$-$C_3$ alkoxy, $C_1$-$C_3$ haloalkyl, hydroxy, $C_1$-$C_3$ hydroxyalkyl, cyano, nitro, $C_3$-$C_6$ cycloalkyl, $C_3$-$C_6$ heterocycloalkyl, phenyl, $C_5$-$C_6$ heteroaryl, carboxyl (-COOH), -N(CO)$R_g$, acetyloxy (AcO), and -N($R_g$)$_2$; and/or

$R_g$ is each independently, at each occurrence, selected from H, deuterium, $C_1$-$C_3$ alkyl, $C_1$-$C_3$ heteroalkyl, $C_1$-$C_3$ alkoxy, $C_2$-$C_3$ alkenyl, $C_2$-$C_3$ alkynyl, $C_1$-$C_3$ haloalkyl, $C_1$-$C_3$ haloalkoxy, $C_1$-$C_3$ hydroxyalkyl, -C(O)-$C_1$-$C_3$ alkyl, phenyl, $C_5$-$C_6$ heteroaryl, $C_3$-$C_6$ cycloalkyl, and $C_3$-$C_6$ heterocycloalkyl, wherein optionally, said $C_1$-$C_3$ alkyl, $C_1$-$C_3$ heteroalkyl, $C_2$-$C_3$ alkenyl, $C_2$-$C_3$ alkynyl, $C_1$-$C_3$ alkoxy, $C_3$-$C_6$ cycloalkyl, $C_3$-$C_6$heterocyclokyl, aryl, and $C_5$-$C_{10}$ heteroaryl are each independently substituted with one or more substituents selected from halo, $C_1$-$C_3$ alkyl, $C_1$-$C_3$ heteroalkyl, $C_1$-$C_3$ alkoxy, $C_1$-$C_3$ haloalkyl, hydroxy, $C_1$-$C_3$ hydroxyalkyl, cyano, amino, nitro, $C_3$-$C_6$ cycloalkyl, $C_3$-$C_6$ heterocyclokyl, $C_6$-$C_{10}$ aryl, $C_5$-$C_{10}$ heteroaryl, carboxyl (-COOH), $C_1$-$C_3$ alkyl amino, -NH(CO)H, -NH(CO)-$C_1$-$C_3$ alkyl, and acetyloxy (AcO); and/or

$R_2$ and $R_3$ are each independently selected from H, $C_1$-$C_3$ alkyl, deuterium, $C_1$-$C_3$ haloalkyl, $C_1$-$C_3$ hydroxyalkyl, $C_1$-$C_3$ alkoxy, and -C(=O)-$C_1$-$C_3$ alkyl.

**3.** The compound of claim 1, **characterized in that** said compound has a structure shown by formula I,

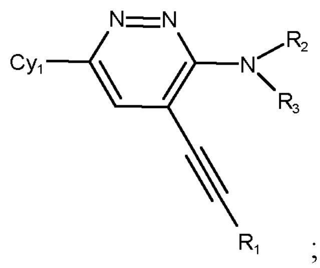

;

I

wherein, $Cy_1$ is aryl or heteroaryl, said aryl and heteroaryl are substituted with at least one hydroxy, amino, or alkyl amino, and optionally being substituted with one or more substituents independently selected from halo, cyano, haloalkyl, haloalkoxy, alkyl, and alkoxy; preferably, $Cy_1$ is 6- to 10-membered aryl or 5- to 10-membered heteroaryl containing 1-3 heteroatoms independently selected from N, O, and S, said 6- to 10-membered aryl or 5- to 10-membered heteroaryl are substituted with at least one hydroxy, amino, or alkyl amino, and optionally being substituted with one or more substituents independently selected from halo, cyano, $C_1$-$C_6$ haloalkyl, $C_1$-$C_6$ haloalkoxy, $C_1$-$C_6$ alkyl, and $C_1$-$C_6$ alkoxy;

$R_1$ is selected from H, $C_1$-$C_6$ alkyl, $C_2$-$C_6$ alkenyl, $C_2$-$C_6$ alkynyl, $C_1$-$C_6$ haloalkyl, $C_1$-$C_6$ haloalkoxy, and $C_1$-$C_6$ hydroxyalkyl; or $R_1$ isunsubstituted or substituted 3- to 15-membered cycloalkyl, or $R_1$ is 3- to 15-membered heterocycloalkyl containing 1-3 heteroatoms independently selected from N, O, and S, said 3- to 15-membered cycloalkyl and 3- to 15-membered heterocycloalkyl are each independently monocyclic, fused-cyclic, bridged-cyclic, or spiro-cyclic group; or $R_1$ is unsubstituted or substituted phenyl or unsubstituted or substituted 8- to 10-membered aryl, said 8- to 10-membered aryl is a monocyclic or fused-cyclic group; or $R_1$ is unsubstituted or substituted 5- to 6-membered heteroaryl containing 1-3 heteroatoms independently selected from N, O, and S; or $R_1$ is unsubstituted or substituted 8- to 10-membered heteroaryl containing 1-3 heteroatoms independently selected from N, O, and S, said 8- to 10-membered heteroaryl is a monocyclic or fused-cyclic group; wherein said substituted 3- to 15-membered cycloalkyl, 3- to 15-membered heterocycloalkyl, phenyl, 8- to 10-membered aryl, 5- to 6-membered heteroaryl, and 8- to 10-membered heteroaryl are each independently substituted with one, two, or more substituents independently selected from halo, oxo (=O), $C_1$-$C_6$ alkyl, $C_2$-$C_6$ alkenyl, $C_2$-$C_6$ alkynyl, $C_1$-$C_6$ haloalkyl, $C_1$-$C_6$ haloalkoxy, $C_1$-$C_6$ hydroxyalkyl, 3- to 15-membered cycloalkyl, 3- to 15-membered heterocycloalkyl, -N($R_g$)$_2$, cyano, -C(O)-N($R_g$)$_2$, -S(O)-N($R_g$)$_2$, -S(O)$_2$-N($R_g$)$_2$, -O-$R_g$, -S-$R_g$, -O-C(O)-$R_g$, -C(O)-$R_g$, -C(O)-O$R_g$, -S(O)-$R_g$, -S(O)$_2$-$R_g$, -N($R_g$)-C(O)-$R_g$, -N($R_g$)-S(O)-$R_g$, -N($R_g$)-C(O)-N($R_g$)$_2$, -N($R_g$)-S(O)$_2$-$R_g$, and nitro; preferably, said substituted 3- to 15-membered cycloalkyl, 3- to 15-membered heterocycloalkyl, phenyl, 8- to 10-membered aryl, 5- to 6-membered heteroaryl, and 8- to 10-membered

heteroaryl are each independently substituted with one, two, or more substituents independently selected from halo, oxo (=O), hydroxy, $C_1$-$C_6$ alkyl, $C_1$-$C_6$ hydroxyalkyl, $C_1$-$C_6$ alkoxy, $C_1$-$C_6$ haloalkyl, $C_1$-$C_6$ haloalkoxy, amino, $C_1$-$C_6$ alkyl amino, cyano, nitro, benzyloxycarbonyl (Cbz), carboxyl (-COOH), -NH(CO)-$C_1$-$C_6$ alkyl, acetyloxy (AcO), benzyl (Bn), tert-butoxycarbonyl (Boc), -(CO)H, 5- to 8-membered heterocycloalkyl containing one nitrogen atom, and 9-fluorenylmethoxycarbonyl (Fmoc);

$R_g$ is each independently, at each occurrence, selected from H, $C_1$-$C_6$ alkyl, $C_2$-$C_6$ alkenyl, $C_2$-$C_6$ alkynyl, $C_1$-$C_6$ haloalkyl, $C_1$-$C_6$ haloalkoxy, $C_1$-$C_6$ hydroxyalkyl, 3- to 15-membered cycloalkyl, and 3- to 15-membered heterocycloalkyl; preferably, $R_g$ is each independently, at each occurrence, selected from H, $C_1$-$C_3$ alkyl, $C_2$-$C_3$ alkenyl, $C_2$-$C_3$ alkynyl, $C_1$-$C_3$ haloalkyl, $C_1$-$C_3$ haloalkoxy, $C_1$-$C_3$ hydroxyalkyl, $C_3$-$C_8$ cycloalkyl, and $C_3$-$C_8$ heterocycloalkyl; and

$R_2$ and $R_3$ are each independently selected from H, alkyl, and -C(=O)-alkyl.

**4.** The compound of any one of claims 1 to 3 or a pharmaceutically acceptable salt thereof, wherein, $Cy_1$ is phenyl or 5- to 6-membered heteroaryl, said phenyl and 5- to 6-membered heteroaryl are substituted with at least one hydroxy, amino, or alkyl amino, and optionally being substituted with one or more substituents independently selected from halo, cyano, haloalkyl, haloalkoxy, alkyl, and alkoxy; preferably, $Cy_1$ is phenyl or 5- to 6-membered heteroaryl said phenyl or heteroaryl are substituted with at least one hydroxy, and optionally being substituted with one or more substituents independently selected from halo, cyano, trifluoromethyl, trifluoromethoxy, $C_1$-$C_6$ alkyl, and $C_1$-$C_6$ alkoxy, preferably optionally being substituted with one or more substituents independently selected from F, Cl, Br, cyano, trifluoromethyl, trifluoromethoxy, $C_1$-$C_3$ alkyl, and $C_1$-$C_3$ alkoxy; preferably, $Cy_1$ is phenyl, and said phenyl is substituted with one hydroxy; and/or

$R_1$ is selected from H, $C_1$-$C_4$ alkyl, $C_2$-$C_4$ alkenyl, $C_2$-$C_4$ alkynyl, $C_1$-$C_4$ haloalkyl, $C_1$-$C_4$ haloalkoxy, and $C_1$-$C_4$ hydroxyalkyl; or $R_1$ is unsubstituted or substituted 3- to 15-membered cycloalkyl, or $R_1$ is 3- to 15-membered heterocycloalkyl containing 1-2 heteroatoms independently selected from N, O, and S, said 3- to 15-membered cycloalkyl and 3- to 15-membered heterocycloalkyl are each independently monocyclic, fused-cyclic, or spiro-cyclic group; or $R_1$ is unsubstituted or substituted phenyl or an unsubstituted or substituted 10-membered aryl, said 10-membered aryl is a fused-cyclic group; or $R_1$ is substituted or unsubstituted 5- to 6-membered heteroaryl containing 1-2 heteroatoms independently selected from N, O, and S, or $R_1$ is substituted or unsubstituted 8- to 10-membered heteroaryl containing 1-3 heteroatoms independently selected from N, O, and S, said 8- to 10-membered heteroaryl is a fused-cyclic group; wherein, said substituted 3- to 15-membered cycloalkyl, 3- to 15-membered heterocycloalkyl, phenyl, 5- to 6-membered heteroaryl, 10-membered aryl, and 8- to 10-membered heteroaryl are each independently substituted with one, two, or more substituents independently selected from halo, oxo (=O), carboxyl, benzyloxycarbonyl (Cbz), acetyloxy (AcO), benzyl (Bn), tert-butoxycarbonyl (Boc), 5- to 8-membered heterocycloalkyl containing one nitrogen atom, 9-fluorenylmethoxycarbonyl (Fmoc), hydroxy, $C_1$-$C_6$ alkyl, $C_1$-$C_6$ hydroxyalkyl, $C_1$-$C_6$ alkoxy, $C_1$-$C_6$ haloalkyl, $C_1$-$C_6$ haloalkoxy, amino, $C_1$-$C_6$ alkyl amino, cyano, -(CO)H, -NH(CO)- $C_1$-$C_6$ alkyl, and nitro; preferably, said substituted 3-to 15-membered cycloalkyl, 3- to 15-membered heterocycloalkyl, phenyl, 5- to 6-membered heteroaryl, 10-membered aryl, and 8- to 10-membered heteroaryl are each independently substituted with one, two, or more substituents independently selected from Cl, Br, F, I, oxo, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_6$ alkyl, 5- to 6-membered heterocycloalkyl containing one nitrogen atom, and 9-fluorenylmethoxycarbonyl (Fmoc); preferably, said substituted 3- to 15-membered cycloalkyl, 3- to 15-membered heterocycloalkyl, phenyl, 5- to 6-membered heteroaryl, 10-membered aryl, and 8- to 10-membered heteroaryl are each independently substituted with one, two, or more substituents independently selected from Cl, Br, F, I, oxo, hydroxy, methyl, hydroxymethyl, methoxy, amino, dimethylamino, cyano, nitro, carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc); and/or

$R_2$ and $R_3$ are each independently H or $C_1$-$C_6$ alkyl; preferably, $R_2$ and $R_3$ are each independently H or $C_1$-$C_3$ alkyl; preferably, $R_2$ and $R_3$ are each independently H.

**5.** The compound of any one of claims 1 to 4 or a pharmaceutically acceptable salt thereof, wherein the compound has a structure shown in formula II,

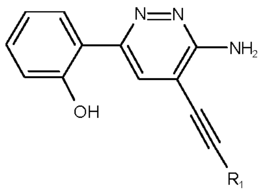

;

II

wherein,

$R_1$ is as defined in any one of claims 1, 2, 3, or 4;

preferably, $R_1$ is unsubstituted or substituted 4-, 5-, 6-, 7-, 8-, 9-, 10-, or 11-membered cycloalkyl, or $R_1$ is 4-, 5-, 6-, 7-, 8-, 9-, 10-, or 11-membered heterocycloalkyl containing 1-2 heteroatoms independently selected from N, O, and S, said cycloalkyl and heterocycloalkyl are each independently monocyclic, fused-cyclic, bridged-cyclic, or spiro-cyclic group; or $R_1$ is unsubstituted or substituted phenyl, or substituted or unsubstituted 10-membered aryl, said 10-membered aryl is a fused-cyclic group; or $R_1$ is substituted or unsubstituted 5- to 6-membered heteroaryl containing 1-2 heteroatoms independently selected from N, O, and S, or $R_1$ is substituted or unsubstituted 8- to 10-membered heteroaryl containing 1-2 heteroatoms independently selected from N, O, and S, said 8- to 10-membered heteroaryl is a fused-cyclic group; wherein, said substituted cycloalkyl, heterocycloalkyl, phenyl, 5- to 6-membered heteroaryl, 10-membered aryl, and 8- to 10-membered heteroaryl are each independently substituted with one, two, or more substituents independently selected from Cl, Br, F, I, oxo, carboxyl, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino group, $C_1$-$C_3$ alkyl amino, cyano, nitro, benzyloxycarbonyl (Cbz), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc), preferably, each independently substituted with one, two, or more substituents independently selected from Cl, Br, F, I, oxo, hydroxyl, methyl, hydroxymethyl, methoxy, amino, dimethylamino, cyano, nitro, carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc);

preferably, $R_1$ is unsubstituted or substituted 4-membered monocycloalkyl, 5-membered monocycloalkyl, 6-membered monocycloalkyl, 7-membered spiro-cycloalkyl, 8-membered spiro-cycloalkyl, 9-membered spiro-cycloalkyl, 10-membered spiro-cycloalkyl, 11-membered spiro-cycloalkyl, 8-membered fused-cycloalkyl, 9-membered fused-cycloalkyl, or 10-membered fused-cycloalkyl; or $R_1$ is 4-membered monocyclic heterocycloalkyl, 5-membered monocyclic heterocycloalkyl, 6-membered monocyclic heterocycloalkyl, 7-membered spiro-cyclic heterocycloalkyl, 8-membered spiro-cyclic heterocycloalkyl, 9-membered spiro-cyclic heterocycloalkyl, 10-membered spiro-cyclic heterocycloalkyl, 11-membered spiro-cyclic heterocycloalkyl, 8-membered fused-cyclic heterocycloalkyl, 9-membered fused-cyclic heterocycloalkyl, or 10-membered fused-cyclic heterocycloalkyl, containing 1-2 heteroatoms independently selected from N, O, and S; or said substituted cycloalkyl and heterocycloalkyl are each independently substituted with one, two, or more substituents independently selected from Cl, Br, F, I, oxo, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc), preferably, each independently substituted with one, two, or more substituents independently selected from Cl, Br, F, I, oxo, hydroxy, methyl, hydroxymethyl, methoxy, amino, dimethylamino, cyano, nitro, carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc).

6. The compound of any one of claims 1 to 4 or a pharmaceutically acceptable salt thereof, **characterized in that** $R_1$ is H, -$CH_3$, or -$C_4H_9$;
or $R_1$ is selected from the following groups optionally substituted with one or more substituents:

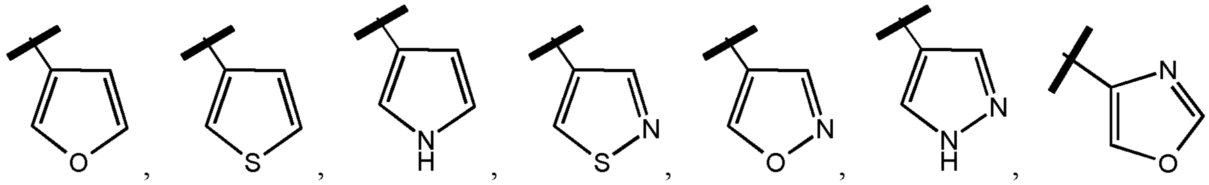

, , , , , , ,

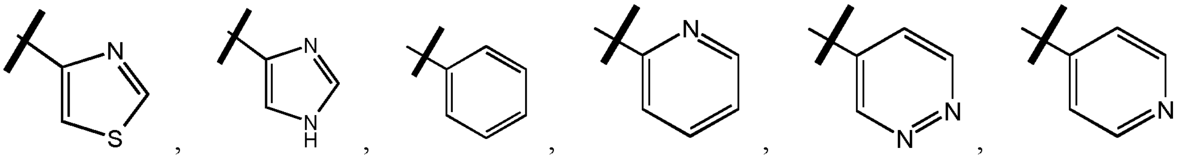

, , , , , ,

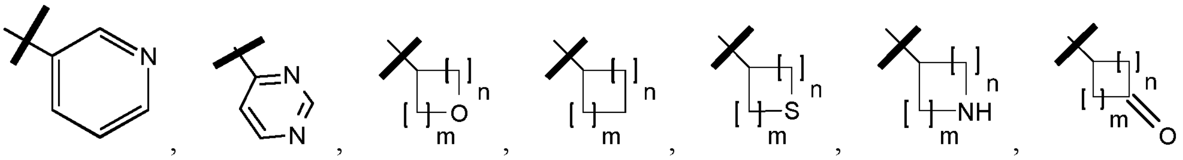

, , , , , , ,

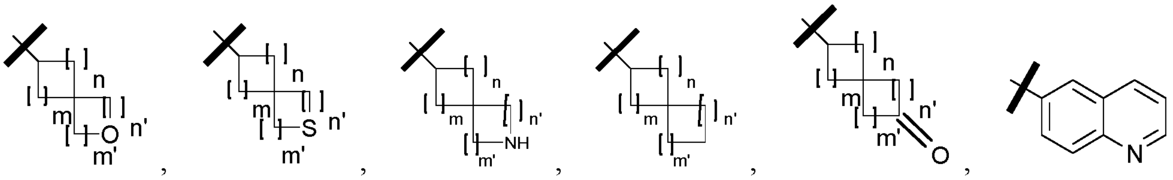

, , , , , ,

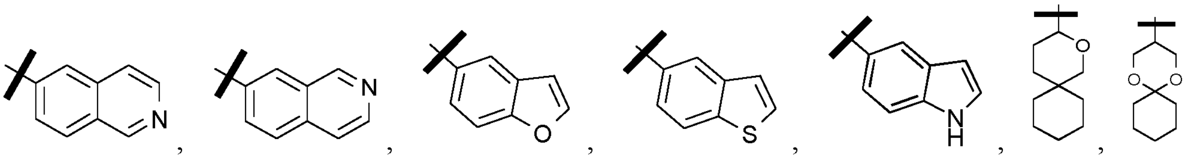

, , , , , , ,

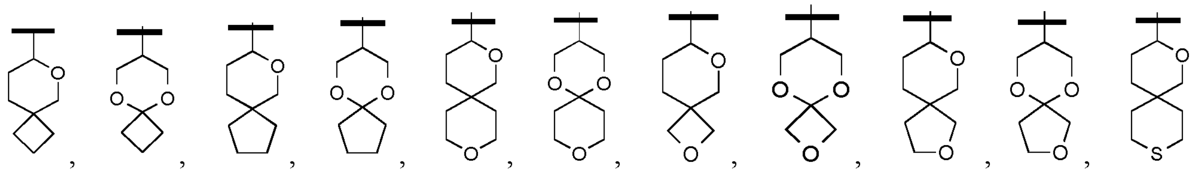

, , , , , , , , , , ,

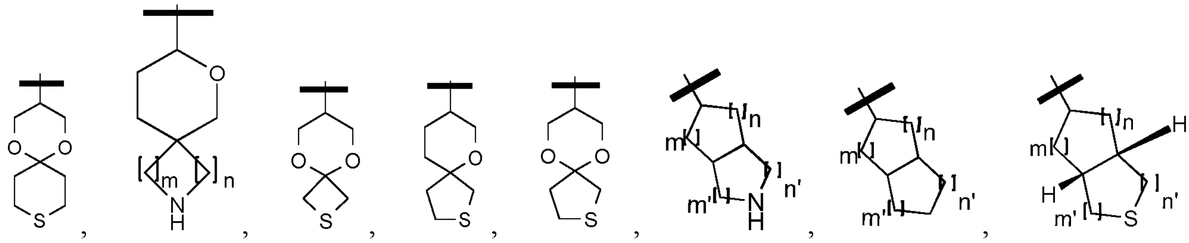

, , , , , , , ,

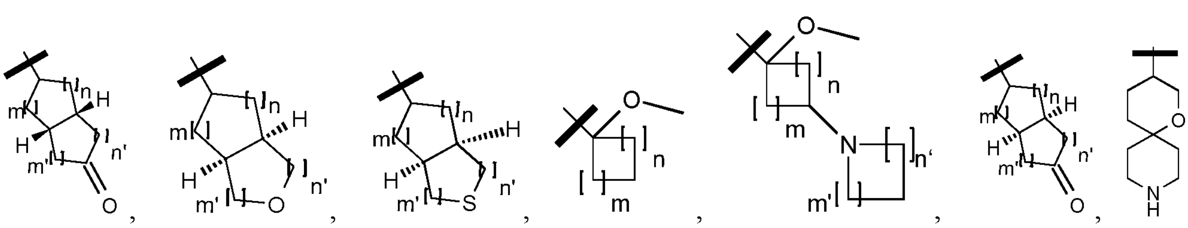

, , , , , , ,

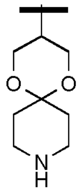, 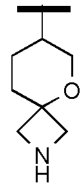, 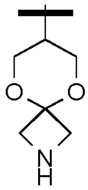, 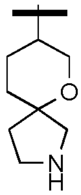 and 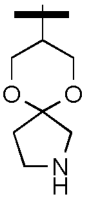;

wherein m, n, m', and n', at each occurrence, are each independently 1 or 2;
said one or more substituents are each independently selected from Cl, Br, F, I, oxo, carboxyl, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc); preferably, each independently selected from Cl, Br, F, I, oxo, hydroxy, methyl, hydroxymethyl, methoxy, amino, dimethylamino, cyano, nitro carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc); and
the bold bar line denotes the attachment point.

7. The compound of claim 6 or a pharmaceutically acceptable salt thereof, **characterized in that** $R_1$ is selected from the following groups optionally substituted with one or more substituents:

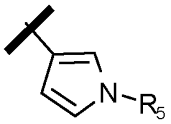, 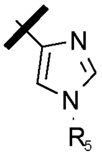, 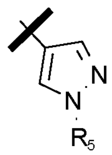, 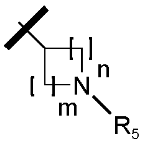, 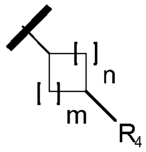, 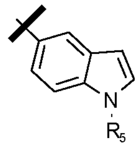, 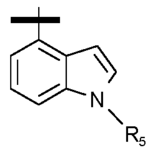,

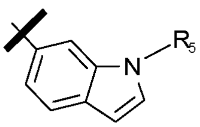, 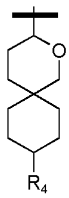, 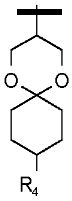, 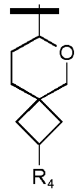, 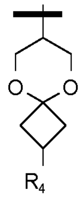, 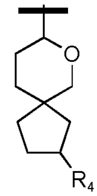, 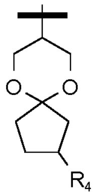, 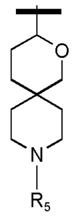, 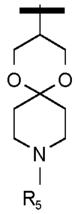,

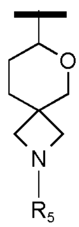, 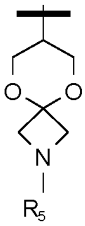, 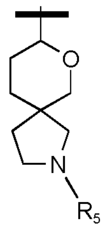, 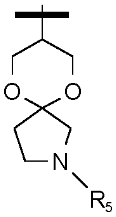, 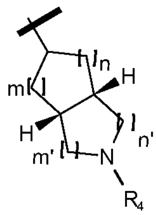, 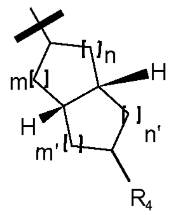, 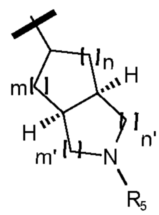

and

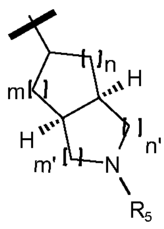;

wherein m, n, m', and n', at each occurrence, are each independently 1 or 2;

said one or more substituents are each independently selected from Cl, Br, F, I, oxo, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc); preferably, said one or more substituents are each independently selected from Cl, Br, F, I, oxo, hydroxy, methyl, hydroxymethyl, methoxy, amino, dimethylamino, cyano, nitro, carboxyl (-COOH), acetyloxy (AcO), benzyloxycarbonyl (Cbz), benzyl (Bn), tert-butoxycarbonyl (Boc), -(CO)H, -NH(CO)-$CH_3$, 5- to 6-membered heterocycloalkyl containing one nitrogen atom, and 9-fluorenylmethoxycarbonyl (Fmoc);
$R_4$ is, at each occurrence, independently selected from H, Cl, Br, F, I, oxo, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc); preferably, $R_4$ is, at each occurrence, independently selected from H, F, Cl, Br, I, -OH, oxo, methyl, amino (-$NH_2$), carboxyl (-COOH), methoxy, hydroxymethyl, dimethylamino, -(CO)H, -NH(CO)-$CH_3$, or 5- to 6-membered heterocycloalkyl containing one nitrogen atom;
$R_5$ is, at each occurrence, independently selected from H, Cl, Br, F, I, hydroxy, $C_1$-$C_3$ alkyl, $C_1$-$C_3$ hydroxyalkyl, $C_1$-$C_3$ alkoxy, $C_1$-$C_3$ haloalkyl, $C_1$-$C_3$ haloalkoxy, amino, $C_1$-$C_3$ alkyl amino, cyano, nitro, benzyloxycarbonyl (Cbz), carboxyl (-COOH), acetyloxy (AcO), benzyl (Bn), tert-butoxycarbonyl (Boc), -(CO)H, -NH(CO)-$C_1$-$C_3$ alkyl, 5- to 8-membered heterocycloalkyl containing one nitrogen atom, and 9-fluorenylmethoxycarbonyl (Fmoc); preferably, $R_5$ is, at each occurrence, independently selected from H, $C_1$-$C_3$ alkyl, acetyloxy (AcO), benzyl (Bn), benzyloxycarbonyl (Cbz), -(CO)H, tert-butoxycarbonyl (Boc), or 9-fluorenylmethoxycarbonyl (Fmoc); and
the bold bar line denotes the attachment point.

**8.** The compound of claim 1 or a pharmaceutically acceptable salt thereof, **characterized in that** said compound is selected from the following compounds:

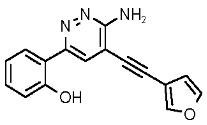, 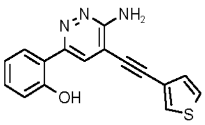, 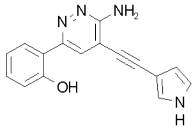, 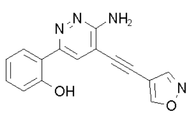, 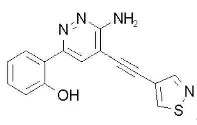,

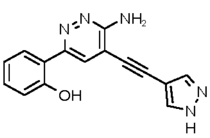, 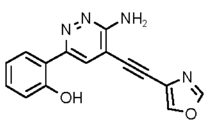, 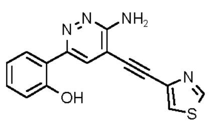, 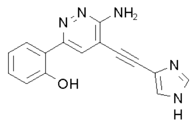, 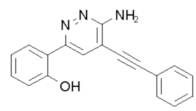,

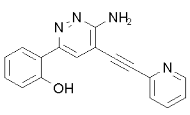, 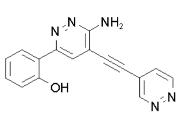, 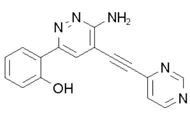, 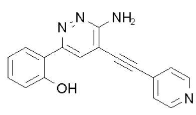, 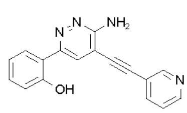,

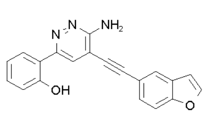, 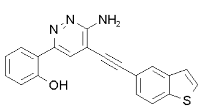, 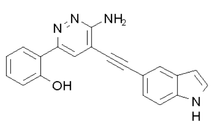, 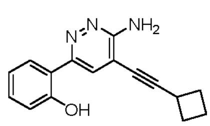, 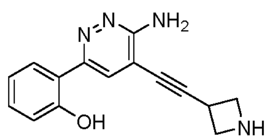,

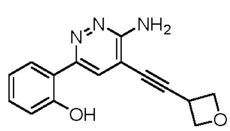, 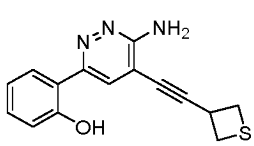, 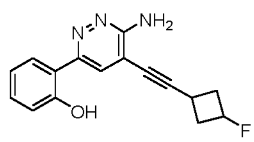, 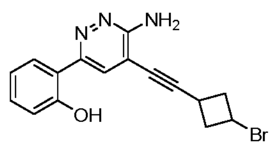,

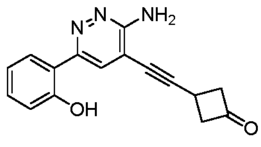, 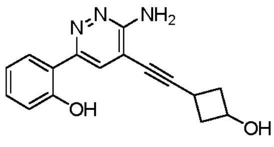, 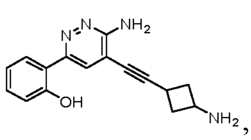, 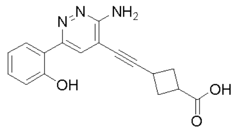,

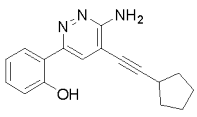, 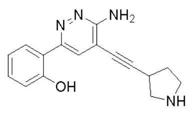, 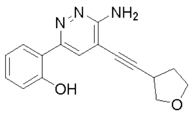, 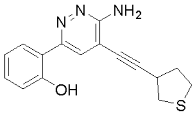, 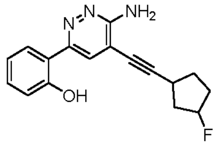,

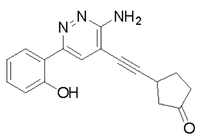, 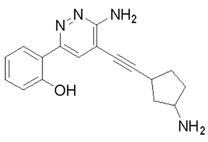, 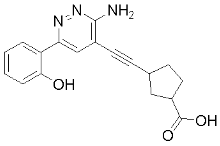, 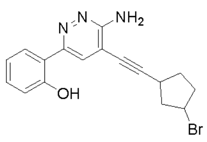, 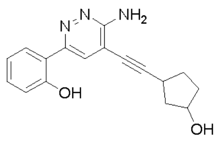,

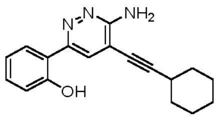, 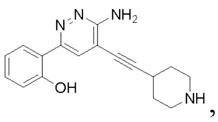, 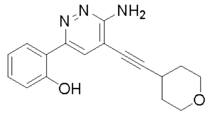, 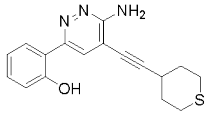, 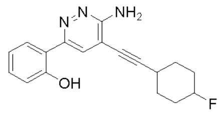,

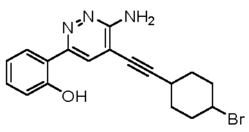, 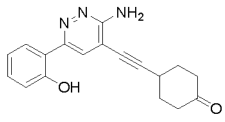, 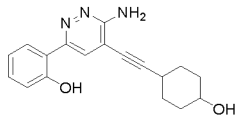, 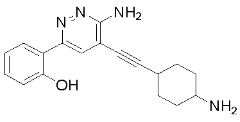,

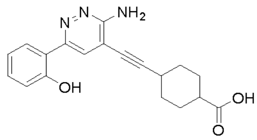, 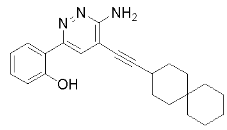, 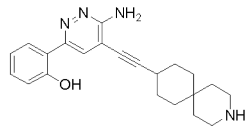, 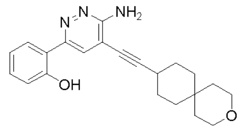,

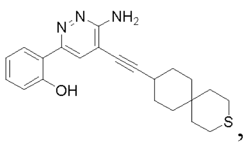, 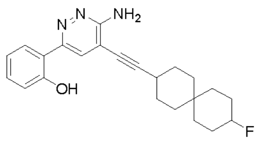, 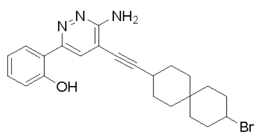, 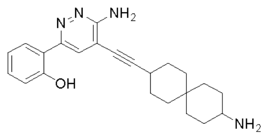,

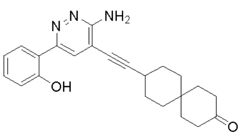, 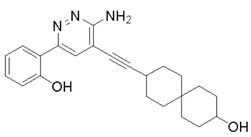, 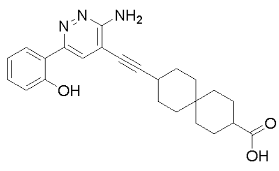, 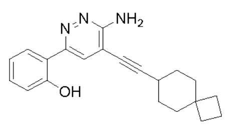,

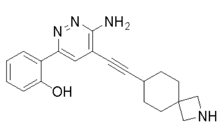, 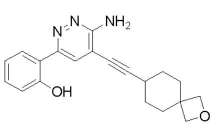, 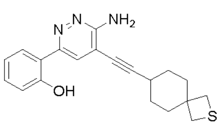, 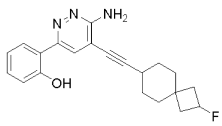, 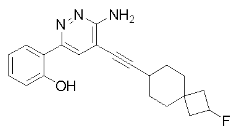,

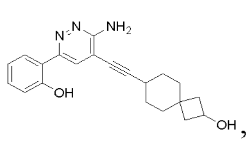, 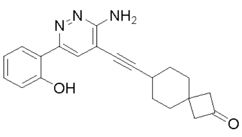, 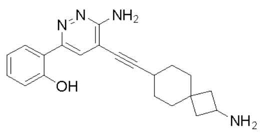, 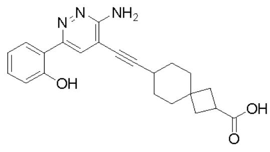,

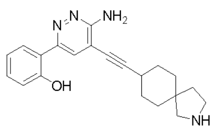, 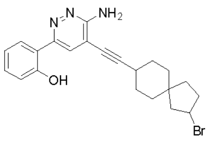, 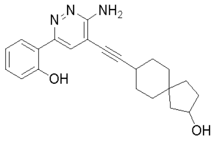, 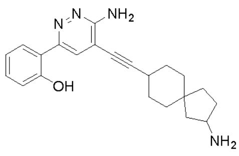, 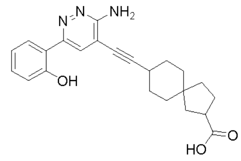,

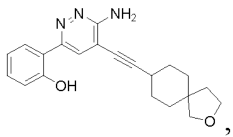, 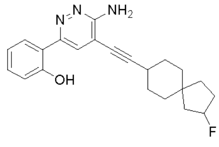, 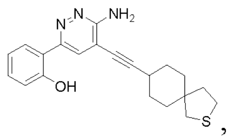, 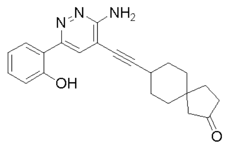, 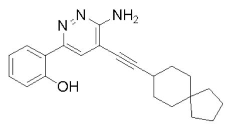,

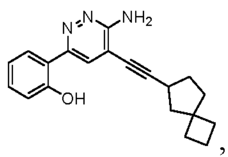, 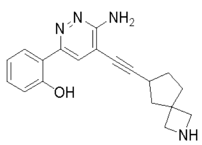, 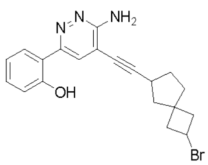, 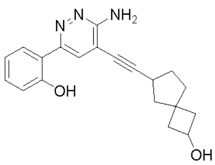, 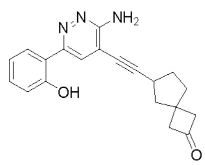,

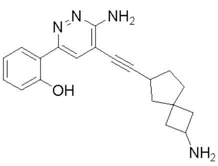, 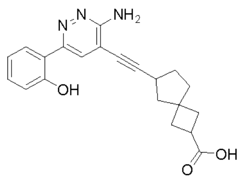, 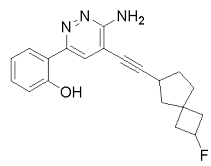, 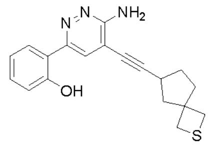, 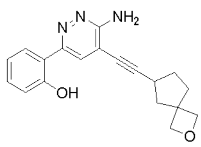,

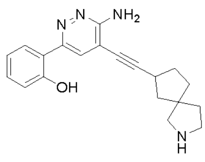, 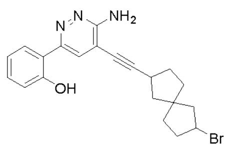, 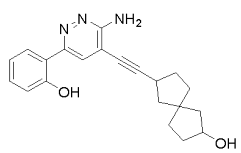, 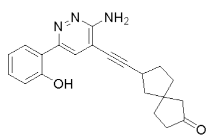, 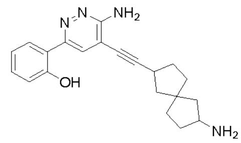,

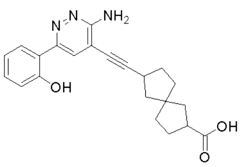, 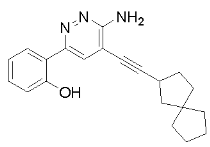, 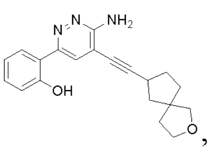, 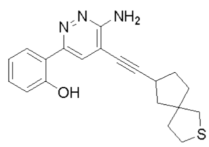, 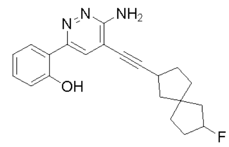,

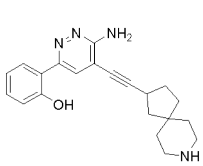, 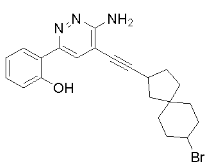, 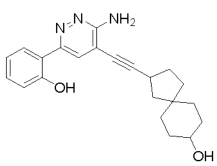, 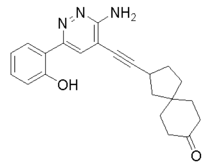, 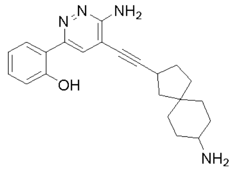,

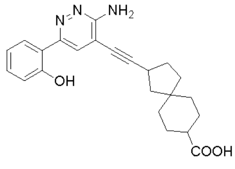, 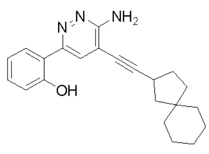, 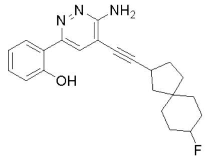, 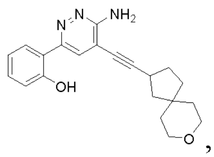, 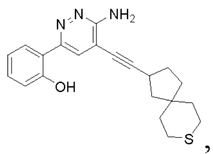,

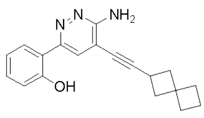, 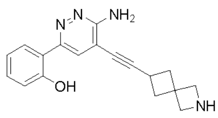, 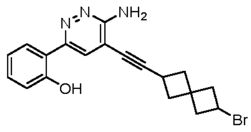, 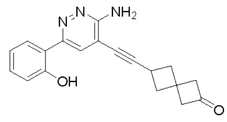, 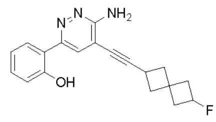,

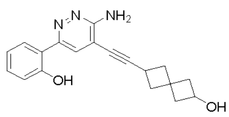, 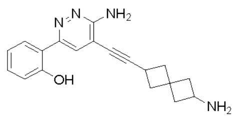, 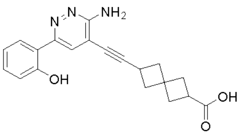, 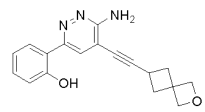, 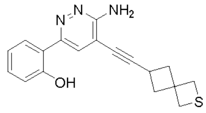,

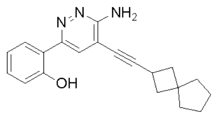, 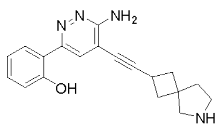, 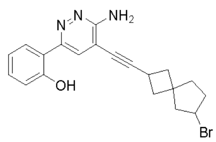, 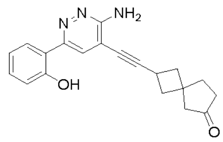, 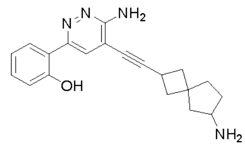,

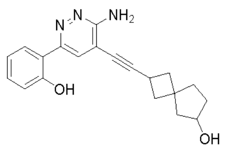, 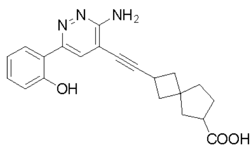, 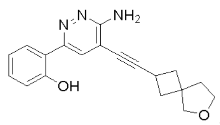, 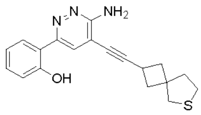, 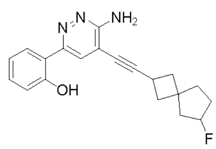,

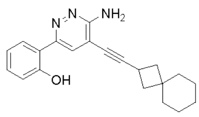, 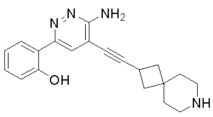, 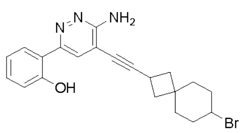, 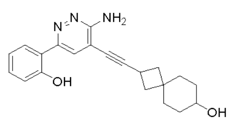, 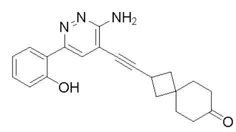,

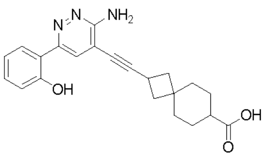, 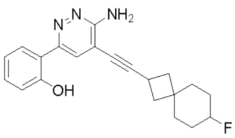, 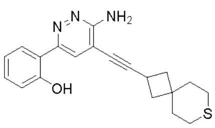, 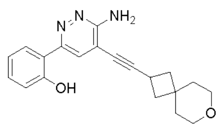,

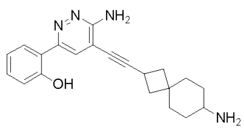, 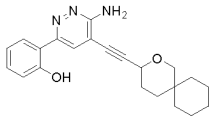, 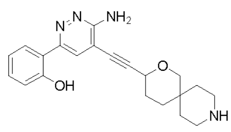, 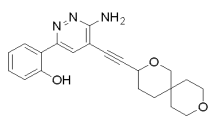, 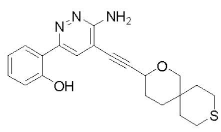,

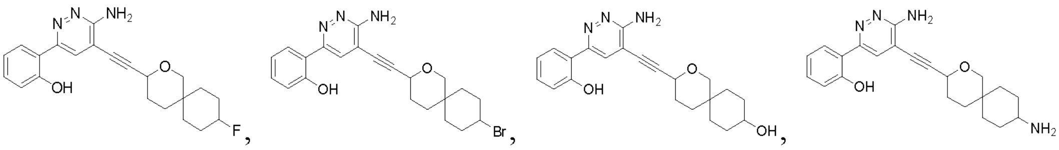, , , ,

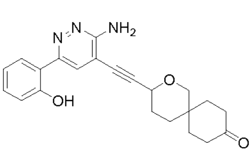, 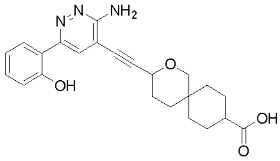, 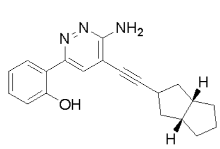, 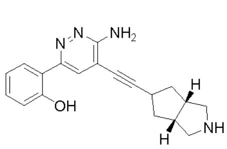,

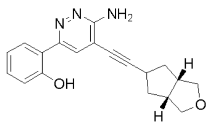, 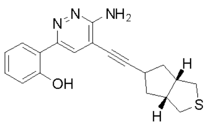, 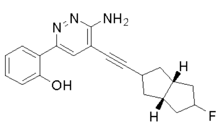, 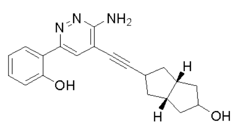, 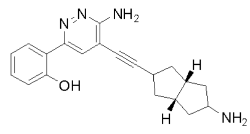,

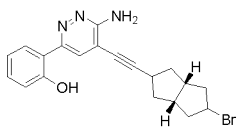, 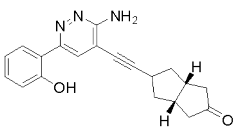 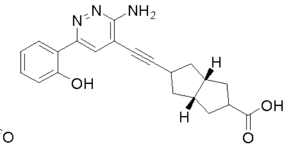, 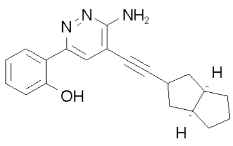,

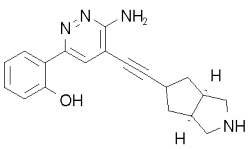, 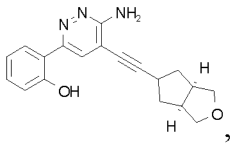, 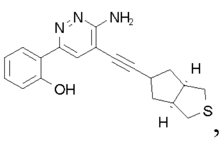, 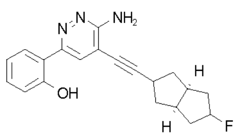,

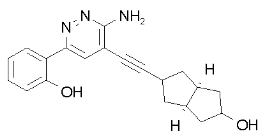, 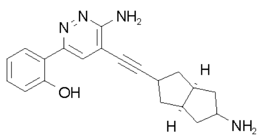, 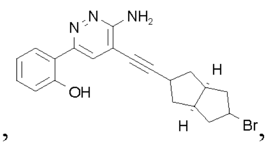, 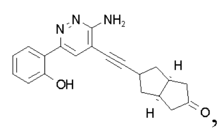,

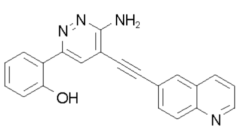, 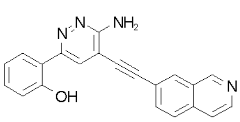, 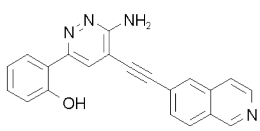, 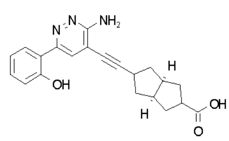,

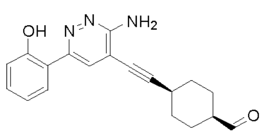, 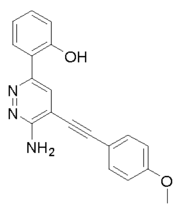, 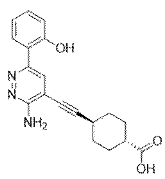, 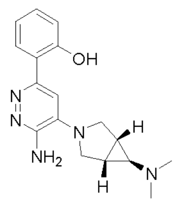, 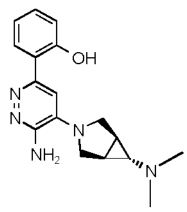,

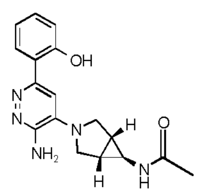, 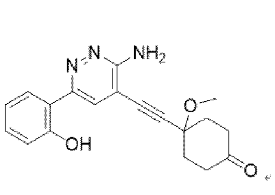, 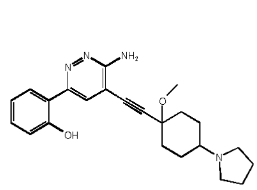, 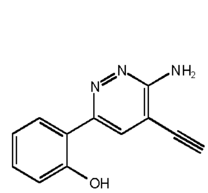,

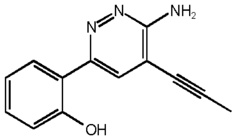, 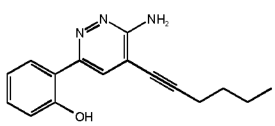,

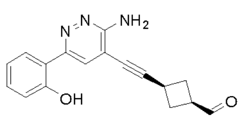, 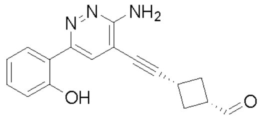, 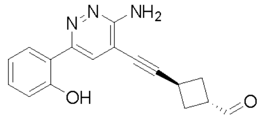, 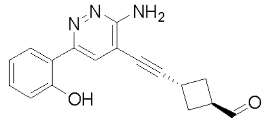,

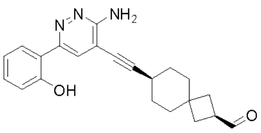, 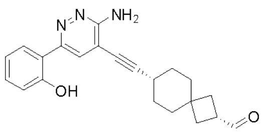, 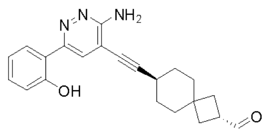, 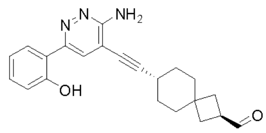,

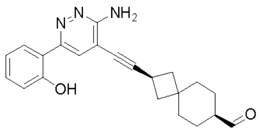, 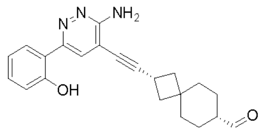, 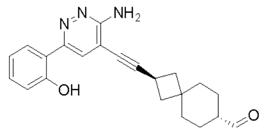, 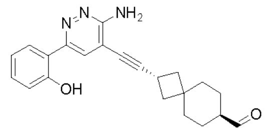

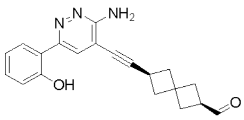, 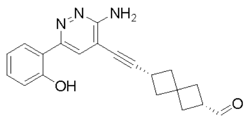, 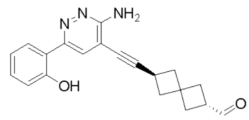, 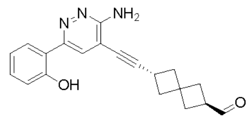,

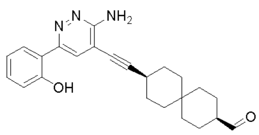, 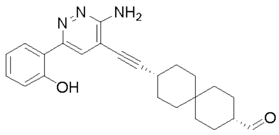, 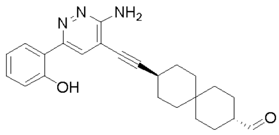, 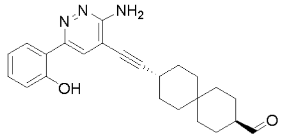,

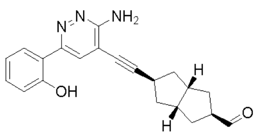, 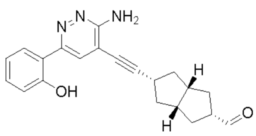, 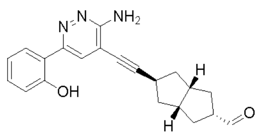, 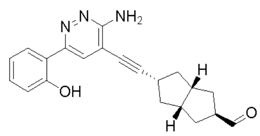,

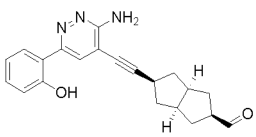, 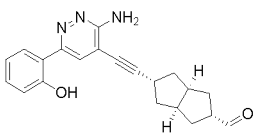, 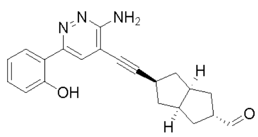, 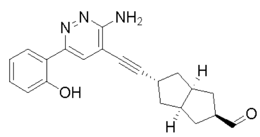,

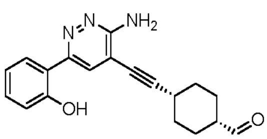, 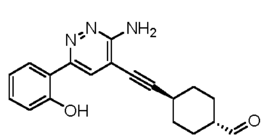 and 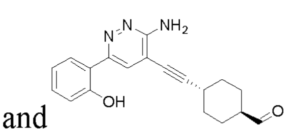.

9. A pharmaceutical composition, **characterized in that** comprising a therapeutically effective amount of the compound of any one of claims 1-8 or a pharmaceutically acceptable salt thereof, and a pharmaceutically acceptable carrier.

10. Use of the compound of any one of claims 1-8 or a pharmaceutically acceptable salt thereof, or the pharmaceutical composition of claim 9, in the preparation of a medicament for treating or preventing a disease or disorder mediated by SMARCA2/4.

11. The use of claim 10, **characterized in that** the disease or disorder mediated by SMARCA2/4 is cancer; preferably, the cancer is lung cancer, cervical cancer, and/or breast cancer.

12. A compound of any one of claims 1-8 or a pharmaceutically acceptable salt thereof, or a pharmaceutical composition of claim 9, for use in the treatment or prevention of a disease or disorder mediated by SMARCA2/4; preferably, the

disease or disorder is cancer, and more preferably, the cancer is lung cancer, cervical cancer, and/or breast cancer.

**13.** A method for treating or preventing a disease or disorder mediated by SMARCA2/4, comprising administering to a subject in need thereof a therapeutically effective amount of the compound of any one of claims 1-8 or a pharmaceutically acceptable salt thereof, or the pharmaceutical composition of claim 9; preferably, the disease or disorder is cancer, and more preferably, the cancer is lung cancer, cervical cancer, and/or breast cancer.

## INTERNATIONAL SEARCH REPORT

International application No.
**PCT/CN2023/105427**

**A. CLASSIFICATION OF SUBJECT MATTER**

C07D237/20(2006.01)i；C07D403/04(2006.01)i；A61K31/501(2006.01)i；A61P35/00(2006.01)i

According to International Patent Classification (IPC) or to both national classification and IPC

**B. FIELDS SEARCHED**

Minimum documentation searched (classification system followed by classification symbols)

IPC：C07D A61K A61P

Documentation searched other than minimum documentation to the extent that such documents are included in the fields searched

Electronic data base consulted during the international search (name of data base and, where practicable, search terms used)

CNTXT, CNKI, VEN, ENTXT, 万方数据, WANFANG DATA, STNext (Caplus, Registry): SMARCA2, SMARCA4, BRG1, BRM, 癌, 肿瘤, 染色质亚家族A成员, 基质相关肌动蛋白依赖调节剂, 哒嗪, 苯酚, 炔, 氨基, 羟基苯, chromatin subfamily, cancer, tumor, pyridazine, phenol, hydroxyphenyl, amino, alkynyl, ethynyl, structure search

**C. DOCUMENTS CONSIDERED TO BE RELEVANT**

| Category* | Citation of document, with indication, where appropriate, of the relevant passages | Relevant to claim No. |
|---|---|---|
| X | CN 107531668 A (GENENTECH, INC. et al.) 02 January 2018 (2018-01-02) claims 1-28 and 51-52, and description, paragraphs 0007, 0035 and 0041-0044, and embodiments 9, 28 and 84-85 | 1-2, 4, 6-12 |
| Y | CN 107531668 A (GENENTECH, INC. et al.) 02 January 2018 (2018-01-02) claims 1-28 and 51-52, and description, paragraphs 0007, 0035 and 0041-0044, and embodiments 9, 28 and 84-85 | 1-12 |
| X | CN 114599428 A (F. HOFFMANN-LA ROCHE AG et al.) 07 June 2022 (2022-06-07) description, paragraph 1015 | 1-2, 4-6, 12 |
| Y | CN 114599428 A (F. HOFFMANN-LA ROCHE AG et al.) 07 June 2022 (2022-06-07) claims 1-25, and description, examples 1-110 | 1-12 |
| Y | CN 112166114 A (ARVINAS OPERATIONS, INC. et al.) 01 January 2021 (2021-01-01) claims 1, 8 and 41-48, and description, table 1A | 1-12 |
| A | CN 114375192 A (AURIGENE DISCOVERY TECHNOLOGIES LIMITED) 19 April 2022 (2022-04-19) entire document | 1-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 November 2023** | **06 November 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/105427**

**C. DOCUMENTS CONSIDERED TO BE RELEVANT**

| Category* | Citation of document, with indication, where appropriate, of the relevant passages | Relevant to claim No. |
|---|---|---|
| X | American Chemical Society (ACS). "STNext Registry Database" *http://www.stn.org,* 21 September 2016 (2016-09-21), compounds with CAS RNs being 1997320-72-5 and 1997320-14-5 | 1-2, 4, 6-7, 12 |

Form PCT/ISA/210 (second sheet) (July 2022)

**INTERNATIONAL SEARCH REPORT**

International application No.
**PCT/CN2023/105427**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/105427**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

   The subject matter of claim 13 relates to a method for treatment of the human or animal body, which falls within the cases set out in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/105427**

| Patent document cited in search report | Publication date (day/month/year) | Patent family member(s) | Publication date (day/month/year) |
|---|---|---|---|
| CN 107531668 A | 02 January 2018 | US 2018086720 A1 | 29 March 2018 |
| | | US 10308614 B2 | 04 June 2019 |
| | | HK 1248696 A1 | 19 October 2018 |
| | | JP 2018510851 A | 19 April 2018 |
| | | JP 6847844 B2 | 24 March 2021 |
| | | MA 41598 A | 02 January 2018 |
| | | EP 3262036 A1 | 03 January 2018 |
| | | EP 3262036 B1 | 13 September 2023 |
| | | WO 2016138114 A1 | 01 September 2016 |
| | | CN 107531688 B | 29 October 2021 |
| CN 114599428 A | 07 June 2022 | JP 2023511471 A | 20 March 2023 |
| | | US 2023002367 A1 | 05 January 2023 |
| | | WO 2021086785 A1 | 06 May 2021 |
| | | EP 4054724 A1 | 14 September 2022 |
| CN 112166114 A | 01 January 2021 | CL 2020002546 A1 | 29 January 2021 |
| | | CA 3094305 A1 | 10 October 2019 |
| | | MX 2020010368 A | 08 January 2021 |
| | | KR 20210005037 A | 13 January 2021 |
| | | AU 2019249849 A1 | 01 October 2020 |
| | | AU 2019249849 B2 | 17 March 2022 |
| | | AU 2019249849 C1 | 29 September 2022 |
| | | CO 2020013762 A2 | 18 January 2021 |
| | | PH 12020500663 A1 | 07 June 2021 |
| | | AU 2022204042 A1 | 30 June 2022 |
| | | IL 277677 A | 30 November 2020 |
| | | BR 112020020196 A2 | 26 January 2021 |
| | | JP 2023071767 A | 23 May 2023 |
| | | US 2019300521 A1 | 03 October 2019 |
| | | PE 20212108 A1 | 04 November 2021 |
| | | RU 2020135754 A | 04 May 2022 |
| | | JP 2021520350 A | 19 August 2021 |
| | | EP 3774789 A1 | 17 February 2021 |
| | | WO 2019195201 A1 | 10 October 2019 |
| | | CR 20200527 A | 09 April 2021 |
| | | SG 11202008898 UA | 29 October 2020 |
| CN 114375192 A | 19 April 2022 | WO 2021048799 A1 | 18 March 2021 |
| | | CA 3150354 A1 | 18 March 2021 |
| | | MX 2022002244 A | 22 March 2022 |
| | | IL 291252 A | 01 May 2022 |
| | | US 2021077486 A1 | 18 March 2021 |
| | | BR 112022004573 A2 | 07 June 2022 |
| | | JP 2022547530 A | 14 November 2022 |
| | | EP 4028557 A1 | 20 July 2022 |
| | | AU 2020345178 A1 | 03 March 2022 |
| | | KR 20220061974 A | 13 May 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016138114 A

### Non-patent literature cited in the description

- *Current Opinion in Chemical Biology*, 2016, vol. 33, 58-66
- *Acta Pharmacol. Sin.*, 2018, vol. 39, 1544-1552